# EUROPEAN PATENT APPLICATION

(11) **EP 4 831 172 A1**
(43) Date of publication of application: **28.10.2026**
(21) Application number: 24906517.8
(22) Date of filing: 20.12.2024
(51) Int. Cl.: C07D 471/04, C07D 487/04, C07D 519/00, A61K 31/5377, A61K 31/437, A61K 31/444, A61K 31/4985, A61P 3/04, A61P 3/06

(54) **A CLASS OF POLYCYCLIC COMPOUNDS AND USES THEREOF**

(30) Priority: 22.12.2023 CN 202311782864; 07.02.2024 CN 202410173188
(71) Applicant: CSPC Baike (Shandong) Biopharmaceutical Co., Ltd., Yantai, Shandong 264100 (CN)
(72) Inventor: WANG, Kuanglei, Yantai, Shandong 264100 (CN); AN, Ran, Yantai, Shandong 264100 (CN); GUO, Jianqiao, Yantai, Shandong 264100 (CN); ZHAO, Chuanwu, Yantai, Shandong 264100 (CN); SONG, Yunlong, Yantai, Shandong 264100 (CN); WU, Bin, Yantai, Shandong 264100 (CN); TIAN, Jiahui, Yantai, Shandong 264100 (CN); CHU, Wenhao, Yantai, Shandong 264100 (CN); LI, Zizhen, Yantai, Shandong 264100 (CN); ZHU, Xingbo, Yantai, Shandong 264100 (CN)
(74) Representative: Høiberg P/S
(86) International application number: PCT/CN2024/140920
(87) International publication number: WO 2025/131043

(57) **Abstract**

Provided in the present invention are a class of compounds as shown in formula (I), stereoisomers, tautomers or pharmaceutically acceptable salts thereof. The compounds of the present invention are used as GLP-1R agonists, and the compounds and pharmaceutical compositions thereof can be used in the preparation of drugs for treating and/or preventing disorders mediated by GLP-1 R.

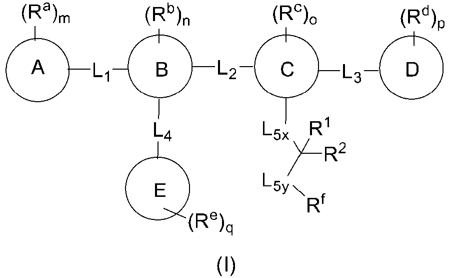

## Description

The present application claims the priority benefit of an earlier application filed by the Applicant:
with the China National Intellectual Property Administration on December 22, 2023, with Patent Application No. 202311782864.9 and entitled "A CLASS OF POLYCYCLIC COMPOUNDS AND USES THEREOF"; with the China National Intellectual Property Administration on February 7, 2024, with Patent Application No. 202410173188.3 and entitled "A CLASS OF POLYCYCLIC COMPOUNDS AND USES THEREOF"; the entire contents of the foregoing earlier applications are incorporated into this application by reference.

### Technical Field

The present application relates to the field of pharmaceutical technology, and specifically relates to a class of polycyclic compounds, pharmaceutical compositions comprising the same, and uses thereof as GLP-1R agonists; the polycyclic compounds can prevent and/or treat diseases, disorders, and conditions mediated by GLP-1R.

### Background Art

Overweight and obesity have become global health problems and are important causes of several chronic diseases, including diabetes, cardiovascular diseases, liver diseases, and cancers. Currently, about 50% of type 2 diabetes, 30% of ischemic cardiovascular and cerebrovascular diseases, and 10%-40% of cancers are caused by obesity or overweight. China has already been the country with the largest population of overweight and obesity in the world, and there remains a tremendous unmet clinical need for obesity.

Diabetes is a group of common metabolic endocrine diseases with disordered glucose and lipid metabolism and elevated plasma glucose levels as the main clinical features. Type 2 diabetes is most common, accounting for more than 90% of people with diabetes. The pathogenesis of type 2 diabetes remains unclear, but it is currently believed to be associated with unhealthy lifestyles, genetic factors, environmental factors, and the toxic effects of lipotoxicity and glucotoxicity on pancreatic β-cells. Moreover, long-term poor blood glucose control in patients may lead to various lesions, for example, the following diabetic complications: blood vessel complication (macroangiopathy (atherosclerosis) and microangiopathy (retinopathy and neuritis, etc.)), renal complications (renal insufficiency and proteinuria), cardiac complications (myocarditis and heart failure), urinary tract infections, and the like.

GLP-1 (glucagon-like peptide-1) is mainly synthesized and secreted by L cells located in the ileum and colon, and acts on GLP-1R (glucagon-like peptide-1 receptor), which is mainly distributed in pancreatic β cells and α cells, the gastrointestinal tract, the central nervous system, and the cardiovascular system, and the like. GLP-1R belongs to the glucagon receptor subfamily of cluster B of G protein-coupled receptors, and is typically characterized by a seven-transmembrane core domain and a relatively large extracellular domain.

In the pancreas, GLP-1 induces glucose-dependent insulin secretion. It increases insulin secretion and decreases glucagon. In the central nervous system, agonism of GLP-1R has effects such as increasing satiety, reducing food intake, causing nausea, and promoting nerve cell survival. In other tissue systems, agonism of GLP-1R also reduces blood pressure, improves the microvascular function, and reduces inflammation; delays gastric emptying, lowers blood glucose, increases insulin sensitivity, and reduces gluconeogenesis. Therefore, agonism of GLP-1R can achieve the effect of lowering blood glucose and/or reducing body weight, thereby preventing and/or treating diabetes and/or obesity. Moreover, agonism of GLP-1R can achieve the effect of losing weight through a variety of biological mechanisms.

In addition, evidence indicates that agonism of GLP-1R is also a promising strategy for preventing and/or treating Alzheimer's disease (AD), non-alcoholic fatty liver disease (NAFLD), and the like.

Alzheimer's disease is the most common neurodegenerative disease, with incurable cognitive impairment. The basic mechanisms underlying the occurrence and development of AD have not been fully elucidated. Recent experimental and clinical studies have shown that AD may be considered a metabolic disorder corresponding to type 2 diabetes mellitus (T2DM), referred to in some cases as type 3 diabetes. Insulin resistance is also present in the brains of deceased patients with AD, with significantly decreased insulin receptor expression accompanied by the disease progresses, suggesting that defective insulin signal transduction is associated with the pathogenesis of AD. In addition, studies have shown that, in AD, when glucose metabolism is impaired and the mitochondrial function is damaged, lactate shuttling during glycolysis in glial cells becomes increasingly important for neuronal survival, which plays an energy replacement role. GLP-1 can partially restore the astrocyte glycolytic function and increase lactate flux, which helps alleviate the energy crisis in neurons. The neuroprotective mechanism of GLP-1 is closely associated with its promotion of aerobic glycolysis, attenuation of oxidative phosphorylation activation, and activation of the PI3K/Akt pathway. Therefore, GLP-1 agonists are a promising strategy for preventing and treating AD.

Non-alcoholic fatty liver diseases are divided into two major categories: non-alcoholic fatty liver (NAFL) and non-alcoholic steatohepatitis (NASH). With an excessive supply of metabolic substrates, excess fat accumulates in hepatocytes, accompanied by the gradual production of potentially toxic lipid species and a progressive increase in de novo lipogenesis (DNL) in NAFLD. NASH, characterized by cellular injury and inflammatory cell infiltration, is considered as a more aggressive form of NAFLD and may progress to liver cirrhosis and hepatocellular carcinoma, with limited treatment options. Obesity and type 2 diabetes are two major risk factors for NASH, and individuals with a history of NASH are significantly more likely to develop liver and cardiovascular diseases. In addition, insulin resistance in the liver and adipose tissue is considered as a key driver of NASH morbidity and mortality, while GLP-1 analogues are able to improve glycemic control, reduce body weight, and activate liver enzymes in patients with T2DM. Some evidence suggests that liraglutide acts directly on human hepatocytes in vitro to reduce steatosis by reducing DNL levels and increasing fatty acid oxidation. Notably, steatosis and hepatocyte ballooning are improved in most patients after treatment with liraglutide. Encouraging experimental evidence obtained in NASH patients demonstrates the potential of liraglutide to reduce lipotoxicity by improving insulin sensitivity in adipose tissue. Only a small proportion of NASH patients experience disease progression after treatment with liraglutide. It has also been reported that semaglutide, an agent similar to liraglutide, can reduce levels of alanine aminotransferase and inflammatory markers, and that inflammatory biomarker levels are significantly reduced after treatment. Given the lack of hepatic GLP-1R expression, the potential mechanism of action of GLP-1R agonists in NASH may be related to indirect beneficial effects on body weight and reductions in metabolic dysfunction, lipotoxic effects, and inflammation.

### Summary of the Invention

In a first aspect, the present invention provides a compound as shown in formula (I), or tautomers, stereoisomers, or pharmaceutically acceptable salts thereof, having the following structure: 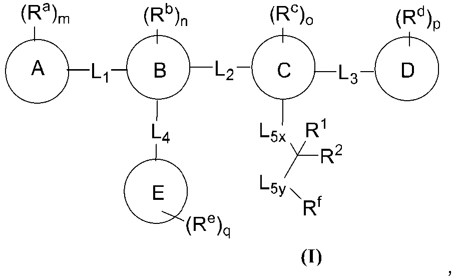 wherein,
Ring A is selected from C₃₋₁₅ carbocyclyl, 3-12 membered heterocyclyl, C₆₋₁₄ aryl, and 5-12 membered heteroaryl;
Ring B is selected from 3-12 membered heterocyclyl, C₆₋₁₄ aryl, and 5-12 membered heteroaryl;
Ring C is selected from 9-membered bicyclic heteroaryl;
Ring D is selected from C₃₋₁₅ carbocyclyl, 3-12 membered heterocyclyl, C₆₋₁₄ aryl, and 5-12 membered heteroaryl;
Ring E is selected from C₃₋₁₅ carbocyclyl, 3-12 membered heterocyclyl, C₆₋₁₄ aryl, and 5-12 membered heteroaryl;
R^{a}, at each occurrence, is independently selected from hydrogen, deuterium, halogen, hydroxy, amino, nitro, mercapto, cyano, oxo, -OR^{a1}, -S(O)R^{a1}, -SO₂(R^{a1}), -C(O)R^{a1}, -C(O)OR^{a1}, -OC(O)R^{a1}, -N(R^{a1})(R^{a2}), - C(O)N(R^{a1})(R^{a2}), -N(R^{a1})C(O)R^{a2}, -S(O)N(R^{a1})(R^{a2}), -SO₂N(R^{a1})(R^{a2}), -N(R^{a1})S(O)R^{a2}, -N(R^{a1})S(O)₂R^{a2}, and the following groups optionally substituted with one or more (for example, 2, 3, 4, 5, 6, 7, or 8) R^{a3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{a}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{a1} and R^{a2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{a3}, at each occurrence, is independently selected from hydrogen, deuterium, halogen, hydroxy, amino, nitro, mercapto, cyano, oxo, -R^{a4}, -OR^{a4}, -SR^{a4}, -S(O)R^{a4}, -S(O)₂R^{a4}, -C(O)R^{a4}, -C(O)OR^{a4}, -OC(O)R^{a4}, - N(R^{a4})(R^{a5}), -C(O)N(R^{a4})(R^{a5}), -N(R^{a4})C(O)R^{a5}, -S(O)N(R^{a4})(R^{a5}), -S(O)₂N(R^{a4})(R^{a5}), -N(R^{a4})S(O)R^{a5}, - N(R^{a4})S(O)₂R^{a5};
R^{a4} and R^{a5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{b}, at each occurrence, is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or one or more (for example, 2, 3, 4, 5, 6, 7, or 8) substitutable sites of a substituted group each independently substituted with R^{b1};
or two R^{b}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{b1}, at each occurrence, is independently selected from deuterium, halogen, hydroxy, amino, nitro, mercapto, cyano, oxo, -R^{b2}, -OR^{b2}, -SR^{b2}, -S(O)R^{b2}, -S(O)₂R^{b2}, -C(O)R^{b2}, -C(O)OR^{b2}, -OC(O)R^{b2}, - N(R^{b2})(R^{b3}), -C(O)N(R^{b2})(R^{b3}), -N(R^{b2})C(O)R^{b3}, -S(O)N(R^{b2})(R^{b3}), -S(O)₂N(R^{b2})(R^{b3}), -N(R^{b2})S(O)R^{b3}, - N(R^{b2})S(O)₂R^{b3};
R^{b2} and R^{b3}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
R^{c}, at each occurrence, is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or one or more (for example, 2, 3, 4, 5, 6, 7, or 8) substitutable sites of a substituted group each independently substituted with R^{c1};
or two R^{c}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{c1}, at each occurrence, is independently selected from deuterium, halogen, hydroxy, amino, nitro, mercapto, cyano, oxo, -R^{c2}, -OR^{c2}, -SR^{c2}, -S(O)R^{c2}, -S(O)₂R^{c2}, -C(O)R^{c2}, -C(O)OR^{c2}, -OC(O)R^{c2}, - N(R^{c2})(R^{c3}), -C(O)N(R^{c2})(R^{c3}), -N(R^{c2})C(O)R^{c3}, -S(O)N(R^{c2})(R^{c3}), -S(O)₂N(R^{c2})(R^{c3}), -N(R^{c2})S(O)R^{c3}, - N(R^{c2})S(O)₂R^{c3};
R^{c2} and R^{c3}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
R^{d}, at each occurrence, is independently selected from hydrogen, deuterium, halogen, hydroxy, amino, nitro, mercapto, cyano, oxo, -OR^{d1}, -S(O)R^{d1}, -S(O)₂R^{d1}, -C(O)R^{d1}, -C(O)OR^{d1}, -OC(O)R^{d1}, -N(R^{d1})(R^{d2}), - C(O)N(R^{d1})(R^{d2}), -N(R^{d1})C(O)R^{d2}, -S(O)N(R^{d1})(R^{d2}), -S(O)₂N(R^{d1})(R^{d2}), -N(R^{d1})S(O)R^{d2}, -N(R^{d1})S(O)₂R^{d2}, and the following groups optionally substituted with one or more (for example, 2, 3, 4, 5, 6, 7, or 8) R^{d3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{d}, together with atoms to which they are attached, form an optionally substituted 3-10 membered heterocyclyl, an optionally substituted 3-7 membered cycloalkyl, or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{d1} and R^{d2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{d3}, at each occurrence, is independently selected from hydrogen, deuterium, halogen, hydroxy, amino, nitro, mercapto, cyano, oxo, -R^{d4}, -OR^{d4}, -SR^{d4}, -S(O)R^{d4}, -S(O)₂R^{d4}, -C(O)R^{d4}, -C(O)OR^{d4}, -OC(O)R^{d4}, - N(R^{d4})(R^{d5}), -C(O)N(R^{d4})(R^{d5}), -N(R^{d4})C(O)R^{d5}, -S(O)N(R^{d4})(R^{d5}), -S(O)₂N(R^{d4})(R^{d5}), -N(R^{d4})S(O)R^{d5},-N(R^{d4})S(O)₂R^{d5};
R^{d4} and R^{d5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{e}, at each occurrence, is independently selected from hydrogen, deuterium, halogen, hydroxy, amino, nitro, mercapto, cyano, oxo, -OR^{e1}, -SR^{e1}, -S(O)R^{e1}, -S(O)₂R^{e1}, -C(O)R^{e1}, -C(O)OR^{e1}, -OC(O)R^{e1}, - N(R^{e1})(R^{e2}), -C(O)N(R^{e1})(R^{e2}), -N(R^{e1})C(O)R^{e2}, -S(O)N(R^{e1})(R^{e2}), -S(O)₂N(R^{e1})(R^{e2}), -N(R^{e1})S(O)R^{e2}, - N(R^{e1})S(O)₂R^{e2}, -C(O)N(R^{e1})S(O)₂N(R^{e1})(R^{e2}), -C(O)N(R^{e1})S(O)₂R^{e2}, -P(O)(R^{e1})R^{e2}, and the following groups optionally substituted with one or more (for example, 2, 3, 4, 5, 6, 7, or 8) R^{e3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{e}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{e1} and R^{e2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from hydrogen, halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{e3}, at each occurrence, is independently selected from hydrogen, deuterium, halogen, hydroxy, amino, nitro, mercapto, cyano, oxo, -R^{e4}, -OR^{e4}, -SR^{e4}, -S(O)R^{e4}, -S(O)₂R^{e4}, -C(O)R^{e4}, -C(O)OR^{e4}, -OC(O)R^{e4}, - N(R^{e4})(R^{e5}), -C(O)N(R^{e4})(R^{e5}), -N(R^{e4})C(O)R^{e5}, -S(O)N(R^{e4})(R^{e5}), -S(O)₂N(R^{e4})(R^{e5}), -N(R^{e4})S(O)R^{e5}, - N(R^{e4})S(O)₂R^{e5}, -C(O)N(R^{e4})S(O)₂N(R^{e4})(R^{e5}), -C(O)N(R^{e4})S(O)₂R^{e5}, -P(O)(R^{e4})R^{e5};
R^{e4} and R^{e5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{f} is selected from -C(O)OR^{f1}, -C(O)N(R^{f1})(R^{f2}), 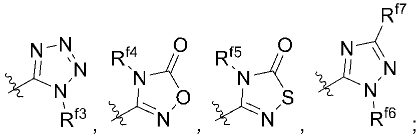
R^{f1}, R^{f2}, R^{f3}, R^{f4}, R^{f5}, R^{f6} and R^{f7}, at each occurrence, are respectively independently selected from hydrogen, deuterium, and the following optionally substituted groups: C₁₋₆ alkyl, C₁₋₆ alkoxy, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R¹ and R², together with atoms to which they are attached, form C₃₋₁₅ carbocyclyl; the C₃₋₁₅ carbocyclyl is optionally substituted with one or more (for example, 2, 3, 4, 5, 6, 7, or 8) R³; R³ is independently selected from hydrogen, deuterium, halogen, hydroxy, or the following optionally substituted groups: C₁₋₆ alkyl, C₁₋₆ alkoxy, C₁₋₆ alkylamino, C₃₋₁₀ cycloalkyl, and 3-10 membered heterocyclyl; optionally substituted means unsubstituted or one or more (for example, 2, 3, 4, 5, 6, 7, or 8) substitutable sites of a substituted group each independently substituted with deuterium, halogen, hydroxy, amino, cyano, oxo, C₃₋₁₀ cycloalkyl, and 3-10 membered heterocyclyl;
L₁ is selected from a bond, -C(R^{L1})₂-, -O-, -C(R^{L1})₂O-, -S-, -C(O)-, -C(O)O-, -OC(O)-, -N(R^{L1})C(O)-,-C(O)N(R^{L1})-, or -N(R^{L1})-; R^{L1} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₂ is independently selected from a bond, -C(R^{L2})₂-, -O-, -C(R^{L2})₂O-, -S-, -C(O)-, -C(O)O-, -OC(O)-, - N(R^{L2})C(O)-, -C(O)N(R^{L2})-, or -N(R^{L2})-; R^{L2} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₃ is independently selected from a bond, -C(R^{L3})₂-, -O-, -S-, -C(O)-, -C(O)O-, -OC(O)-, -N(R ^{L3})C(O)-, - C(O)N(R^{L3})-, or -N(R^{L3})-; R^{L3} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₄ is independently selected from a bond, -L₄ₓ-C(R^{L4a}) (R^{L4b})-L_{4y}-, -L₄ₓ-O-L_{4y}-, -L₄ₓ-S-L_{4y}-, -L₄ₓ-C(O)-L_{4y}-, - L₄ₓ-C(O)O-L_{4y}-, -L₄ₓ-OC(O)-L_{4y}-, -L₄ₓ-N(R^{L4a})C(O)-L_{4y}-, -L₄ₓ-C(O)N(R^{L4a})-L_{4y}-, -L₄ₓ-N(R^{L4a})-L_{4y}-, -L₄ₓ-N(R^{L4a})C(O)N(R^{L4b})-L_{4y}-, -L₄ₓ-N(R^{L4a})C(S)N(R^{L4b})-L_{4y}-, or 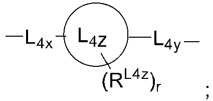
L₄ₓ and L_{4y} are respectively independently selected from a bond or optionally substituted C₁₋₁₀ alkylene; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
R^{L4a} and R^{L4b} are respectively independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
Ring L_{4Z} is selected from C₃₋₁₅ carbocyclyl, 3-12 membered heterocyclyl, C₆₋₁₄ aryl, and 5-12 membered heteroaryl;
R^{L4z} is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl; optionally substituted means unsubstituted or one or more substitutable sites of a substituted group independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₅ₓ and L_{5y} are respectively independently selected from a bond or optionally substituted C₁₋₁₀ alkylene; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
m, n, o, p, q, and r are each independently 0, 1, 2, or 3;
unless otherwise stated, heteroatoms in the above-mentioned heterocyclyl and heteroaryl are independently selected from O, N or S, and the number of the heteroatoms is 1, 2, 3 or 4;
provided that a structure formed by the definitions of the above variables, when combined, is a stable chemical structure.

In some embodiments, R^{d}, at each occurrence, is independently selected from hydrogen, deuterium, halogen, hydroxy, amino, nitro, mercapto, cyano, oxo, -OR^{d1}, -S(O)R^{d1}, -S(O)₂R^{d1}, -C(O)R^{d1}, -C(O)OR^{d1},-OC(O)R^{d1}, -N(R^{d1})(R^{d2}), -C(O)N(R^{d1})(R^{d2}), -N(R^{d1})C(O)R^{d2}, -S(O)N(R^{d1})(R^{d2}), -S(O)₂N(R^{d1})(R^{d2}), - N(R^{d1})S(O)R^{d2}, -N(R^{d1})S(O)₂R^{d2}, and the following groups optionally substituted with one or more (for example, 2, 3, 4, 5, 6, 7, or 8) R^{d3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{d}, together with atoms to which they are attached, form an optionally substituted 3-10 membered heterocyclyl or 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl.

In some embodiments, wherein Ring A is selected from C₃₋₁₀ carbocyclyl, 3-10 membered heterocyclyl, C₆₋₁₀ aryl, and 5-10 membered heteroaryl;
or Ring A is selected from C₃₋₈ carbocyclyl, 3-8 membered heterocyclyl, C₆₋₈ aryl, and 5-8 membered heteroaryl;
or Ring A is selected from C₃₋₆ carbocyclyl, 3-6 membered heterocyclyl, phenyl, and 5-6 membered heteroaryl;
wherein the heteroatoms in the heterocyclyl and heteroaryl are selected from N, O, or S, and the number of the heteroatoms is 1 or 2.

In some embodiments, wherein Ring A is C₃₋₁₀ carbocyclyl; or Ring A is C₃₋₆ carbocyclyl;
or Ring A is C₃₋₆ carbocyclyl, provided that Ring A is not 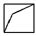 or cyclohexyl.

In some embodiments, wherein Ring A is 3-10 membered heterocyclyl; or Ring A is 3-6 membered heterocyclyl; preferably, the heteroatoms in the heterocyclyl are selected from N, O, or S, and the number of the heteroatoms is 1 or 2.

In some embodiments, Ring A is 5-10 membered heteroaryl; or Ring A is 5-8 membered heteroaryl, the heteroatoms in the heteroaryl are selected from N, O, or S, and the number of the heteroatoms is 1, 2, or 3; or Ring A is selected from 5-membered heteroaryl and 6-membered heteroaryl, the heteroatoms in the heteroaryl are selected from N, O, or S, and the number of the heteroatoms is 1 or 2; or Ring A is selected from pyridyl, pyrrolyl, furyl, and thienyl; or Ring A is pyrrolyl, furyl, or thienyl.

In some embodiments, Ring A is pyridyl.

In some embodiments, wherein Ring A is selected from C₆₋₁₀ aryl; or Ring A is selected from C₆₋₈ aryl; or Ring A is selected from phenyl.

In some embodiments, wherein R^{a} is independently selected from deuterium, halogen, hydroxy, amino, nitro, mercapto, cyano, oxo, -C(O)R^{a1}, -C(O)OR^{a1}, -OC(O)R^{a1}, -N(R^{a1})(R^{a2}), -C(O)N(R^{a1})(R^{a2}), - N(R^{a1})C(O)(R^{a2}), and the following groups optionally substituted with one or more (for example, 2, 3, 4, 5, 6, 7, or 8) R^{a3}: C₁₋₆ alkyl, C₁₋₆ alkoxy, C₁₋₆ alkylamino, C₂₋₄ alkenyl, C₂₋₄ alkynyl, C₃₋₆ cycloalkyl, 3-6 membered heterocyclyl, C₆₋₈ aryl, and 5-6 membered heteroaryl; or R^{a} is independently selected from halogen, hydroxy, amino, nitro, mercapto, cyano, oxo, -C(O)N(R^{a1})(R^{a2}), -N(R^{a1})C(O)(R^{a2}), and the following groups optionally substituted with one or more (for example, 2, 3, 4, 5, 6, 7, or 8) R^{a3}: C₁₋₆ alkyl, C₁₋₆ alkoxy, C₃₋₆ cycloalkyl, and 3-6 membered heterocyclyl; the heteroatoms in the heterocyclyl are O or N, and the number of the heteroatoms is 1 or 2;
or R^{a} is independently selected from halogen, hydroxy, amino, cyano, and the following groups optionally substituted with one or more (for example, 2, 3, 4, 5, 6, 7, or 8) R^{a3}: C₁₋₄ alkyl, C₁₋₄ alkoxy, C₃₋₄ cycloalkyl, and 4-5 membered heterocyclyl; the heteroatoms in the heterocyclyl are O or N, and the number of the heteroatoms is 1 or 2;
or R^{a} is independently selected from halogen, hydroxy, amino, cyano, and the following groups optionally substituted with one or more (for example, 2 or 3) substituents: C₁₋₄ alkyl, C₁₋₄ alkoxy, C₃₋₄ cycloalkyl, and 4-5 membered heterocyclyl; the substituents are selected from halogen, hydroxy, and amino; the heteroatom in the heterocyclyl is O, and the number of heteroatoms is 1.

In some embodiments, wherein R^{a} is independently selected from halogen, hydroxy, amino, nitro, mercapto, cyano, oxo, -C(O)N(R^{a1})(R^{a2}), -N(R^{a1})C(O)(R^{a2}), C₁₋₆ alkyl, and C₁₋₆ alkoxy; the C₁₋₆ alkyl and C₁₋₆ alkoxy are optionally substituted with one or more R^{a3};
or R^{a} is independently selected from halogen, hydroxy, amino, cyano, C₁₋₄ alkyl, and C₁₋₄ alkoxy; the C₁₋₄ alkyl and C₁₋₄ alkoxy are optionally substituted with one or more R^{a3}.

In some embodiments, wherein R^{a} is independently selected from halogen, hydroxy, amino, cyano, and the following optionally substituted groups: methyl, ethyl, n-propyl, isopropyl, n-butyl, methoxy, or ethoxy; optionally substituted means unsubstituted or substituted with one or more (for example, 2, 3, or 4) substituents selected from deuterium, halogen, hydroxy, and amino;

In some embodiments, wherein R^{a} is independently selected from halogen, methyl, and cyclopropyl; or R^{a} is independently selected from fluoro, methyl, and cyclopropyl.

In some embodiments, wherein R^{a} is independently selected from fluoro, methyl, deuterated methyl (-CD₃), -CF₃, methoxy, cyclopropyl, and -OCF₃.

In some embodiments, wherein two R^{a}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₃₋₆ cycloalkyl, 3-6 membered heterocyclyl, C₆₋₈ aryl, and 5-6 membered heteroaryl;
or two R^{a}, together with the atoms to which they are attached, form an optionally substituted 3-6 membered heterocyclyl or an optionally substituted 5-6 membered heteroaryl, and optionally substituted means unsubstituted or substituted with one or more (for example, 2, 3, or 4) groups selected from halogen, hydroxy, amino, nitro, mercapto, cyano, oxo, methyl, ethyl, n-propyl, isopropyl, n-butyl, methoxy, and ethoxy.

In some embodiments, wherein R^{a1} and R^{a2} are respectively independently selected from hydrogen, deuterium, and the following optionally substituted groups: C₁₋₆ alkyl, C₁₋₆ alkoxy, C₃₋₆ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₃₋₆ cycloalkyl, 3-6 membered heterocyclyl, phenyl, and 5-6 membered heteroaryl;
or R^{a1} and R^{a2} are respectively independently selected from hydrogen and C₁₋₆ alkyl, for example, C₁₋₃ alkyl; preferably methyl or ethyl; the C₁₋₆ alkyl is optionally substituted with one or more substituents selected from hydrogen, halogen, hydroxy, and amino; or R^{a1} and R^{a2} are respectively independently selected from hydrogen.

In some embodiments, wherein R^{a3} is independently selected from hydrogen, deuterium, halogen, hydroxy, amino, nitro, mercapto, cyano, oxo, -R^{a4}, -OR^{a4}, -C(O)R^{a4}, -C(O)OR^{a4}, -OC(O)R^{a4}, -N(R^{a4})(R^{a5}), - C(O)N(R^{a4})(R^{a5}), -N(R^{a4})C(O)R^{a5}.

In some embodiments, wherein R^{a3} is independently selected from hydrogen, halogen, hydroxy, amino, and -R^{a4}; wherein R^{a4} is independently selected from C₁₋₆ alkyl (for example, C₁₋₃ alkyl; preferably methyl or ethyl), and C₁₋₆ alkoxy (for example, C₁₋₃ alkoxy; preferably methoxy or ethoxy); the C₁₋₆ alkyl is optionally substituted with one or more substituents selected from hydrogen and halogen.

In some embodiments, wherein R^{a3} is independently selected from hydrogen and halogen; or R^{a3} is independently selected from hydrogen, fluoro, and chloro.

In some embodiments, wherein R^{a4} and R^{a5} are respectively independently selected from hydrogen and the following optionally substituted groups: C₁₋₄ alkyl, C₁₋₄ alkoxy, C₃₋₆ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from halogen, hydroxy, amino, cyano, methyl, ethyl, n-propyl, isopropyl, n-butyl, methoxy, and ethoxy;
or R^{a4} and R^{a5} are respectively independently selected from hydrogen and C₁₋₄ alkyl; the C₁₋₄ alkyl is optionally substituted with one or more (for example, 2, 3, or 4) substituents selected from hydrogen and halogen.

In some embodiments, wherein R^{a4} and R^{a5} are respectively independently selected from hydrogen, methyl, ethyl, n-propyl, isopropyl, trifluoromethyl, difluoromethyl, monofluoromethyl, trichloromethyl, dichloromethyl, and monochloromethyl.

In some embodiments, wherein m is selected from 0, 1, 2, or 3; or m is selected from 2 or 3.

In some embodiments, wherein L₁ is selected from a bond, -C(R^{L1})₂-, -C(R^{L1})₂O-, -O-, -C(O)-, -N(R^{L1})C(O)-, -C(O)N(R^{L1})-; R^{L1} is selected from hydrogen or the following optionally substituted groups: C₁₋₆ alkyl and C₁₋₆ alkoxy; optionally substituted means unsubstituted or substituted with one or more (for example, 2, 3, 4, 5, 6, 7, or 8) groups selected from deuterium, halogen, hydroxy, amino, cyano, C₁₋₄ alkyl, C₁₋₄ alkoxy, C₃₋₆ cycloalkyl, and 3-6 membered heterocyclyl;
or L₁ is selected from a bond, -C(R^{L1})₂-, -C(R^{L1})₂O-; R^{L1} is selected from hydrogen or optionally substituted C₁₋₄ alkyl, and optionally substituted means unsubstituted or substituted with one or more (for example, 2 or 3) groups selected from halogen, hydroxy, amino, and cyano;
or L₁ is selected from a bond, -C(R^{L1})₂-, -C(R^{L1})₂O-; R^{L1} is selected from hydrogen, optionally substituted methyl, or optionally substituted ethyl, and optionally substituted means unsubstituted or substituted with one or more (for example, 2 or 3) groups selected from fluoro, chloro, bromo, hydroxy, amino, and cyano; or L₁ is selected from a bond, -CH(CH₃)-, -CH₂-, -CH₂O-; or L₁ is selected from a bond and -CH₂-; or L₁ is a bond.

In some embodiments, L₁ is not -CH(CH₃)-.

In some embodiments, wherein Ring B is selected from 3-10 membered heterocyclyl, C₆₋₁₀ aryl, and 5-10 membered heteroaryl;
or Ring B is selected from 4-6 membered monocyclic heterocyclyl, 8-10 membered bicyclic heterocyclyl, phenyl, naphthyl, 5-6 membered monocyclic heteroaryl, and 8-10 membered bicyclic heteroaryl;
or Ring B is selected from 8-10 membered bicyclic heterocyclyl and 8-10 membered bicyclic heteroaryl;
or Ring B is selected from 8-9 membered bicyclic heterocyclyl and 8-9 membered bicyclic heteroaryl; the heteroatoms in the 8-9 membered bicyclic heterocyclyl and 8-9 membered bicyclic heteroaryl are selected from N or S, and the number of the heteroatoms is 1, 2, or 3;

In some embodiments, Ring B is selected from 9-membered fused heterocyclyl and 9-membered bicyclic heteroaryl; wherein the fused heterocyclyl is a heterocyclyl fused to a heteroaryl, and the bicyclic heteroaryl is a heteroaryl fused to a heteroaryl or a heteroaryl fused to an aryl; the heteroatoms in the 9-membered fused heterocyclyl are N, and the number of the heteroatoms is 3; the heteroatoms in the 9-membered bicyclic heteroaryl are N, and the number of the heteroatoms is 1 or 2.

In some embodiments, wherein Ring B is selected from 4-6 membered heterocyclyl, 4-6 membered monocyclic heterocycloalkenyl, phenyl, naphthyl, 5-6 membered monocyclic heteroaryl, 3-membered/5-membered fused heterocyclyl, 5-membered/3-membered fused heterocyclyl, 4-membered/4-membered fused heterocyclyl, 4-membered/5-membered fused heterocyclyl, 5-membered/4-membered fused heterocyclyl, 5-membered/5-membered fused heterocyclyl, 4-membered/6-membered fused heterocyclyl, 6-membered/4-membered fused heterocyclyl, 5-membered/6-membered fused heterocyclyl, 6-membered/5-membered fused heterocyclyl, 6-membered/6-membered fused heterocyclyl, 6-membered/7-membered fused heterocyclyl, 7-membered/6-membered fused heterocyclyl, 5-membered/5-membered fused heteroaryl, 5-membered/6-membered fused heteroaryl, 6-membered/5-membered fused heteroaryl, and 6-membered/6-membered fused heteroaryl;
or Ring B is selected from 5-membered/5-membered fused heterocyclyl, 5-membered/6-membered fused heterocyclyl, 6-membered/5-membered fused heterocyclyl, 5-membered/5-membered fused heteroaryl, 5-membered/6-membered fused heteroaryl, and 6-membered/5-membered fused heteroaryl;
or Ring B is selected from 5-membered/5-membered fused heterocyclyl, 5-membered/6-membered fused heterocyclyl, and 6-membered/5-membered fused heterocyclyl, and the heteroatoms in the 5-membered/5-membered fused heterocyclyl, 5-membered/6-membered fused heterocyclyl, and 6-membered/5-membered fused heterocyclyl are selected from N, and the number of heteroatoms is 1, 2, or 3;
or Ring B is selected from 5-membered/6-membered fused heterocyclyl and 6-membered/5-membered fused heterocyclyl, wherein the heteroatoms in the 5-membered/6-membered fused heterocyclyl and 6-membered/5-membered fused heterocyclyl are selected from N, and the number of the heteroatoms is 3. In some embodiments, wherein Ring B is selected from the following groups:

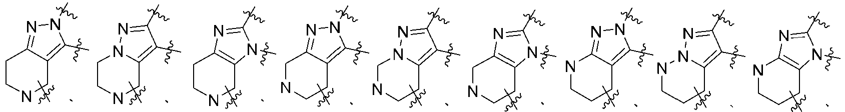

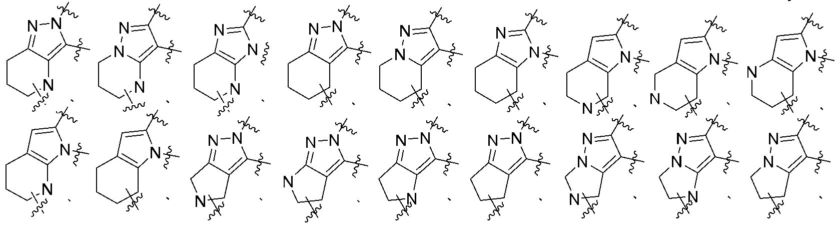

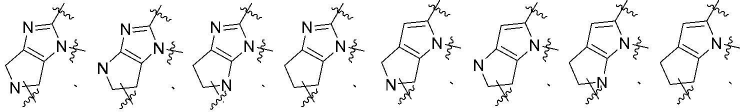

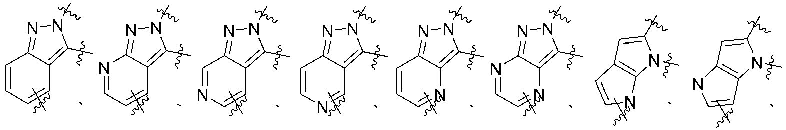

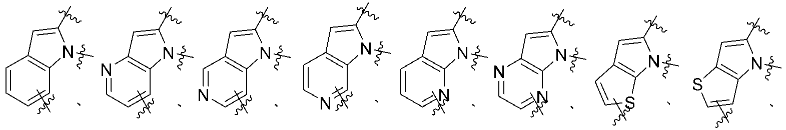

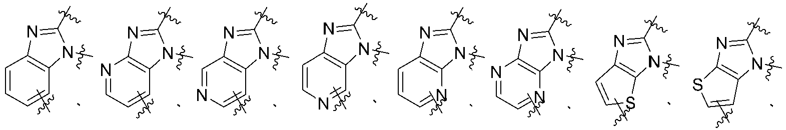

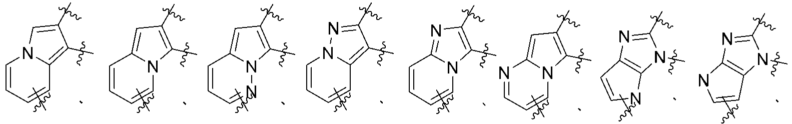

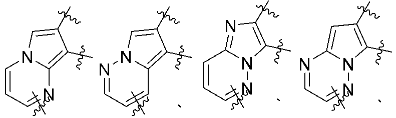

In some embodiments, wherein Ring B is selected from the following groups: 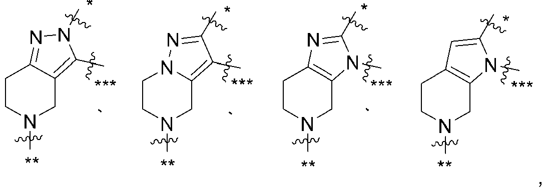 wherein a "*" end is an end connected to L₁, a "**" end is an end connected to L₂, and a "***" end is an end connected to L₄;
or Ring B is selected from the following groups: 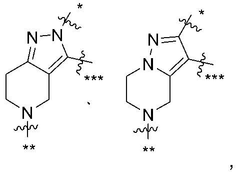 wherein a "*" end is an end connected to L₁, a "**" end is an end connected to L₂, and a "***" end is an end connected to L₄;
or Ring B is selected from 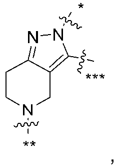 wherein a "*" end is an end connected to L₁, a "**" end is an end connected to L₂, and a "***" end is an end connected to L₄.

In some embodiments, wherein R^{b} is independently selected from deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₄ alkenyl, C₂₋₄ alkynyl, C₃₋₆ cycloalkyl, 3-6 membered heterocyclyl, C₆₋₈ aryl, and 5-6 membered heteroaryl; optionally substituted means unsubstituted or one or more (for example, 2, 3, 4, 5, 6, 7, or 8) substitutable sites of a substituted group each independently substituted with R^{b1};
or R^{b} is independently selected from halogen, hydroxy, amino, mercapto, cyano, oxo, or the following optionally substituted groups: C₁₋₆ alkyl and C₁₋₆ alkoxy; optionally substituted means unsubstituted or one or more (for example, 2, 3, 4, 5, 6, 7, or 8) substitutable sites of a substituted group each independently substituted with R^{b1};
or R^{b} is independently selected from halogen, hydroxy, amino, cyano, or the following optionally substituted groups: C₁₋₄ alkyl and C₁₋₄ alkoxy; optionally substituted means unsubstituted or one or more (for example, 2, 3, 4, or 5) substitutable sites of a substituted group each independently substituted with R^{b1};
or R^{b} is independently selected from C₁₋₄ alkyl (preferably C₁₋₃ alkyl);
or R^{b} is independently selected from methyl.

In some embodiments, wherein two R^{b}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₃₋₆ cycloalkyl, 3-6 membered heterocyclyl, C₆₋₈ aryl, and 5-6 membered heteroaryl;
or two R^{b}, together with the atoms to which they are attached, form an optionally substituted 3-7 membered heterocyclyl or an optionally substituted 5-6 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, methyl, ethyl, methoxy, and ethoxy.

In some embodiments, wherein R^{b1} is independently selected from deuterium, halogen, hydroxy, amino, nitro, mercapto, cyano, oxo, -R^{b2}, -OR^{b2}, -C(O)R^{b2}, -C(O)OR^{b2}, -OC(O)R^{b2}, -N(R^{b2})(R^{b3}), -C(O)N(R^{b2})(R^{b3}), -N(R^{b2})C(O)(R^{b3});
or R^{b1} is independently selected from halogen, hydroxy, amino, and -R^{b2};
or R^{b1} is independently selected from halogen (for example, fluoro, chloro, or bromo).

In some embodiments, wherein R^{b2} and R^{b3} are respectively independently selected from hydrogen and the following optionally substituted groups: C₁₋₆ alkyl, C₁₋₆ alkoxy, C₃₋₆ cycloalkyl, 3-6 membered heterocyclyl, C₆₋₈ aryl, and 5-6 membered heteroaryl; optionally substituted means unsubstituted or substituted with one or more (for example, 2, 3, 4, 5, 6, 7, or 8) substituents selected from halogen, hydroxy, amino, cyano, C₁₋₆ alkyl, and C₁₋₆ alkoxy;
or R^{b2} and R^{b3} are respectively independently selected from hydrogen and the following optionally substituted groups: C₁₋₄ alkyl and C₁₋₄ alkoxy; optionally substituted means unsubstituted or substituted with one or more (for example, 2, 3, 4, or 5) substituents selected from halogen, hydroxy, amino, and cyano; or R^{b2} and R^{b3} are respectively independently selected from hydrogen, methyl, ethyl, n-propyl, isopropyl, methoxy, trifluoromethyl, difluoromethyl, monofluoromethyl, trichloromethyl, dichloromethyl, and monochloromethyl.

In some embodiments, wherein n is independently selected from 0, 1, or 2; preferably, n is independently selected from 0 or 1; more preferably, n is 1.

In some embodiments, wherein L₂ is independently selected from a bond, -C(R^{L2})₂-, -O-, -C(R^{L2})₂O-, -C(O)-, -N(R^{L2})C(O)-, and -C(O)N(R^{L2})-; R^{L2} is selected from hydrogen or the following optionally substituted groups: C₁₋₆ alkyl and C₁₋₆ alkoxy; optionally substituted means unsubstituted or substituted with one or more (for example, 2, 3, 4, 5, 6, 7, or 8) groups selected from deuterium, halogen, hydroxy, amino, cyano, C₁₋₄ alkyl, C₁₋₄ alkoxy, C₃₋₆ cycloalkyl, and 3-6 membered heterocyclyl;
or L₂ is independently selected from a bond, -C(R^{L2})₂-, -C(R^{L2})₂O-, and -C(O)-; R^{L2} is selected from hydrogen or C₁₋₄ alkyl optionally substituted with one or more (for example, 2, 3, or 4) groups selected from halogen, hydroxy, amino, and cyano;
or L₂ is independently selected from a bond, -C(R^{L2})₂-, -C(O)-; R^{L2} is selected from hydrogen or methyl and ethyl optionally substituted with one or more (for example, 2 or 3) groups selected from fluoro, chloro, bromo, hydroxy, amino, and cyano.

In some embodiments, wherein L₂ is independently selected from a bond, -CH₂-, -CH₂O-, -O-, -C(O)-, - NHC(O)-, -C(O)NH-;
or L₂ is independently selected from a bond, -CH₂-, and -C(O)-;
or L₂ is -C(O)-.

In some embodiments, wherein Ring C is independently selected from 9-membered bicyclic heteroaryl (for example, a bicyclic ring composed of heteroaryl and heteroaryl, or a bicyclic ring composed of aryl and heteroaryl); the heteroatoms in the 9-membered bicyclic heteroaryl are N, and the number of the heteroatoms is 1, 2, or 3;
or Ring C is independently selected from 5-membered/6-membered fused heteroaryl (for example, a bicyclic ring formed by fusion of heteroaryl and heteroaryl, or a bicyclic ring formed by fusion of aryl and heteroaryl), the heteroatoms in the 5-membered/6-membered fused heteroaryl are selected from N, and the number of the heteroatoms is 1 or 2;
or Ring C is independently selected from the following groups: 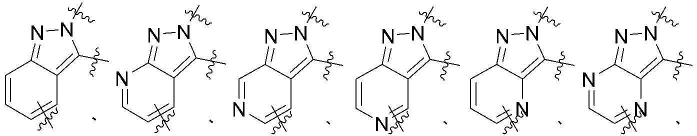 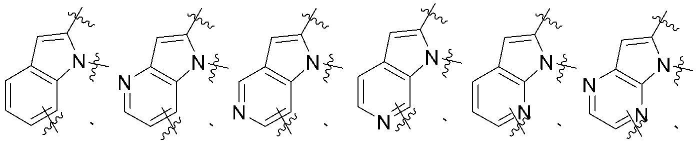 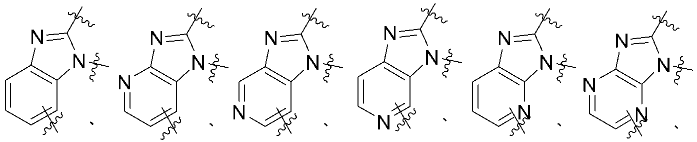 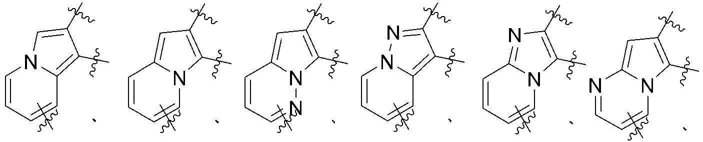 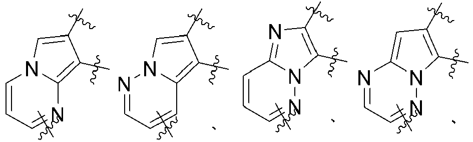
or Ring C is selected from the following groups: 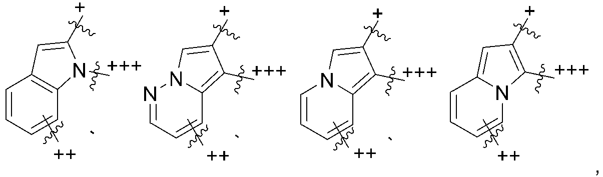 wherein a "+" end is an end connected to L₂, a "++" end is an end connected to L₃, and a "+++" end is an end connected to L₅ₓ;
or Ring C is independently selected from the following groups: 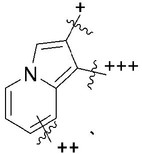 and 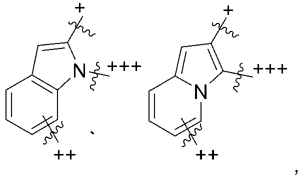 wherein a "+" end is the end connected to L₂, a "++" end is the end connected to L₃, and a "+++" end is the end connected to L₅ₓ.
or Ring C is selected from the following groups: 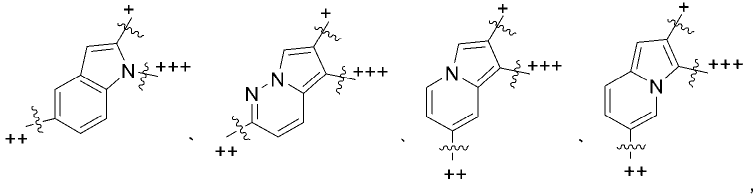 wherein a "+" end is an end connected to L₂, a "++" end is an end connected to L₃, and a "+++" end is an end connected to L₅ₓ;

In some embodiments, Ring C is independently selected from 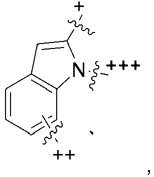 wherein a "+" end is an end connected to L₂, a "++" end is an end connected to L₃, and a "+++" end is an end connected to L₅ₓ.

In some embodiments, Ring C is 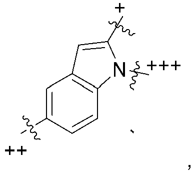 wherein a "+" end is an end connected to L₂, a "++" end is an end connected to L₃, and a "+++" end is an end connected to L₅ₓ.

In some embodiments, wherein R^{c} is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₄ alkenyl, C₂₋₄ alkynyl, C₃₋₆ cycloalkyl, 3-6 membered heterocyclyl, phenyl, and 5-6 membered heteroaryl; optionally substituted means unsubstituted or one or more (for example, 2, 3, 4, 5, 6, 7, or 8) substitutable sites of a substituted group each independently substituted with R^{c1};
or R^{c} is independently selected from hydrogen, halogen, hydroxy, amino, cyano, oxo, or the following optionally substituted groups: C₁₋₆ alkyl and C₁₋₆ alkoxy (preferably C₁₋₃ alkoxy, for example, methoxy and ethoxy); optionally substituted means unsubstituted or one or more (for example, 2, 3, 4, 5, or 6) substitutable sites of a substituted group independently substituted with R^{c1};
or R^{c} is independently selected from hydrogen, halogen, hydroxy, amino, cyano, oxo, or optionally substituted C₁₋₄ alkyl; optionally substituted means unsubstituted or one or more (for example, 2 or 3) substitutable sites of a substituted group independently substituted with R^{c1};
or R^{c} is independently selected from hydrogen, halogen, hydroxy, amino, cyano, oxo, and C₁₋₄ alkyl (preferably C₁₋₃ alkyl);
or R^{c} is independently selected from hydrogen, fluoro, chloro, oxo, methyl, ethyl, n-propyl, isopropyl, and n-butyl;
or R^{c} is independently selected from hydrogen, fluoro, and chloro;
or R^{c} is independently selected from hydrogen.

In some embodiments, wherein two R^{c}, together with the atoms to which they are attached, form an optionally substituted 3-6 membered heterocyclyl or an optionally substituted 5-6 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl (preferably C₁₋₃ alkyl), C₁₋₆ alkoxy (preferably C₁₋₃ alkoxy), C₃₋₆ cycloalkyl, 3-6 membered heterocyclyl, phenyl, and 5-6 membered heteroaryl;
or two R^{c}, together with the atoms to which they are attached, form an optionally substituted 3-6 membered heterocyclyl or an optionally substituted 5-6 membered heteroaryl, and optionally substituted means unsubstituted or substituted with one or more (for example, 2, 3, 4, 5, 6, 7, or 8) groups selected from halogen, hydroxy, amino, cyano, oxo, methyl, ethyl, methoxy, and ethoxy.

In some embodiments, wherein R^{c1} is independently selected from deuterium, halogen, hydroxy, amino, nitro, mercapto, cyano, oxo, -R^{c2}, -OR^{c2}, -C(O)R^{c2}, -C(O)OR^{c2}, -OC(O)R^{c2}, -N(R^{c2})(R^{c3}), -C(O)N(R^{c2})(R^{c3}), and -N(R^{c2})C(O)R^{c3};
or R^{c1} is independently selected from halogen, hydroxy, amino, nitro, mercapto, cyano, oxo, -R^{c2}, and - OR^{c2};
or R^{c1} is independently selected from halogen (for example, fluoro, chloro, or bromo).

In some embodiments, wherein R^{c2} and R^{c3} are respectively independently selected from hydrogen and the following optionally substituted groups: C₁₋₄ alkyl, C₁₋₄ alkoxy, C₂₋₄ alkenyl, C₂₋₄ alkynyl, C₃₋₆ cycloalkyl, 3-6 membered heterocyclyl, C₆₋₈ aryl, and 5-6 membered heteroaryl; optionally substituted means unsubstituted or substituted with one or more (for example, 2, 3, 4, 5, 6, 7, or 8) groups selected from halogen, hydroxy, amino, cyano, C₁₋₆ alkyl, and C₁₋₆ alkoxy;
or R^{c2} and R^{c3} are respectively independently selected from hydrogen and the following optionally substituted groups: C₁₋₄ alkyl (preferably C₁₋₃ alkyl), and C₁₋₄ alkoxy (preferably C₁₋₃ alkoxy); optionally substituted means unsubstituted or substituted with one or more (for example, 2, 3, or 4) groups selected from halogen, hydroxy, amino, and cyano;
or R^{c2} and R^{c3} are respectively independently selected from hydrogen, methyl, ethyl, n-propyl, isopropyl, methoxy, trifluoromethyl, difluoromethyl, monofluoromethyl, trichloromethyl, dichloromethyl, and monochloromethyl.

In some embodiments, wherein o is independently selected from 0, 1, or 2; preferably, o is independently selected from 0 or 1; preferably, o is 0.

In some embodiments, wherein L₃ is independently selected from a bond, -C(R^{L3})₂-, -O-, -C(O)-, - N(R^{L3})C(O)-, and -C(O)N(R^{L3})-; R^{L3} is selected from hydrogen or the following optionally substituted groups: C₁₋₆ alkyl and C₁₋₆ alkoxy; optionally substituted means unsubstituted or substituted with one or more (for example, 2, 3, 4, 5, 6, 7, or 8) groups selected from deuterium, halogen, hydroxy, amino, cyano, C₁₋₆ alkyl, C₁₋₆ alkoxy, C₃₋₆ cycloalkyl, and 3-6 membered heterocyclyl;
or L₃ is independently selected from a bond, -C(R^{L3})₂-, and -C(O)-; R^{L3} is selected from hydrogen or optionally substituted C₁₋₄ alkyl, and optionally substituted means unsubstituted or substituted with one or more (for example, 2, 3, or 4) groups selected from halogen, hydroxy, amino, and cyano.
or L₃ is independently selected from a bond, -C(R^{L3})₂-, and -C(O)-; R^{L3} is selected from hydrogen, methyl, ethyl, n-propyl, isopropyl, and n-butyl.

In some embodiments, wherein L₃ is independently selected from a bond, -CH₂-, -CH(CH₃)-, -O-, -C(O)-, - NHC(O)-, and -C(O)NH-;
or L₃ is independently selected from a bond, -CH₂-, and -C(O)-;
or L₃ is independently selected from a bond.

In some embodiments, wherein Ring D is selected from C₃₋₁₀ carbocyclyl, 3-10 membered heterocyclyl, C₆₋₁₀ aryl, and 5-10 membered heteroaryl;
or Ring D is selected from C₃₋₈ carbocyclyl, 3-8 membered heterocyclyl, C₆₋₈ aryl, and 5-8 membered heteroaryl;
or Ring D is selected from C₃₋₆ carbocyclyl, 3-6 membered heterocyclyl, phenyl, and 5-6 membered heteroaryl; the heteroatoms in the heterocyclyl and heteroaryl are selected from N, O, or S, and the number of the heteroatoms is 1, 2, or 3.

In some embodiments, wherein Ring D is independently selected from C₃₋₁₀ carbocyclyl;
or Ring D is independently selected from C₃₋₆ carbocyclyl.

In some embodiments, wherein Ring D is independently selected from 3-10 membered heterocyclyl;
or Ring D is independently selected from 3-6 membered heterocyclyl.

In some embodiments, wherein Ring D is independently selected from 4-6 membered heterocyclyl; the heteroatoms in the heterocyclyl are selected from N or O, and the number of the heteroatoms is 1 or 2.

In some embodiments, wherein Ring D is selected from tetrahydropyranyl, morpholinyl, piperidinyl, pyrazinyl, and tetrahydrofuranyl;
or Ring D is selected from tetrahydropyranyl.

In some embodiments, wherein Ring D is independently selected from 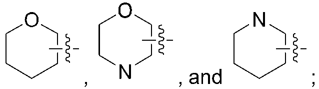 or Ring D is independently selected from

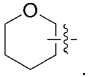

In some embodiments, wherein Ring D is independently selected from 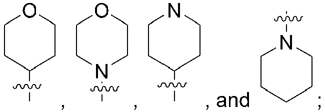 or Ring D is independently selected from

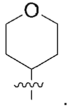

In some embodiments, wherein Ring D is independently selected from C₆₋₁₀ aryl and 5-10 membered heteroaryl;
or Ring D is independently selected from C₆₋₈ aryl and 5-8 membered heteroaryl, the heteroatoms in the heteroaryl are selected from N, O, or S, and the number of the heteroatoms is 1 or 2;
or Ring D is independently selected from phenyl, 5-membered heteroaryl, and 6-membered heteroaryl, the heteroatoms in the heteroaryl are selected from N, O, or S, and the number of heteroatoms is 1 or 2;
or Ring D is independently selected from phenyl, pyridyl, pyrrolyl, furyl, and thienyl;
or Ring D is independently selected from pyridyl.

In some embodiments, wherein R^{d} is independently selected from deuterium, halogen, hydroxy, amino, nitro, mercapto, cyano, oxo, -C(O)R^{d1}, -C(O)OR^{d1}, -OC(O)R^{d1}, -N(R^{d1})(R^{d2}), -C(O)N(R^{d1})(R^{d2}),-N(R^{d1})C(O)(R^{d2}), and the following groups optionally substituted with one or more (for example, 2, 3, 4, 5, 6, 7, or 8) R^{d3}: C₁₋₆ alkyl, C₁₋₆ alkoxy, C₁₋₆ alkylamino, C₂₋₄ alkenyl, C₂₋₄ alkynyl, C₃₋₆ cycloalkyl, 3-6 membered heterocyclyl, C₆₋₈ aryl, and 5-6 membered heteroaryl;
or R^{d} is independently selected from halogen, hydroxy, amino, nitro, mercapto, cyano, oxo, -N(R^{d1})(R^{d2}), - C(O)N(R^{d1})(R^{d2}), -N(R^{d1})C(O)(R^{d2}), and the following groups optionally substituted with one or more (for example, 2, 3, 4, 5, 6, 7, or 8) R^{d3}: C₁₋₆ alkyl, C₁₋₆ alkoxy, C₃₋₆ cycloalkyl, and 3-6 membered heterocyclyl; the heteroatoms in the heterocyclyl are O or N, and the number of the heteroatoms is 1 or 2;
or R^{d} is independently selected from halogen, hydroxy, amino, cyano, and the following groups optionally substituted with one or more (for example, 2 or 3) R^{d3}: C₁₋₄ alkyl, C₁₋₄ alkoxy, C₃₋₄ cycloalkyl, and 4-5 membered heterocyclyl; the heteroatoms in the heterocyclyl are O or N, and the number of the heteroatoms is 1 or 2;
or R^{d} is independently selected from halogen, hydroxy, amino, cyano, and the following optionally substituted groups: C₁₋₄ alkyl (preferably C₁₋₃ alkyl), C₁₋₄ alkoxy (preferably C₁₋₃ alkoxy), C₃₋₄ cycloalkyl, and 4-5 membered heterocyclyl; optionally substituted means unsubstituted or substituted with one or more (for example, 2 or 3) substituents selected from halogen, hydroxy, and amino; the heteroatom in the heterocyclyl is O, and the number of heteroatoms is 1.

In some embodiments, wherein R^{d} is independently selected from halogen, hydroxy, amino, nitro, mercapto, cyano, oxo, -N(R^{d1})(R^{d2}), -C(O)N(R^{d1})(R^{d2}), -N(R^{d1})C(O)(R^{d2}), and the following groups optionally substituted with one or more (for example, 2, 3, 4, 5, 6, 7, or 8) R^{d3}: C₁₋₆ alkyl and C₁₋₆ alkoxy;
or R^{d} is independently selected from halogen, hydroxy, amino, cyano, and the following groups optionally substituted with one or more (for example, 2, 3, or 4) R^{d3}: C₁₋₄ alkyl and C₁₋₄ alkoxy;
or R^{d} is independently selected from halogen, hydroxy, amino, cyano, and the following optionally substituted groups: C₁₋₄ alkyl (preferably C₁₋₃ alkyl), and C₁₋₄ alkoxy (preferably C₁₋₃ alkoxy); optionally substituted means unsubstituted or substituted with one or more (for example, 2 or 3) substituents selected from halogen, hydroxy, and amino.

In some embodiments, wherein R^{d} is independently selected from halogen, hydroxy, amino, cyano, and the following optionally substituted groups: methyl, ethyl, n-propyl, isopropyl, n-butyl, methoxy, and ethoxy; optionally substituted means unsubstituted or substituted with one or more (for example, 2 or 3) substituents selected from halogen, hydroxy, and amino;
preferably, R^{d} is independently selected from methyl, methoxy, and ethoxy.

In some embodiments, wherein two R^{d}, together with the atoms to which they are attached, form an optionally substituted 3-10 membered heterocyclyl, an optionally substituted 3-7 membered cycloalkyl, or an optionally substituted 5-12 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₃₋₆ cycloalkyl, 3-6 membered heterocyclyl, C₆₋₈ aryl, and 5-6 membered heteroaryl;
or two R^{d}, together with the atoms to which they are attached, form an optionally substituted 3-6 membered heterocyclyl (for example, 3, 4, 5, or 6-membered heterocyclyl), an optionally substituted 3-6 membered cycloalkyl (for example, 3, 4, 5, or 6-membered cycloalkyl), or an optionally substituted 5-6 membered heteroaryl, and optionally substituted means unsubstituted or substituted with one or more (for example, 2, 3, 4, 5, 6, 7, or 8) groups selected from halogen, hydroxy, amino, nitro, mercapto, cyano, oxo, methyl, ethyl, n-propyl, isopropyl, n-butyl, methoxy, and ethoxy.

In some embodiments, wherein R^{d1} and R^{d2} are respectively independently selected from hydrogen, deuterium, and the following optionally substituted groups: C₁₋₆ alkyl (preferably C₁₋₃ alkyl), C₁₋₆ alkoxy (preferably C₁₋₃ alkoxy), C₃₋₆ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₃₋₆ cycloalkyl, 3-6 membered heterocyclyl, phenyl, and 5-6 membered heteroaryl;
or R^{d1} and R^{d2} are respectively independently selected from hydrogen and C₁₋₆ alkyl (preferably C₁₋₃ alkyl); the C₁₋₆ alkyl (preferably C₁₋₃ alkyl) is optionally substituted with one or more (for example, 2, 3, or 4) substituents selected from hydrogen, halogen, hydroxy, and amino;
or R^{d1} and R^{d2} are respectively independently selected from hydrogen.

In some embodiments, wherein R^{d3} is independently selected from hydrogen, deuterium, halogen, hydroxy, amino, nitro, mercapto, cyano, oxo, -R^{d4}, -OR^{d4}, -C(O)R^{d4}, -C(O)OR^{d4}, -OC(O)R^{d4}, -N(R^{d4})(R^{d5}),-C(O)N(R^{d4})(R^{d5}), -N(R^{d4})C(O)R^{d5}.

In some embodiments, wherein R^{d3} is independently selected from hydrogen, halogen, hydroxy, amino, and -R^{d4}; R^{d4} is independently selected from C₁₋₆ alkyl and C₁₋₆ alkoxy; the C₁₋₆ alkyl and C₁₋₆ alkoxy are optionally substituted with one or more (for example, 2, 3, 4, 5, 6, 7, or 8) substituents selected from hydrogen and halogen;
or R^{d3} is independently selected from hydrogen, halogen, hydroxy, amino, and -R^{d4}; R^{d4} is independently selected from C₁₋₄ alkyl and C₁₋₄ alkoxy; the C₁₋₄ alkyl and C₁₋₄ alkoxy are optionally substituted with one or more (for example, 2, 3, 4, or 5) substituents selected from hydrogen and halogen;
or R^{d3} is independently selected from hydrogen, halogen, hydroxy, amino, and -R^{d4}; R^{d4} is independently selected from C₁₋₃ alkyl and C₁₋₃ alkoxy; the C₁₋₃ alkyl and C₁₋₃ alkoxy are optionally substituted with one or more (for example, 2 or 3) substituents selected from hydrogen and halogen.

In some embodiments, wherein R^{d3} is independently selected from hydrogen and halogen;
or R^{d3} is independently selected from hydrogen, fluoro, and chloro.

In some embodiments, wherein R^{d4} and R^{d5} are respectively independently selected from hydrogen and the following optionally substituted groups: C₁₋₄ alkyl, C₁₋₄ alkoxy, C₃₋₆ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from halogen, hydroxy, amino, cyano, methyl, ethyl, n-propyl, isopropyl, n-butyl, methoxy, and ethoxy;
or R^{d4} and R^{d5} are respectively independently selected from hydrogen and C₁₋₄ alkyl. the C₁₋₄ alkyl is optionally substituted with one or more (for example, 2, 3, or 4) substituents selected from hydrogen and halogen;
preferably, R^{d4} and R^{d5} are respectively independently selected from hydrogen and C₁₋₃ alkyl; the C₁₋₃ alkyl is optionally substituted with one or more (for example, 2 or 3) substituents selected from hydrogen and halogen.

In some embodiments, wherein R^{d4} and R^{d5} are respectively independently selected from hydrogen, methyl, ethyl, n-propyl, isopropyl, methoxy, trifluoromethyl, difluoromethyl, monofluoromethyl, trichloromethyl, dichloromethyl, and monochloromethyl.

In some embodiments, wherein p is independently selected from 0, 1, or 2;
or p is independently selected from 1 or 2;
or p is 2.

In some embodiments, wherein L₄ is selected from a bond, -L₄ₓ-O-L_{4y}-, -L₄ₓ-S-L_{4y}-, -L₄ₓ-C(O)-L_{4y}-, , -L₄ₓ-N(R^{L4a})C(O)-L_{4y}-, -L₄ₓ-C(O)N(R^{L4a})-L_{4y}-, -L₄ₓ-N(R^{L4a})-L_{4y}-, -L₄ₓ-N(R^{L4a})C(O) N(R^{L4b})-L_{4y}-, and -L₄ₓ-N(R^{L4a})C(S) N(R^{L4b})-L_{4y}-; L₄ₓ and L_{4y} are respectively independently selected from a bond or optionally substituted C₁₋₆ alkylene (preferably C₁₋₃ alkylene), and optionally substituted means unsubstituted or one or more (for example, 2, 3, 4, 5, 6, 7, or 8) substitutable sites of a substituted group independently substituted with a substituent selected from deuterium, halogen, hydroxy, amino, cyano, oxo, C₁₋₄ alkyl, C₁₋₄ alkoxy, C₃₋₁₆ cycloalkyl, and 3-6 membered heterocyclyl; R^{L4a} and R^{L4b} are respectively independently selected from hydrogen or the following optionally substituted groups: C₁₋₆ alkyl (preferably C₁₋₃ alkyl), C₁₋₆ alkoxy (preferably C₁₋₃ alkoxy), and C₃₋₆ cycloalkyl; optionally substituted means unsubstituted or one or more (for example, 2, 3, 4, 5, 6, 7, or 8) substitutable sites of a substituted group independently substituted with a substituent selected from deuterium, halogen, hydroxy, amino, cyano, C₁₋₄ alkyl, C₁₋₄ alkoxy, C₃₋₆ cycloalkyl, and 3-6 membered heterocyclyl.

In some embodiments, wherein L₄ is selected from -L₄ₓ-N(R^{L4a})-L_{4y}-, -L₄ₓ-N(R^{L4a})C(O) N(R^{L4b})-L_{4y}-, and -L₄ₓ-N(R^{L4a})C(S) N(R^{L4b})-L_{4y}-; L₄ₓ and L_{4y} are respectively independently selected from a bond, or optionally substituted methylene or ethylene, and optionally substituted means unsubstituted or one or more (for example, 2, 3, 4, 5, 6, 7, or 8) substitutable sites of a substituted group independently substituted with a substituent selected from fluoro, chloro, bromo, hydroxy, amino, cyano, and oxo; R^{L4a} and R^{L4b} are respectively independently selected from hydrogen, or optionally substituted methyl, ethyl, n-propyl, isopropyl, n-butyl, cyclopropyl, or cyclobutyl, and optionally substituted means unsubstituted or one or more (for example, 2, 3, 4, 5, 6, 7, or 8) substitutable sites of a substituted group independently substituted with a substituent selected from fluoro, chloro, bromo, hydroxy, amino, and cyano.

In some embodiments, wherein L₄ is selected from -NHC(O)NH-, -N(cyclopropyl)C(O)NH-, - N(cyclopropyl)C(O)N(cyclopropyl)-, -N(methyl)C(O)NH-, -N(methyl)C(O)N(methyl)-, -NHC(S)NH-, and - NH-;
or L₄ is selected from -NHC(O)NH-, -N(cyclopropyl)C(O)NH-, -N(cyclopropyl)C(O)N(cyclopropyl)-, and - N(methyl)C(O)NH-;
or L₄ is selected from -NHC(O)NH-.

In some embodiments, wherein L₄ is selected from 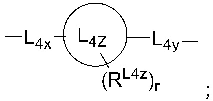 Ring L_{4Z} is independently selected from C₃₋₁₀ carbocyclyl, 5-10 membered heterocyclyl, C₆₋₈ aryl, and 5-10 membered heteroaryl; L₄ₓ and L_{4y} are respectively independently selected from a bond or optionally substituted C ₁₋₆ alkylene (preferably C₁₋₃ alkylene, more preferably C₁₋₂ alkylene); optionally substituted means unsubstituted or one or more (for example, 2, 3, 4, 5, 6, 7, or 8) substitutable sites of a substituted group independently substituted with a group selected from deuterium, halogen, hydroxy, amino, cyano, oxo, C₁₋₄ alkyl, C₁₋₄ alkoxy, C₃₋₆ cycloalkyl, and 3-6 membered heterocyclyl; R^{L4z} is independently selected from hydrogen, halogen, oxo, or the following optionally substituted groups: C₁₋₆ alkyl and C₁₋₆ alkoxy; optionally substituted means unsubstituted or one or more (for example, 2, 3, 4, 5, 6, 7, or 8) substitutable sites of a substituted group independently substituted with a group selected from deuterium, halogen, hydroxy, amino, cyano, C₁₋₄ alkyl, C₁₋₄ alkoxy, C₃₋₆ cycloalkyl, and 3-6 membered heterocyclyl; r is independently selected from 0, 1, or 2.

In some embodiments, wherein L₄ is selected from 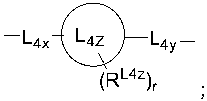 Ring L_{4Z} is selected from 5-10 membered heterocyclyl, phenyl, 6-8 membered monocyclic heteroaryl, and 8-10 membered bicyclic heteroaryl; L₄ₓ and L_{4y} are respectively independently selected from a bond or the following optionally substituted groups: methylene and ethylene, and optionally substituted means unsubstituted or one or more (for example, 2 or 3) substitutable sites of a substituted group independently substituted with a group selected from fluoro, chloro, bromo, hydroxy, amino, cyano, and oxo; R^{L4z} is independently selected from hydrogen, halogen, oxo, or the following optionally substituted groups: C₁₋₄ alkyl and C₁₋₄ alkoxy, and optionally substituted means unsubstituted or one or more (for example, 2 or 3) substitutable sites of a substituted group independently substituted with a group selected from halogen, hydroxy, amino, and cyano; r is independently selected from 0 or 1.

In some embodiments, wherein L₄ is selected from 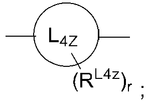 Ring L_{4Z} is selected from 5-10 membered heterocyclyl (preferably 5-6 membered heterocyclyl), phenyl, and 6-8 membered monocyclic heteroaryl; R^{L4z} is independently selected from hydrogen, fluoro, chloro, bromo, oxo, methyl, ethyl, n-propyl, isopropyl, methoxy, trifluoromethyl, difluoromethyl, monofluoromethyl, trichloromethyl, dichloromethyl, and monochloromethyl; r is independently selected from 1.

In some embodiments, wherein L₄ is selected from 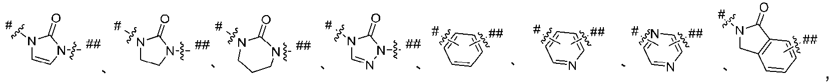 wherein a "#" end is the end connected to Ring B, and a "##" end is the end connected to Ring E;
or L₄ is independently selected from 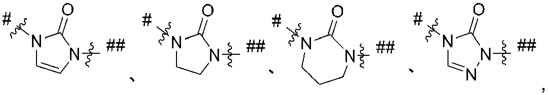 wherein a "#" end is the end connected to Ring B, and a "##" end is the end connected to Ring E;
or L₄ is independently selected from 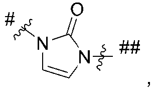 wherein a "#" end is the end connected to Ring B, and a "##" end is the end connected to Ring E.

In some embodiments, L₄ is -NHC(O)NH-, -N(methyl)C(O)NH-, -N(cyclopropyl)C(O)NH-, - N(cyclopropyl)C(O)N(cyclopropyl)-, or 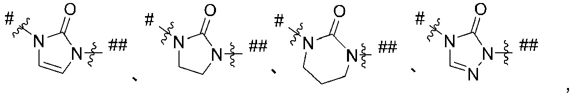 wherein a "#" end is the end connected to Ring B, and a "##" end is the end connected to Ring E.

In some embodiments, L₄ is -NHC(O)NH- or 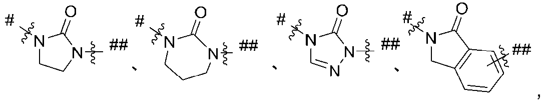 wherein a "#" end is the end connected to Ring B, and a "##" end is the end connected to Ring E.

In some embodiments, wherein Ring E is independently selected from C₃₋₁₀ carbocyclyl, 3-10 membered heterocyclyl, C₆₋₁₀ aryl, and 5-10 membered heteroaryl;
or Ring E is independently selected from C₃₋₈ carbocyclyl, 3-8 membered heterocyclyl, phenyl, naphthyl, and 5-10 membered heteroaryl;
or Ring E is independently selected from C₃₋₆ carbocyclyl, 3-6 membered heterocyclyl, phenyl, naphthyl, and 5-10 membered heteroaryl; the heteroatoms in the heterocyclyl and heteroaryl are selected from N, O, or S, and the number of the heteroatoms is 1, 2, or 3.

In some embodiments, wherein Ring E is independently selected from C₃₋₁₀ carbocyclyl;
or Ring E is independently selected from C₃₋₆ carbocyclyl.

In some embodiments, wherein Ring E is independently selected from 3-10 membered heterocyclyl;
or Ring E is independently selected from 3-6 membered heterocyclyl.

In some embodiments, wherein Ring E is independently selected from C₆₋₁₀ aryl and 5-10 membered heteroaryl;
or Ring E is independently selected from phenyl, naphthyl, and 5-10 membered heteroaryl, wherein the heteroatoms in the heteroaryl are selected from N, O, or S, and the number of the heteroatoms is 1, 2, or 3;
or Ring E is independently selected from phenyl, naphthyl, and 8-9 membered bicyclic heteroaryl; the heteroatoms in the heteroaryl are selected from N, O, or S, and the number of the heteroatoms is 1, 2, or 3;
or Ring E is independently selected from 9-membered bicyclic heteroaryl; the heteroatoms in the heteroaryl are selected from N, O, or S, and the number of the heteroatoms is 1, 2, or 3.

In some embodiments, wherein Ring E is independently selected from phenyl, naphthyl, 5-membered heteroaryl, 6-membered heteroaryl, 5-membered/5-membered fused heteroaryl, 5-membered/6-membered fused heteroaryl, 6-membered/5-membered fused heteroaryl, and 6-membered/6-membered fused heteroaryl; the heteroatoms in the heteroaryl are selected from N, O, or S, and the number of the heteroatoms is 1 or 2;
or Ring E is independently selected from 5-membered/6-membered fused heteroaryl and 6-membered/5-membered fused heteroaryl; the heteroatoms in the heteroaryl are selected from N, O, or S, and the number of the heteroatoms is 1 or 2.

In some embodiments, wherein Ring E is independently selected from phenyl, naphthyl, pyridyl, pyrrolyl, furyl, thienyl, benzimidazolyl, benzofuryl, benzothienyl, benzoxazolyl, benzothiazolyl, indolyl, and indazolyl; or Ring E is independently selected from indazolyl.

In some embodiments, wherein Ring E is independently selected from 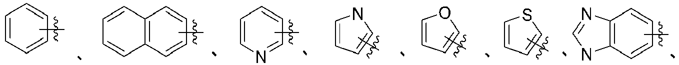 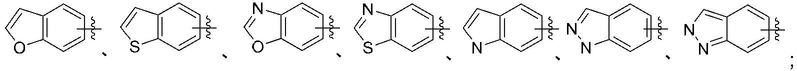 or Ring E is independently selected from

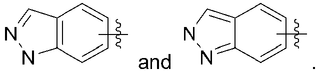

In some embodiments, wherein Ring E is independently selected from 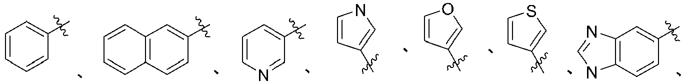 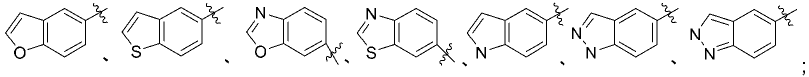
or Ring E is independently selected from 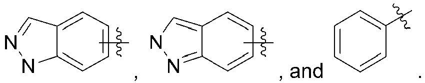
or Ring E is independently selected from

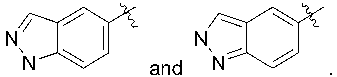

In some embodiments, wherein R^{e} is independently selected from deuterium, halogen, hydroxy, amino, nitro, cyano, -OR^{e1}, -SO₂(R^{e1}), -C(O)R^{e1}, -C(O)OR^{e1}, -OC(O)R^{e1}, -N(R^{e1})(R^{e2}), -C(O)N(R^{e1})(R^{e2}), - N(R^{e1})C(O)(R^{e2}), -S(O)N(R^{e1})(R^{e2}), -SO₂N(R^{e1})(R^{e2}), -N(R^{e1})SO₂(R^{e2}), -P(O)(R^{e1})(R^{e2}), or the following optionally substituted groups: C₁₋₆ alkyl, C₁₋₆ alkoxy, C₁₋₆ alkylamino, C₂₋₄ alkenyl, C₂₋₄ alkynyl, C₃₋₆ cycloalkyl (preferably C₃₋₅ cycloalkyl), 3-6 membered heterocyclyl, phenyl, and 5-6 membered heteroaryl; optionally substituted means unsubstituted or one or more (for example, 2, 3, 4, 5, 6, 7, or 8) substitutable sites of a substituted group independently substituted with R^{e3};
or R^{e} is independently selected from deuterium, halogen, hydroxy, amino, cyano, -OR^{e1}, -N(R^{e1})(R^{e2}), - SO₂(R^{e1}), -C(O)R^{e1}, -SO₂N(R^{e1})(R^{e2}), -N(R^{e1})SO₂(R^{e2}), -P(O)(R^{e1})(R^{e2}), or the following optionally substituted groups: C₁₋₆ alkyl, C₁₋₆ alkoxy, C₃₋₆ cycloalkyl (preferably C₃₋₅ cycloalkyl), and 3-6 membered heterocyclyl; optionally substituted means unsubstituted or one or more (for example, 2, 3, 4, 5, 6, 7, or 8) substitutable sites of a substituted group independently substituted with R^{e3}; the heteroatoms in the heterocyclyl are O or N, and the number of the heteroatoms is 1 or 2;
or R^{e} is independently selected from halogen, hydroxy, amino, cyano, -OR^{e1}, -N(R^{e1})(R^{e2}), -SO₂(R^{e1}), - P(O)(R^{e1})(R^{e2}), or the following optionally substituted groups: C₁₋₄ alkyl, C₁₋₄ alkoxy, C₃₋₄ cycloalkyl, and 4-5 membered heterocyclyl; optionally substituted means unsubstituted or one or more (for example, 2, 3, 4, 5, 6, 7, or 8) substitutable sites of a substituted group independently substituted with R^{e3}; the heteroatoms in the heterocyclyl are O or N, and the number of the heteroatoms is 1 or 2;
or R^{e} is independently selected from halogen, hydroxy, amino, cyano, -N(R^{e1})(R^{e2}), -P(O)(R^{e1})(R^{e2}), or the following optionally substituted groups: C₁₋₄ alkyl, preferably C₁₋₃ alkyl, C₁₋₄ alkoxy, C₃₋₄ cycloalkyl, and 4-5 membered heterocyclyl; optionally substituted means unsubstituted or one or more (for example, 2, 3, or 4) substitutable sites of a substituted group independently substituted with a substituent selected from halogen, hydroxy, and amino; the heteroatom in the heterocyclyl is O, and the number of heteroatoms is 1. In some embodiments, wherein R^{e} is independently selected from halogen, hydroxy, amino, cyano, - N(R^{e1})(R^{e2}), -P(O)(R^{e1})(R^{e2}), or the following optionally substituted groups: C₁₋₆ alkyl, C₁₋₆ alkoxy, and C₃₋₆ cycloalkyl; optionally substituted means unsubstituted or one or more (for example, 2, 3, 4, 5, 6, 7, or 8) substitutable sites of a substituted group independently substituted with R^{e3};
or R^{e} is independently selected from halogen, hydroxy, amino, cyano, -N(R^{e1})(R^{e2}), -P(O)(R^{e1})(R^{e2}), or the following optionally substituted groups: C₁₋₄ alkyl, C₁₋₄ alkoxy, and C₃₋₄ cycloalkyl; optionally substituted means unsubstituted or one or more (for example, 2 or 3) substitutable sites of a substituted group independently substituted with R^{e3}.

In some embodiments, wherein R^{e} is independently selected from halogen, hydroxy, amino, cyano, or the following optionally substituted groups: methyl, ethyl, n-propyl, isopropyl, n-butyl, methoxy, ethoxy, cyclopropyl, cyclobutyl, cyclopentyl, -N(C₁₋₄ alkyl)₂, and -P(O)(C₁₋₄ alkyl)₂; optionally substituted means unsubstituted or one or more (for example, 2, 3, or 4) substitutable sites of a substituted group independently substituted with a substituent selected from halogen, hydroxy, amino, cyclopropyl, and methyl.

In some embodiments, wherein R^{e} is independently selected from halogen, methyl, cyclopropyl, -NH(CH₃), and -P(O)(C₂H₅)₂; or R^{e} is independently selected from fluoro, methyl, cyclopropyl, -NH(CH₃), and - P(O)(C₂H₅)₂.

In some embodiments, wherein R^{e} is fluoro, methyl, or cyclopropyl.

In some embodiments, wherein R^{e} is fluoro or cyclopropyl.

In some embodiments, wherein q is 2, and R^{e} is fluoro and cyclopropyl, respectively.

In some embodiments, wherein

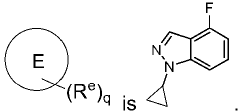

In some embodiments, wherein

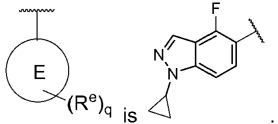

In some embodiments, wherein the proviso is that R^{e} is not methyl.

In some embodiments, wherein the proviso is that when Ring E is 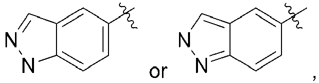 Ring A is 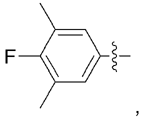 and q is 2, the two R^{e} are not methyl and fluoro.

In some embodiments, wherein two R^{e}, together with the atoms to which they are attached, form an optionally substituted 3-7 membered heterocyclyl or an optionally substituted 5-6 membered heteroaryl, and optionally substituted means unsubstituted or substituted with one or more (for example, 2, 3, 4, 5, 6, 7, or 8) groups selected from deuterium, halogen, hydroxy, amino, oxo, cyano, C₁₋₄ alkyl, and C₁₋₄ alkoxy; or two R^{e}, together with the atoms to which they are attached, form an optionally substituted 3-7 membered heterocyclyl or an optionally substituted 5-6 membered heteroaryl, and optionally substituted means unsubstituted or substituted with one or more (for example, 2, 3, or 4) groups selected from deuterium, fluoro, chloro, bromo, hydroxy, amino, oxo, cyano, methyl, ethyl, n-propyl, isopropyl, methoxy, and ethoxy; or two R^{e}, together with the atoms to which they are attached, form an optionally substituted 5-7 membered heterocyclyl or an optionally substituted 5-6 membered heteroaryl, and optionally substituted means unsubstituted or substituted with one or more (for example, 2, 3, or 4) groups selected from deuterium, fluoro, chloro, bromo, hydroxy, oxo, amino, cyano, methyl, ethyl, n-propyl, isopropyl, methoxy, and ethoxy; or two R^{e} (preferably two adjacent R^{e}; further preferably, two adjacent R^{e} located on different rings), together with the atoms to which they are attached, form an optionally substituted 6-7 membered heterocyclyl, and optionally substituted means unsubstituted or substituted with one or more (for example, 2, 3, or 4) groups selected from deuterium, fluoro, chloro, bromo, hydroxy, oxo, amino, cyano, methyl, ethyl, n-propyl, isopropyl, methoxy, and ethoxy;
preferably, the heterocyclyl or heteroaryl contains heteroatoms independently selected from N, O, or S, and the number of the heteroatoms is 1, 2, 3, or 4; further preferably, preferably the heterocyclyl or heteroaryl contains heteroatoms independently selected from N or O, and the number of the heteroatoms is 1 or 2;
preferably, the 6-7 membered heterocyclyl and Ring E form a fused tricyclic ring (i.e., the heterocyclyl and Ring E share 1 chemical bond (i.e., share 2 adjacent atoms), or 2 adjacent chemical bonds (i.e., share 3 adjacent atoms)); preferably, the 6-7 membered heterocyclyl is selected from 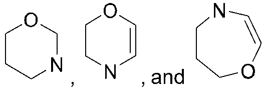 ; preferably, the 6-7 membered heterocyclyl is selected from 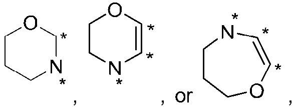 wherein * represents an atom shared with Ring E; preferably, the fused tricyclic ring formed by the 6-7 membered heterocyclyl and Ring E is selected from

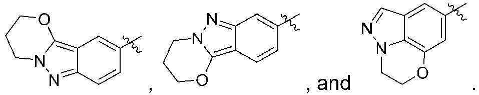

In some embodiments, wherein 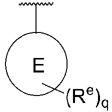 is

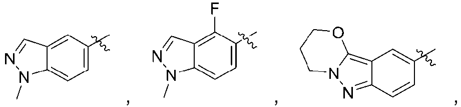

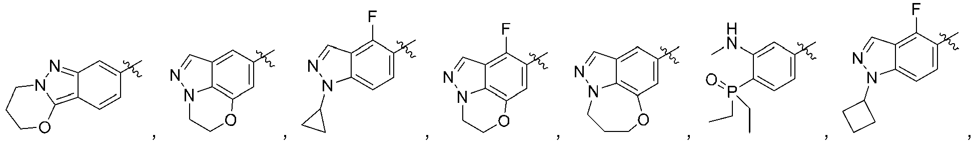

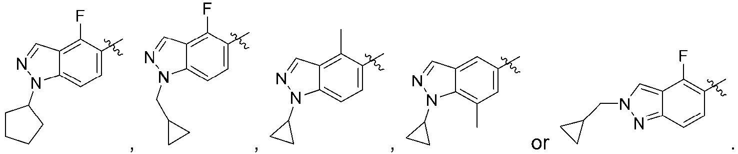

In some embodiments, wherein 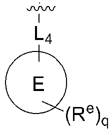 is selected from

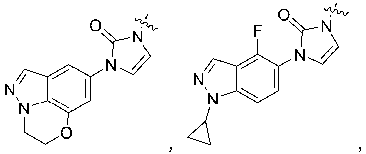

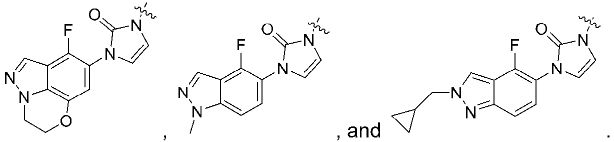

In some embodiments, wherein R^{e1} and R^{e2} are respectively independently selected from hydrogen, deuterium, and the following optionally substituted groups: C₁₋₆ alkyl, C₁₋₆ alkoxy, C₃₋₆ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from deuterium, halogen, hydroxy, amino, nitro, mercapto, cyano, oxo, C₁₋₆ alkyl, C₁₋₆ alkoxy, C₃₋₆ cycloalkyl, 3-6 membered heterocyclyl, phenyl, and 5-6 membered heteroaryl;
or R^{e1} and R^{e2} are respectively independently selected from hydrogen, or optionally substituted C₁₋₆ alkyl or C₃₋₆ cycloalkyl; optionally substituted means unsubstituted or substituted with one or more (for example, 2, 3, 4, 5, 6, 7, or 8) groups selected from halogen, hydroxy, amino, and cyano;
or R^{e1} and R^{e2} are respectively independently selected from hydrogen, or optionally substituted C₁₋₄ alkyl (for example, methyl, ethyl, n-propyl, isopropyl, or n-butyl), and C₃₋₄ cycloalkyl (for example, cyclopropyl or cyclobutyl); optionally substituted means unsubstituted or substituted with one or more (for example, 2, 3, 4, 5, 6, 7, or 8) groups selected from fluoro, chloro, and cyano.

In some embodiments, wherein R^{e1} and R^{e2} are respectively independently selected from hydrogen, hydroxy, amino, trifluoromethyl, difluoromethyl, monofluoromethyl, trichloromethyl, dichloromethyl, monochloromethyl, methyl, ethyl, n-propyl, isopropyl, n-butyl, methoxy, ethoxy, -CH₂CN, -CH₂CH₂CN, cyclopropyl, and cyclobutyl;
or R^{e1} and R^{e2} are respectively independently selected from hydrogen, methyl, and ethyl.

In some embodiments, wherein R^{e3} is independently selected from deuterium, halogen, hydroxy, amino, nitro, cyano, -R^{e4}, -OR^{e4}, -S(O)₂R^{e4}, -C(O)R^{e4}, -C(O)OR^{e4}, -OC(O)R^{e4}, -N(R^{e4})(R^{e5}), -C(O)N(R^{e4})(R^{e5}), - N(R^{e4})C(O)R^{e5}, -S(O)N(R^{e4})(R^{e5}), -SO₂N(R^{e4})(R^{e5}), -N(R^{e4})S(O)₂R^{e5}, and -P(O)(R^{e4})R^{e5}; R^{e4} and R^{e5} are respectively independently selected from hydrogen, or the following optionally substituted groups: C ₁₋₆ alkyl, C₁₋₆ alkoxy, C₂₋₄ alkenyl, C₂₋₄ alkynyl, C₃₋₆ cycloalkyl, for example, C₃₋₅ cycloalkyl or C₃₋₄ cycloalkyl, 3-6 membered heterocyclyl, phenyl, and 5-6 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₄ alkyl, C₁₋₄ alkoxy, C₂₋₄ alkenyl, C₂₋₄ alkynyl, C₃₋₆ cycloalkyl, 3-6 membered heterocyclyl, phenyl, and 5-6 membered heteroaryl;
or R^{e3} is independently selected from deuterium, halogen, hydroxy, amino, cyano, -R^{e4}, OR^{e4}, -SO₂(R^{e4}), - C(O)R^{e4}, -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or the following optionally substituted with one or more (for example, 2, 3, 4, or 5) groups selected from hydrogen, halogen, hydroxy, amino, and cyano: C₁₋₆ alkyl (preferably C₁₋₄ alkyl; preferably C₁₋₃ alkyl; for example, methyl, ethyl, propyl, or isopropyl), or C₃₋₆ cycloalkyl (for example, C₃₋₅ cycloalkyl or C₃₋₄ cycloalkyl).

In some embodiments, wherein q is independently selected from 0, 1, 2, or 3;
or q is independently selected from 1, 2, or 3;
or q is 2 or 3.

In some embodiments, wherein L₅ₓ and L_{5y} are respectively independently selected from a bond or optionally substituted C₁₋₆ alkylene; optionally substituted means unsubstituted or one or more (for example, 2, 3, 4, 5, 6, 7, or 8) substitutable sites of a substituted group independently substituted with deuterium, halogen, hydroxy, amino, cyano, oxo, C₁₋₆alkyl, C₁₋₆ alkoxy, C₃₋₆ cycloalkyl, and 3-6 membered heterocyclyl; or L₅ₓ and L_{5y} are respectively independently selected from a bond, hydrogen, optionally substituted methylene, or optionally substituted ethylene, and optionally substituted means unsubstituted or substituted with one or more (for example, 2, 3, 4, 5, 6, 7, or 8) substituents selected from fluoro, chloro, bromo, hydroxy, amino, cyano, and oxo;
or L₅ₓ and L_{5y} are respectively independently selected from a bond.

In some embodiments, wherein R¹ and R², together with the atoms to which they are attached, form C₃₋₁₀ carbocyclyl; the C₃₋₁₀ carbocyclyl is optionally substituted with one or more (for example, 2, 3, 4, 5, 6, 7, or 8) R³; R³ is independently selected from hydrogen, halogen, or the following optionally substituted groups: C₁₋₆ alkyl and C₁₋₆ alkoxy; optionally substituted means unsubstituted or one or more (for example, 2, 3, 4, 5, 6, 7, or 8) substitutable sites of a substituted group each independently substituted with deuterium, halogen, hydroxy, amino, cyano, oxo, C₃₋₆ cycloalkyl, and 3-6 membered heterocyclyl;
or R¹ and R², together with the atoms to which they are attached, form C₃₋₈ carbocyclyl (preferably C₃₋₆ carbocyclyl; further preferably C₃₋₅ carbocyclyl, for example, cyclopropyl, cyclobutyl, and cyclopentyl); the C₃₋₈ carbocyclyl is optionally substituted with one or more (for example, 2, 3, 4, 5, 6, 7, or 8) R³; R³ is independently selected from hydrogen, halogen, or the following optionally substituted groups: C₁₋₅ alkyl and C₁₋₅ alkoxy; optionally substituted means unsubstituted or substituted with one or more (for example, 2, 3, 4, or 5) groups selected from halogen, hydroxy, amino, and cyano;
or R¹ and R², together with the atoms to which they are attached, form the following groups optionally substituted with one or more (for example, 2, 3, 4, or 5) R³: 3-membered carbocyclyl, 4-membered carbocyclyl, 3-membered/3-membered spirocarbocyclyl, 3-membered/4-membered spirocarbocyclyl, 3-membered/5-membered spirocarbocyclyl, and 3-membered/6-membered spirocarbocyclyl; wherein R³ is independently selected from hydrogen, fluoro, chloro, bromo, methyl, ethyl, n-propyl, isopropyl, methoxy, trifluoromethyl, difluoromethyl, monofluoromethyl, trichloromethyl, dichloromethyl, and monochloromethyl; or a carbocycle formed by R¹ and R² together with atoms to which they are attached is independently selected from 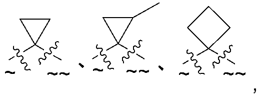 wherein a "~" end is an end connected to L₅ₓ, and a "~~" end is an end connected to L_{5y};
or a carbocycle formed by R¹ and R² together with atoms to which they are attached is independently selected from 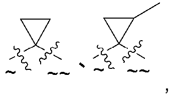 wherein a "~" end is an end connected to L₅ₓ, and a "~~" end is an end connected to L_{5y};
or a carbocycle formed by R¹ and R² together with atoms to which they are attached is 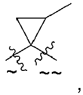 wherein a "~" end is an end connected to L₅ₓ, and a "~~" end is an end connected to L_{5y};

In some embodiments, wherein R^{f} is independently selected from -C(O)OR^{f1} and -C(O)N(R^{f1})(R^{f2}); or R^{f} is independently selected from -C(O)OR^{f1};
or R^{f} is independently selected from the following group: -COOH.

In some embodiments, wherein R^{f} is independently selected from 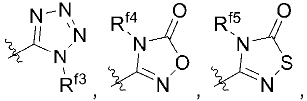 and 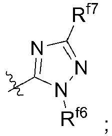 or R^{f} is independently selected from

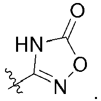

In some embodiments, wherein R^{f1}, R^{f2}, R^{f3}, R^{f4}, R^{f5}, R^{f6}, and R^{f7} are respectively independently selected from hydrogen, deuterium, or the following optionally substituted groups: C₁₋₆ alkyl, C₁₋₆ alkoxy, C₃₋₆ carbocyclyl, 3-6 membered heterocyclyl, C₆₋₈ aryl, and 5-6 membered heteroaryl; optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₂₋₄ alkenyl, C₂₋₄ alkynyl, C₃₋₆ cycloalkyl, 3-6 membered heterocyclyl, phenyl, and 5-6 membered heteroaryl;
or R^{f1}, R^{f2}, R^{f3}, R^{f4}, R^{f5}, R^{f6}, and R^{f7} are respectively independently selected from hydrogen, deuterium, or the following optionally substituted groups: C₁₋₆ alkyl, C₁₋₆ alkoxy, C₃₋₆ carbocyclyl, 3-6 membered heterocyclyl, phenyl, and 5-6 membered heteroaryl; optionally substituted means unsubstituted or substituted with one or more (for example, 2, 3, 4, 5, 6, 7, or 8) groups selected from halogen, hydroxy, amino, cyano, oxo, C₁₋₄ alkyl, and C₁₋₄ alkoxy;
or R^{f1}, R^{f2}, R^{f3}, R^{f4}, R^{f5}, R^{f6}, and R^{f7} are respectively independently selected from hydrogen, deuterium, or the following optionally substituted groups: C₁₋₄ alkyl, C₁₋₄ alkoxy, C₃₋₆ carbocyclyl, 3-6 membered heterocyclyl, phenyl, and 5-6 membered heteroaryl; optionally substituted means unsubstituted or substituted with one or more (for example, 2, 3, 4, 5, 6, 7, or 8) groups selected from halogen, hydroxy, amino, cyano, oxo, C₁₋₄ alkyl, and C₁₋₄ alkoxy;
or R^{f1}, R^{f2}, R^{f3}, R^{f4}, R^{f5}, R^{f6}, and R^{f7} are respectively independently selected from hydrogen, deuterium, or the following optionally substituted groups: C₁₋₃ alkyl, C₁₋₃ alkoxy, C₃₋₆ carbocyclyl, 3-6 membered heterocyclyl, phenyl, and 5-6 membered heteroaryl; optionally substituted means unsubstituted or substituted with one or more (for example, 2, 3, 4, 5, 6, 7, or 8) groups selected from halogen, hydroxy, amino, cyano, oxo, C₁₋₃ alkyl, and C₁₋₃ alkoxy.

In some embodiments, wherein R^{f1}, R^{f2}, R^{f3}, R^{f4}, R^{f5}, R^{f6}, and R^{f7} are respectively independently selected from hydrogen, deuterium, trifluoromethyl, difluoromethyl, monofluoromethyl, trichloromethyl, dichloromethyl, monochloromethyl, methyl, ethyl, n-propyl, isopropyl, methoxy, and ethoxy.

In some embodiments, wherein R^{f1}, R^{f2}, R^{f3}, R^{f4}, R^{f5}, R^{f6}, and R^{f7} are respectively independently selected from hydrogen.

In some embodiments, the compound must satisfy the following conditions:
a) Ring C is not 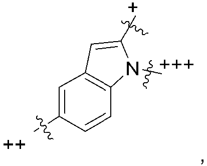 wherein a "+" end is an end connected to L₂, a "++" end is an end connected to L₃, and a "+++" end is an end connected to L₅ₓ; and/or
b) L₄ is not 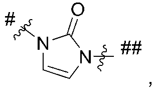 wherein a "#" end is the end connected to Ring B, and a "##" end is the end connected to Ring E; and/or
c) when the structure of the compound as shown in formula (I) is 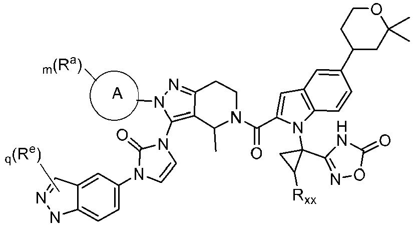 (wherein Rₓₓ is H or CH₃, and R^{e} may be substituted on any ring of the bicyclic system), and when 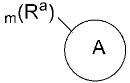 is 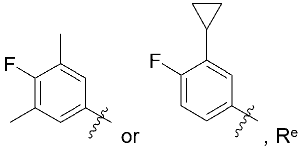 is selected from halogen and optionally substituted C₃₋₆ cycloalkyl; q is 1 or 2; optionally substituted means unsubstituted or one or more (for example, 2, 3, 4, or 5) substitutable sites of a substituted group independently substituted with R^{e3}; R^{e3} is independently selected from deuterium, halogen, hydroxy, amino, cyano, -OR^{e4}, -SO₂(R^{e4}), -C(O)R^{e4}, -N(R^{e3})(R^{e4}), -SO₂N(R^{e4})(R^{e5}), -N(R^{e4})S(O)₂R^{e5}, and -P(O)(R^{e4})(R^{e5}); R^{e4} and R^{e5} are respectively independently selected from hydrogen or optionally substituted C₁₋₆ alkyl, and optionally substituted means unsubstituted or substituted with one or more substituents selected from hydrogen, halogen, hydroxy, amino, and cyano; or two R^{e}, together with the atoms to which they are attached, form an optionally substituted 3-10 membered heterocyclyl (preferably 3-10 membered heterocycloalkyl, further preferably 5-7 membered heterocycloalkyl), and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl; and/or
d) when the structure of the compound as shown in formula (I) is 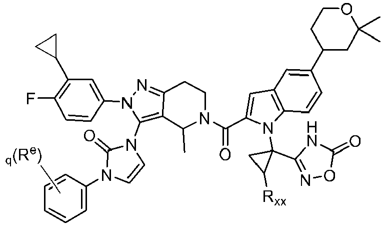 (wherein Rₓₓ is H or CH₃), 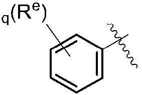 is

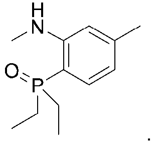

In some embodiments, wherein Ring A is selected from phenyl and 3-6 membered heterocyclyl;
Ring B is selected from 8-9 membered bicyclic heterocyclyl and 8-9 membered bicyclic heteroaryl;
Ring C is selected from 9-membered bicyclic heteroaryl;
Ring D is selected from 6-membered heterocyclyl and 6-membered heteroaryl;
Ring E is selected from C₆₋₈ aryl and 5-10 membered heteroaryl;
R^{a}, at each occurrence, is independently selected from halogen, hydroxy, amino, nitro, mercapto, cyano, oxo, -C(O)N(R^{a1})(R^{a2}), -N(R^{a1})C(O)(R^{a2}), and the following optionally substituted groups: C₁₋₆ alkyl, C₁₋₆ alkoxy, C₃₋₆ cycloalkyl, and 3-6 membered heterocyclyl; optionally substituted means unsubstituted or substituted with one or more (for example, 2, 3, 4, 5, 6, 7, or 8) substituents selected from hydrogen, halogen, hydroxy, and amino;
R^{b}, at each occurrence, is independently selected from halogen, hydroxy, amino, cyano, oxo, and C₁₋₆ alkyl; R^{c}, at each occurrence, is independently selected from halogen, hydroxy, amino, cyano, oxo, and C₁₋₆ alkyl; R^{d}, at each occurrence, is independently selected from halogen, hydroxy, amino, cyano, oxo, or the following optionally substituted groups: C₁₋₄ alkyl and C₁₋₄ alkoxy; optionally substituted means unsubstituted or substituted with one or more (for example, 2, 3, 4, or 5) substituents selected from hydrogen, halogen, hydroxy, and amino;
R^{e}, at each occurrence, is independently selected from deuterium, halogen, hydroxy, amino, cyano, -OR^{e1}, -SO₂(R^{e1}), -C(O)R^{e1}, -N(R^{e1})(R^{e2}), -SO₂N(R^{e1})(R^{e2}), -N(R^{e1})SO₂(R^{e2}), -P(O)(R^{e1})(R^{e2}), optionally substituted C₁₋₆ alkyl, or optionally substituted C₃₋₆ cycloalkyl; R^{e1} and R^{e2} are respectively independently selected from hydrogen, methyl, and ethyl; optionally substituted means unsubstituted or one or more (for example, 2, 3, 4, or 5) substitutable sites of a substituted group independently substituted with R^{e3}; R^{e3} is independently selected from deuterium, halogen, hydroxy, amino, cyano, -R^{e4}, OR^{e4}, -SO₂(R^{e4}), -C(O)R^{e4}, -N(R^{e4})(R^{e5}), - SO₂N(R^{e4})(R^{e5}), -N(R^{e4})SO₂(R^{e5}), and -P(O)(R^{e4})(R^{e5}); R^{e4} and R^{e5} are respectively independently selected from hydrogen or the following optionally substituted with one or more (for example, 2, 3, 4, or 5) selected from hydrogen, halogen, hydroxy, amino, and cyano: C₁₋₆ alkyl or C₃₋₆ cycloalkyl (preferably C₃₋₅ cycloalkyl, preferably C₃₋₄ cycloalkyl, for example cyclopropyl or cyclobutyl);
R¹ and R², together with atoms to which they are attached, form C₃₋₈ carbocyclyl; the C₃₋₈ carbocyclyl is optionally substituted with one or more R³; R³ is independently selected from hydrogen, halogen, or the following optionally substituted groups: C₁₋₅ alkyl and C₁₋₅ alkoxy; optionally substituted means unsubstituted or substituted with one or more (for example, 2, 3, 4, or 5) groups selected from hydrogen, halogen, hydroxy, amino, and cyano;
R^{f} is selected from -COOH and 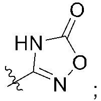
L₁ is selected from a bond and -CH₂-;
L₂ is selected from a bond, -CH₂-, and -C(O)-;
L₃ is selected from a bond, -CH₂-, and -C(O)-;
L₅ₓ and L_{5y} are respectively independently selected from a bond;
m, n, o, p, q, and r are each independently 0, 1, 2, or 3;
L₄ is selected from -NHC(O)NH-, -NHC(S)NH-, -NH-, -N(cyclopropyl)C(O)NH-, - N(cyclopropyl)C(O)N(cyclopropyl)-, -N(methyl)C(O)NH-, -N(methyl)C(O)N(methyl)-, -NHC(S)NH-, -NH- 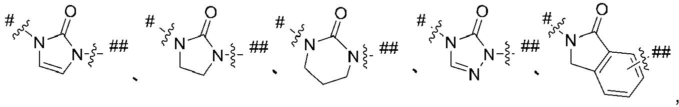 wherein an "#" end is the end connected to Ring B, and an "##" end is the end connected to Ring E.

In some embodiments, wherein Ring A is selected from phenyl and 3-6 membered heterocyclyl;
Ring B is selected from 9-membered fused heterocyclyl and 9-membered bicyclic heteroaryl;
Ring C is selected from 9-membered bicyclic heteroaryl;
Ring D is selected from 6-membered heterocyclyl and 6-membered heteroaryl; the heteroatoms in the 6-membered heterocyclyl are O, and the number of the heteroatoms is 1; the heteroatoms in the 6-membered heteroaryl are N, and the number of the heteroatoms is 1 or 2;
Ring E is selected from phenyl, 5-membered/6-membered fused heteroaryl, and 6-membered/5-membered fused heteroaryl; the heteroatoms contained in the 5-membered/6-membered fused heteroaryl or 6-membered/5-membered fused heteroaryl are N, and the number of the heteroatoms is 1, 2, or 3;
R^{a}, at each occurrence, is independently selected from halogen, hydroxy, amino, cyano, and the following optionally substituted groups: C₁₋₄ alkyl, C₁₋₄ alkoxy, C₃₋₄ cycloalkyl, and 4-5 membered heterocyclyl; optionally substituted means unsubstituted or substituted with one or more (for example, 2, 3, 4, 5, 6, 7, or 8) substituents selected from hydrogen, halogen, hydroxy, and amino;
R^{b}, at each occurrence, is independently selected from halogen, hydroxy, amino, cyano, oxo, and C₁₋₄ alkyl; R^{c}, at each occurrence, is independently selected from halogen, hydroxy, amino, cyano, oxo, and C₁₋₄ alkyl; R^{d}, at each occurrence, is independently selected from halogen, hydroxy, amino, cyano, or the following optionally substituted groups: C₁₋₄ alkyl and C₁₋₄ alkoxy; optionally substituted means unsubstituted or substituted with one or more (for example, 2, 3, 4, or 5) substituents selected from hydrogen, halogen, hydroxy, and amino;
R^{d}, at each occurrence, is independently selected from halogen, -N(R^{e1})(R^{e2}), -P(O)(R^{e1})(R^{e2}), optionally substituted C₁₋₆ alkyl, or optionally substituted C₃₋₆ cycloalkyl; R^{e1} and R^{e2} are respectively independently selected from hydrogen, methyl, and ethyl; optionally substituted means unsubstituted or one or more (for example, 2, 3, 4, or 5) substitutable sites of a substituted group independently substituted with R^{e3}; R^{e3} is independently selected from deuterium, halogen, hydroxy, amino, cyano, -R^{e4}, - OR^{e4}, -SO₂(R^{e4}), -C(O)R^{e4}, -N(R^{e4})(R^{e5}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or optionally substituted C₁₋₆ alkyl or C₃₋₆ cycloalkyl (preferably C₃₋₅ cycloalkyl, preferably C₃₋₄ cycloalkyl, for example, cyclopropyl or cyclobutyl); optionally substituted means unsubstituted or substituted with one or more (for example, 2, 3, 4, or 5) substituents selected from hydrogen, halogen, hydroxy, amino, and cyano;
a carbocycle formed by R¹ and R² together with atoms to which they are attached is independently selected from 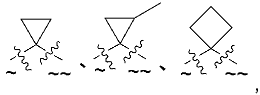 wherein a "~" end is an end connected to L₅ₓ, and a "~~" end is an end connected to L_{5y};
R^{f} is selected from -COOH and 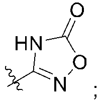
L₁ is selected from a bond and -CH₂-;
L₂ is selected from a bond, -CH₂-, and -C(O)-;
L₃ is selected from a bond, -CH₂-, and -C(O)-;
L₄ is selected from -NHC(O)NH-, -N(cyclopropyl)C(O)NH-, -N(cyclopropyl)C(O)N(cyclopropyl)-, - N(methyl)C(O)NH-, -N(methyl)C(O)N(methyl)-, -NHC(S)NH-, and -NH- 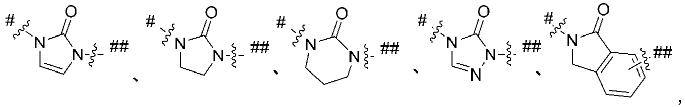 wherein a "#" end is the end connected to Ring B, and a "##" end is the end connected to Ring E;
L₅ₓ and L_{5y} are respectively independently selected from a bond;
m, n, o, p, q, and r are each independently 0, 1, 2, or 3.

In some embodiments, wherein Ring A is selected from phenyl and 3-6 membered heterocyclyl;
Ring B is selected from 9-membered fused heterocyclyl and 9-membered bicyclic heteroaryl; the heteroatoms in the 9-membered fused heterocyclyl and 9-membered bicyclic heteroaryl are selected from N, and the number of the heteroatoms is 1, 2, or 3;
Ring C is selected from 9-membered bicyclic heteroaryl; the heteroatoms in the 9-membered bicyclic heteroaryl are selected from N, and the number of the heteroatoms is 1 or 2;
Ring D is selected from tetrahydropyranyl and pyridyl;
Ring E is selected from phenyl and indazolyl;
R^{a}, at each occurrence, is independently selected from halogen, methyl, and cyclopropyl;
R^{b}, at each occurrence, is independently selected from fluoro, chloro, oxo, and methyl;
R^{c}, at each occurrence, is independently selected from fluoro, chloro, oxo, methyl, ethyl, n-propyl, isopropyl, and n-butyl;
R^{d}, at each occurrence, is independently selected from optionally substituted C₁₋₄ alkyl and optionally substituted C₁₋₄ alkoxy; the C₁₋₄ alkyl or C₁₋₄ alkoxy is optionally substituted with one or more (for example, 2, 3, 4, or 5) substituents selected from hydrogen, halogen, hydroxy, and amino;
R^{e} is independently selected from halogen, -N(R^{e1})(R^{e2}), -P(O)(R^{e1})(R^{e2}), optionally substituted C₁₋₆ alkyl, and optionally substituted C₃₋₆ cycloalkyl; R^{e1} and R^{e2} are respectively independently selected from hydrogen, methyl, and ethyl; optionally substituted means unsubstituted or one or more (for example, 2, 3, 4, or 5) substitutable sites of a substituted group independently substituted with R^{e3}; R^{e3} is independently selected from deuterium, halogen, hydroxy, amino, cyano, -R^{e4}, - OR^{e4}, -SO₂(R^{e4}), -C(O)R^{e4}, -N(R^{e3})(R^{e4}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or optionally substituted C₁₋₆ alkyl (preferably C₁₋₃ alkyl, for example, methyl or ethyl), or C₃₋₆ cycloalkyl (preferably C₃₋₅ cycloalkyl, preferably C₃₋₄ cycloalkyl, such as cyclopropyl or cyclobutyl), and optionally substituted means unsubstituted or substituted with one or more substituents selected from hydrogen, halogen, hydroxy, amino, and cyano;
a carbocycle formed by R¹ and R² together with atoms to which they are attached is independently selected from 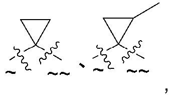 wherein a "~" end is an end connected to L₅ₓ, and a "~~" end is an end connected to L_{5y};
R^{f} is selected from -COOH and 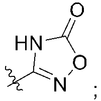
L₁ is selected from a bond and -CH₂-;
L₂ is selected from -C(O)-;
L₃ is selected from a bond;
L₄ is selected from -NHC(O)NH- and 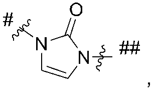 wherein a "#" end is the end connected to Ring B, and a "##" end is the end connected to Ring E;
L₅ₓ and L_{5y} are respectively independently selected from a bond;
m, n, o, p, q, and r are each independently 0, 1, or 2.

In some embodiments, wherein Ring A is selected from phenyl;
Ring B is selected from 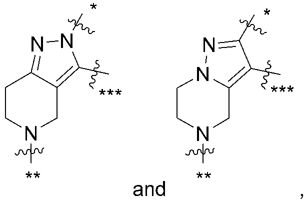 wherein a "*" end is an end connected to L₁, a "**" end is an end connected to L₂, and a "***" end is an end connected to L₄;
Ring C is selected from the following group: 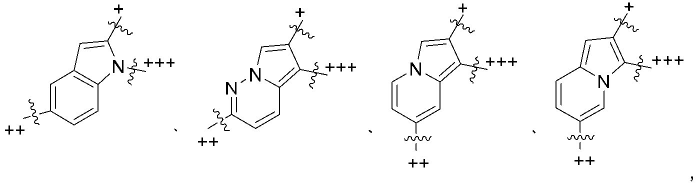 wherein a "+" end is an end connected to L₂, a "++" end is an end connected to L₃, and a "+++" end is an end connected to L₅ₓ;
Ring D is 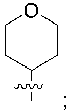
Ring E is 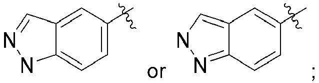
L₁ is selected from a bond;
L₂ is selected from -C(O)-;
L₃ is selected from a bond;
L₄ is selected from -NHC(O)NH-, -N(methyl)C(O)NH-, -N(cyclopropyl)C(O)NH-, - N(cyclopropyl)C(O)N(cyclopropyl)-, and 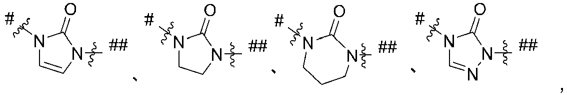 wherein a "#" end is an end connected to the Ring B, and a "##" end is an end connected to the Ring E; L₅ₓ and L_{5y} are respectively independently selected from a bond;
R^{a}, at each occurrence, is independently selected from halogen, methyl, and C₃₋₅ cycloalkyl-(CH₂)_{w}-;
R^{b}, at each occurrence, is independently selected from fluoro, chloro, oxo, and methyl;
R^{c}, at each occurrence, is independently selected from hydrogen, fluoro, chloro, oxo, methyl, ethyl, n-propyl, isopropyl, and n-butyl;
R^{d}, at each occurrence, is independently selected from optionally substituted C ₁₋₃ alkyl and optionally substituted C₁₋₃ alkoxy; optionally substituted means unsubstituted or substituted with one or more (for example, 2, 3, 4, or 5) substituents selected from hydrogen, deuterium, halogen, hydroxy, and amino;
R^{e}, at each occurrence, is each independently selected from fluoro or the following optionally substituted groups: C₁₋₃ alkyl (for example, methyl or ethyl), C₃₋₅ cycloalkyl (for example, cyclopropyl, cyclobutyl, and cyclopentyl), and C₃₋₅ cycloalkyl-(CH₂)_{w}-; provided that, when L₄ is 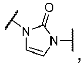 at least 1 (for example, 1 or 2) of R^{a} or substituents on the Ring E (or R^{e}) is C₃₋₅ cycloalkyl-(CH₂)_{w}-;
or two R^{e} (preferably two adjacent R^{e}; further preferably, two adjacent R^{e} located on different rings), together with atoms to which they are each attached, form optionally substituted 5-7 membered heterocyclyl (for example, 5-membered, 6-membered, or 7-membered heterocyclyl), and the heterocyclyl contains 1-3 (for example, 1, 2, or 3) heteroatoms each independently selected from N, O, or S; optionally substituted means that hydrogen on a substituted group is unsubstituted or one or more substitutable sites of the substituted group are independently substituted with R^{e3};
a carbocycle formed by R¹ and R² together with atoms to which they are attached is independently selected from 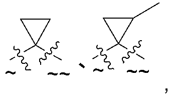 wherein a "~" end is an end connected to L₅ₓ, and a "~~" end is an end connected to L_{5y};
R^{f} is selected from 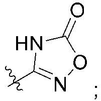
m is 2 or 3; q is 1, 2, or 3;
n, o, and p are each independently 0, 1, or 2;
w is 0, 1, or 2;
the R^{e3} is as defined for the compound of formula (I);
provided that the following compounds are not included:

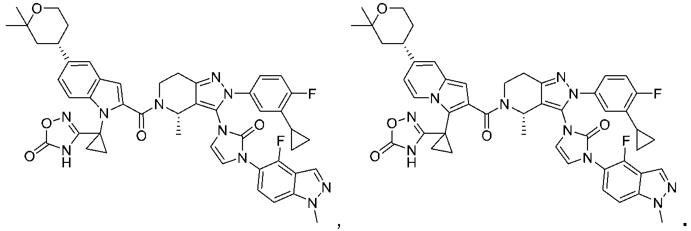

In some embodiments, two R^{e} (preferably two adjacent R^{e}; further preferably, two adjacent R^{e} located on different rings), together with the atoms to which they are respectively attached, form an optionally substituted 6-7 membered heterocyclyl, and the heterocyclyl (preferably heterocycloalkyl) contains 1-3 (for example, 1, 2, or 3) heteroatoms each independently selected from N, O, or S; preferably, the 6-7 membered heterocyclyl and Ring E form a fused tricyclic ring (i.e., the heterocyclyl and Ring E share 1 chemical bond (i.e., share 2 adjacent atoms), or 2 adjacent chemical bonds (i.e., share 3 adjacent atoms)); preferably, the 6-7 membered heterocyclyl is selected from 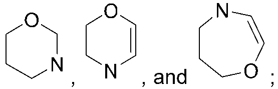 preferably, the 6-7 membered heterocyclyl is selected from 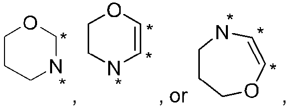 wherein * represents an atom shared with Ring E; preferably, the fused tricyclic ring formed by the 6-7 membered heterocyclyl and Ring E is selected from

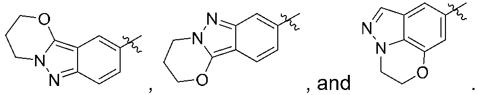

In some embodiments, wherein the compound as shown in formula (I) is a compound as shown in the following formula:

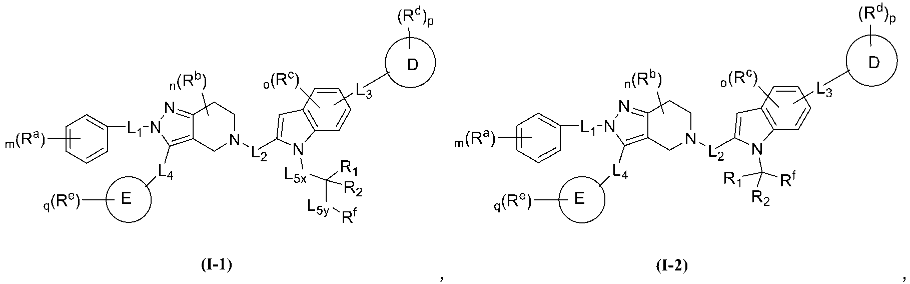

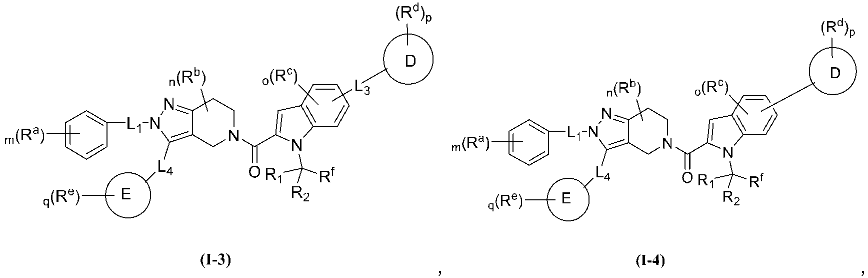

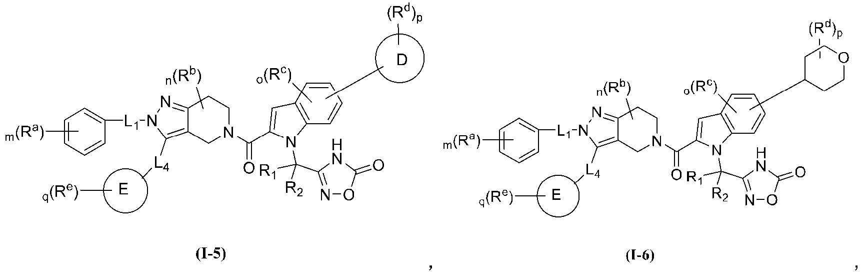

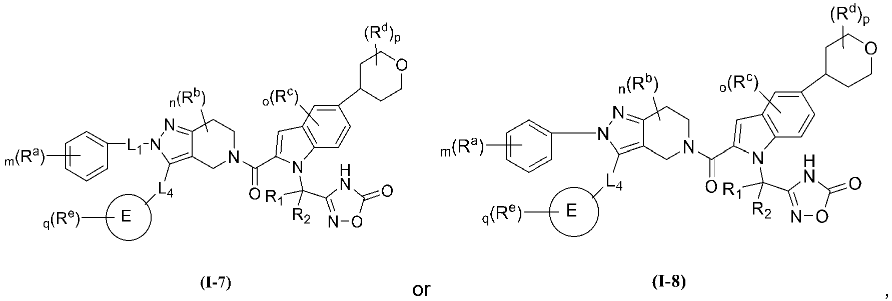

Ring D, Ring E, R^{a}, R^{b}, R^{c}, R^{d}, R^{e}, R¹, R², L¹, L⁴, m, n, o, p, and q are as defined for the compound of formula (I).

In some embodiments, L₄ is -NHC(O)NH-, -N(cyclopropyl)C(O)NH-, -N(cyclopropyl)C(O)N(cyclopropyl)-, - N(methyl)C(O)NH-, -N(methyl)C(O)N(methyl)-, -NHC(S)NH-, -NH-, or 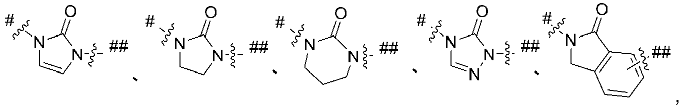 wherein the "#" end is the end connected to Ring B, and the "##" end is the end connected to Ring E; when L₄ is 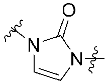 and the substitution position of R^{a} on the benzene ring is 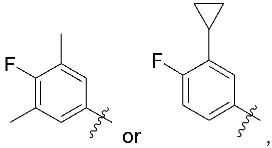 R^{e} is selected from halogen and optionally substituted C₃₋₆ cycloalkyl; q is 1 or 2; optionally substituted means unsubstituted or one or more (for example, 2, 3, 4, or 5) substitutable sites of a substituted group independently substituted with R^{e3};
R^{e3} is independently selected from deuterium, halogen, hydroxy, amino, cyano, OR^{e4}, -SO₂(R^{e4}), -C(O)R^{e4}, -N(R^{e3})(R^{e4}), -SO₂N(R^{e4})(R^{e5}), -N(R^{e4})SO₂(R^{e5}), and -P(O)(R^{e4})(R^{e5}); R^{e4} and R^{e5} are respectively independently selected from hydrogen or optionally substituted C₁₋₆ alkyl, and optionally substituted means unsubstituted or substituted with one or more substituents selected from hydrogen, halogen, hydroxy, amino, and cyano; or two R^{e}, together with the atoms to which they are attached, form an optionally substituted 3-10 membered heterocyclyl (preferably 3-10 membered heterocycloalkyl, further preferably 5-7 membered heterocycloalkyl), and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl.

In some embodiments, wherein the compound as shown in formula (I) is a compound as shown in the following formula: 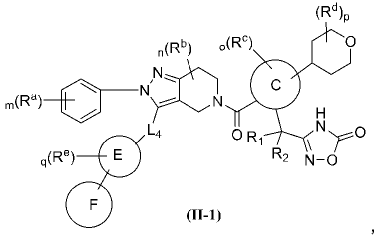
Ring C, Ring E, R^{a}, R^{b}, R^{c}, R^{d}, R^{e}, R¹, R², L¹, L⁴, m, n, o, p, and q are as defined for the compounds of formula (I), formula (II), or formulae (I-1) to (I-8);
wherein Ring F is C₃₋₆ cycloalkyl.

In some embodiments, Ring F is cyclopropyl, cyclobutyl, cyclopentyl, or cyclohexyl; preferably, Ring F is cyclopropyl.

In some embodiments, wherein the compound as shown in formula (I) is a compound as shown in the following formula:

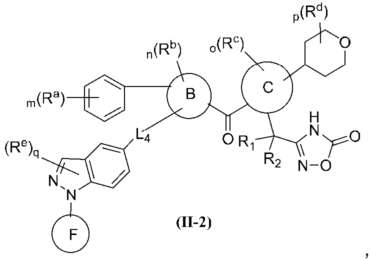

Ring C, Ring B, R^{a}, R^{b}, R^{c}, R^{d}, R^{e}, R¹, R², L¹, L⁴, m, n, o, p, and q are as defined for the compounds of formula (I), formula (II), or formulae (I-1) to (I-8); wherein Ring F is C₃₋₆ cycloalkyl.

In some embodiments, Ring F is cyclopropyl, cyclobutyl, cyclopentyl, or cyclohexyl; preferably, Ring F is cyclopropyl.

In some embodiments, wherein the compound as shown in formula (I) is a compound as shown in the following formula:

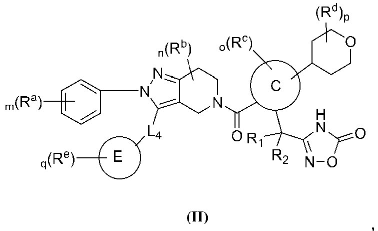

Ring C, Ring E, R^{a}, R^{b}, R^{c}, R^{d}, R^{e}, R¹, R², L¹, L⁴, m, n, o, p, and q are as defined for the compounds of formula (I) and formulae (I-1) to (I-8).

In some embodiments, in the compound as shown in formula (II), wherein Ring C is selected from the following group: 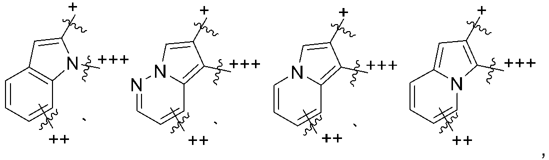 wherein the "+" end is the end connected to -C(=O)-, the "++" end is the end connected to tetrahydropyran, and the "+++" end is the end connected to 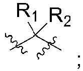 provided that, when Ring C is 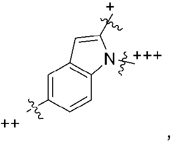 L₄ is not 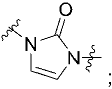 or, when Ring C is 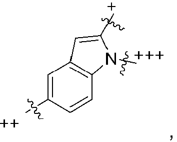 L₄ is 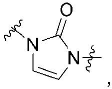 and the substitution position of R^{a} on the benzene ring is 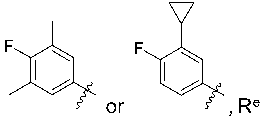 is selected from halogen, optionally substituted C₃₋₆ cycloalkyl, and substituted C₁₋₃ alkyl, and q is 1 or 2; optionally substituted means unsubstituted or one or more (for example, 2, 3, 4, or 5) substitutable sites of the substituted group being independently substituted with R^{e3}, and substituted means unsubstituted or one or more (for example, 2, 3, 4, or 5) substitutable sites of the substituted group being independently substituted with R^{e3};
R^{e3} is independently selected from deuterium, halogen, hydroxy, amino, cyano, -R^{e4}, - OR^{e4}, -SO₂(R^{e4}), - C(O)R^{e4}, -N(R^{e3})(R^{e4}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or optionally substituted C₁₋₆ alkyl (preferably C₁₋₃ alkyl, for example, methyl or ethyl), or C₃₋₆ cycloalkyl (preferably C₃₋₅ cycloalkyl, for example, cyclopropyl, cyclobutyl, and cyclopentyl), and optionally substituted means unsubstituted or substituted with one or more substituents selected from hydrogen, halogen, hydroxy, amino, and cyano;
or two R^{e}, together with the atoms to which they are attached, form an optionally substituted 3-10 membered heterocyclyl (preferably a 5-7 membered heterocyclyl, such as a 5-membered heterocyclyl, a 6-membered heterocyclyl, or a 7-membered heterocyclyl; further preferably, a 5-7 membered heterocycloalkyl, for example, a 5-membered heterocycloalkyl, a 6-membered heterocycloalkyl, or a 7-membered heterocycloalkyl), and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl (preferably C₁₋₃ alkyl, for example, methyl or ethyl), C₁₋₆ alkoxy (preferably C₁₋₃ alkoxy, for example, methoxy or ethoxy), C₃₋₁₀ cycloalkyl, 3-10 membered heterocyclyl, C₆₋₁₄ aryl, and 5-12 membered heteroaryl; preferably, the heteroatoms in the heterocyclyl or heterocycloalkyl are independently selected from N, O, or S, and the number of the heteroatoms is 1, 2, 3, or 4.

In some embodiments, Ring C is selected from the following groups: 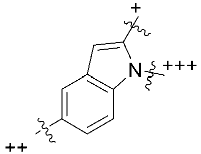 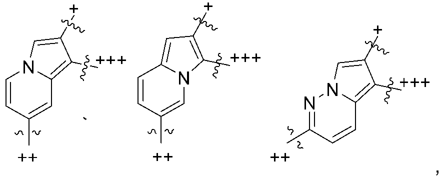 wherein a "+" end is an end connected to L₂, a "++" end is an end connected to L₃, and a "+++" end is an end connected to L₅ₓ. or
Ring C is selected from the following group: 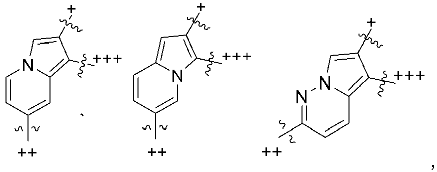 wherein a "+" end is an end connected to L₂, a "++" end is an end connected to L₃, and a "+++" end is an end connected to L₅ₓ.

In some embodiments, in the compound as shown in formula (I), formulae (I-1) to (I-8), formula (II), formula (11-1), or formula (II-2) as described above, wherein,
Ring E is selected from phenyl and indazolyl;
R^{a}, at each occurrence, is independently selected from halogen, methyl, and cyclopropyl;
R^{b}, at each occurrence, is independently selected from fluoro, chloro, oxo, and methyl;
R^{c}, at each occurrence, is independently selected from fluoro, chloro, oxo, methyl, ethyl, n-propyl, isopropyl, and n-butyl;
R^{d}, at each occurrence, is independently selected from C₁₋₄ alkyl and C₁₋₄ alkoxy; the C₁₋₄ alkyl or C₁₋₄ alkoxy is optionally substituted with one or more (for example, 2, 3, 4, or 5) substituents selected from hydrogen, halogen, hydroxy, and amino;
a carbocycle formed by R¹ and R² together with atoms to which they are attached is independently selected from 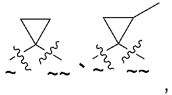 wherein a "~" end is an end connected to L₅ₓ, and a "~~" end is an end connected to L_{5y};
L₄ is selected from -NHC(O)NH-, -N(cyclopropyl)C(O)NH-, -N(cyclopropyl)C(O)N(cyclopropyl)-, - N(methyl)C(O)NH-, -N(methyl)C(O)N(methyl)-, -NHC(S)NH-, -NH-, and 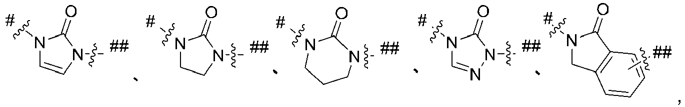 wherein a "#" end is the end connected to Ring B, and a "##" end is the end connected to Ring E;
m, n, o, p, q, and r are each independently 0, 1, or 2.

In some embodiments, in the compound as shown in formula (I), formulae (1-1) to (I-8), or formula (II) as described above, wherein L₄ is selected from -NHC(O)NH-, -N(cyclopropyl)C(O)NH-, - N(cyclopropyl)C(O)N(cyclopropyl)-, -N(methyl)C(O)NH-, -N(methyl)C(O)N(methyl)-, -NHC(S)NH-, -NH-, and 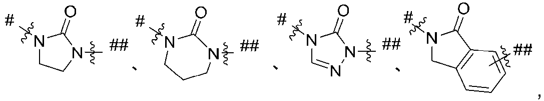 wherein the "#" end is the end connected to Ring B, and the "##" end is the end connected to Ring E.

In some embodiments, wherein the compound as shown in formula (I), formulae (1-1) to (I-8), or formula (II) as described above is a compound as shown in the following formula: 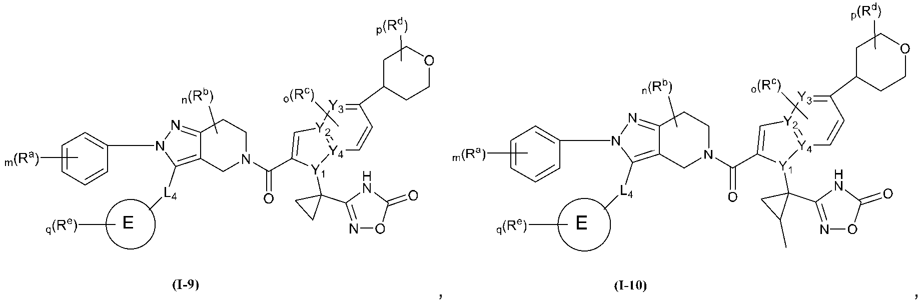 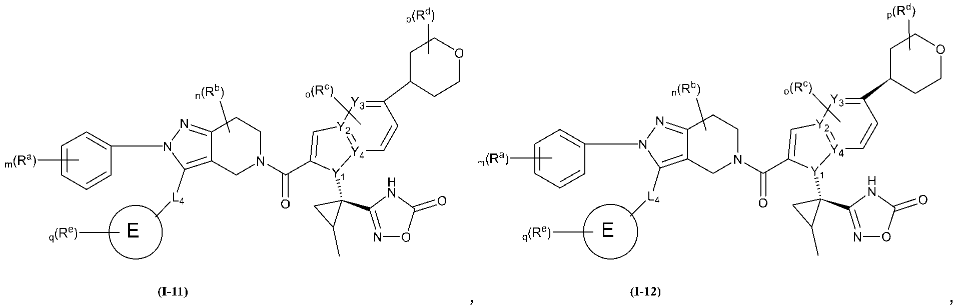 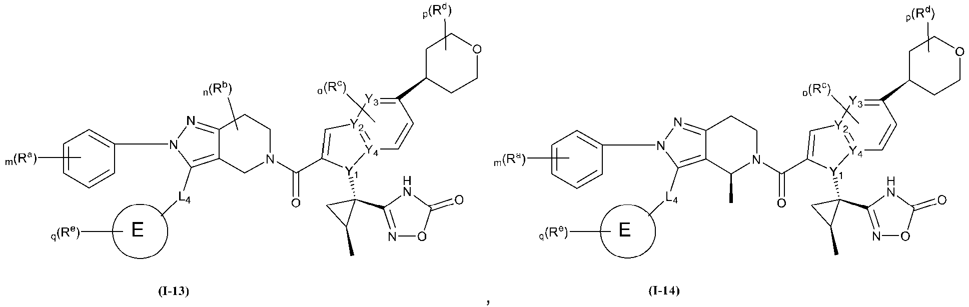 or,
wherein Y₁ is N, Y₂ and Y₃ are both C, and Y₄ is C or CH; or Y₁ is C, Y₂is N, Y₃is C or CH, Y₄is C; or Y₁ is C, Y₂is C, Y₃is C or CH, Y₄is N; or Y₁ is C, Y₂is N, Y₃is N, Y₄is C;
Ring E, R^{a}, R^{b}, R^{c}, R^{d}, R^{e}, R¹, R², L¹, L⁴, m, n, o, p, and q are as defined for the compounds of formula (I), formulae (1-1) to (I-8), or formula (II).

In some embodiments, wherein the compound as shown in formula (I), formulae (1-1) to (I-8), or formula (II) as described above is a compound as shown in the following formula: 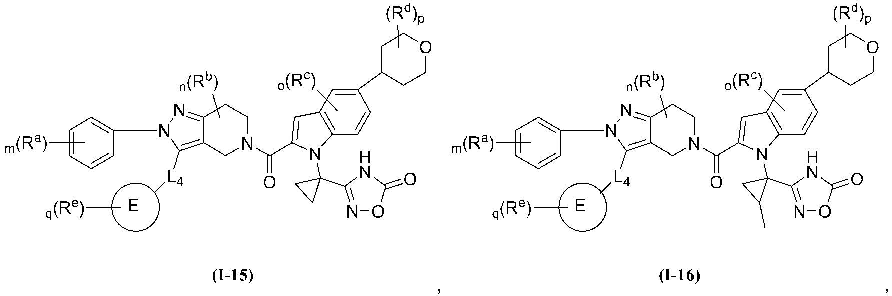 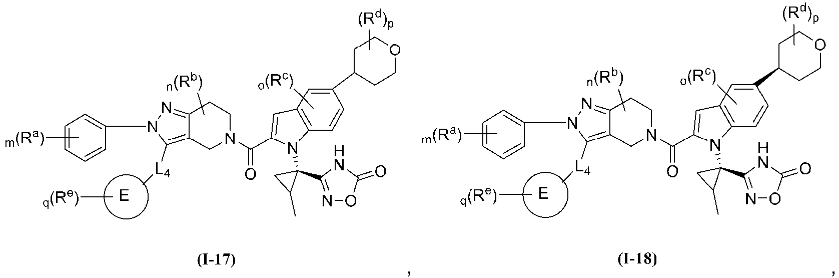 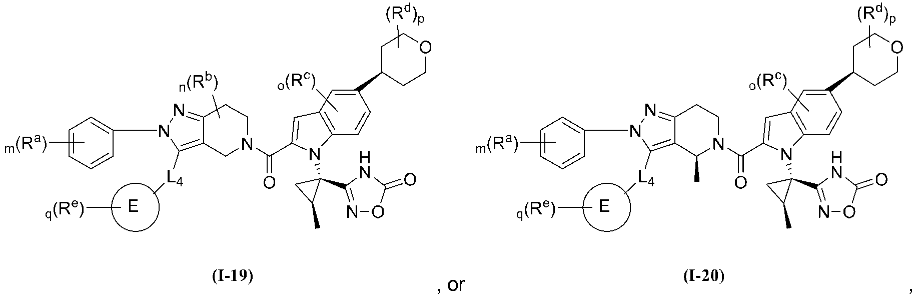 wherein Ring E, R^{a}, R^{b}, R^{c}, R^{d}, R^{e}, R¹, R², L¹, L⁴, m, n, o, p, and q are as defined for the compounds of formula (I), formulae (1-1) to (I-8), or formula (II).

In some embodiments, the proviso is that the following compounds are not included:

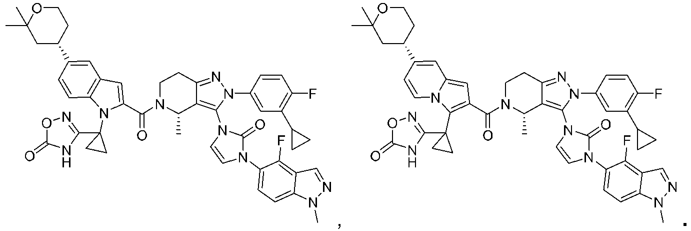

In some embodiments, wherein the compound as shown in formula (I), formula (II), or formulae (1-1) to (I-8) is a compound as shown in formula (III): 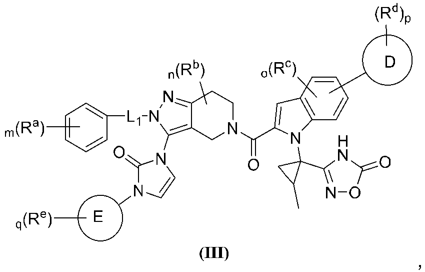
Ring D is independently selected from 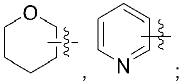
Ring E is selected from 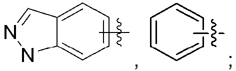
R^{a} is selected from fluoro, methyl, and cyclopropyl;
R^{b} is selected from methyl;
R^{c} is selected from fluoro and chloro;
R^{d} is selected from methyl, methoxy, and ethoxy;
R^{e} is selected from halogen, -N(R^{e1})(R^{e2}), -P(O)(R^{e1})(R^{e2}), or the following optionally substituted groups: C₁₋₆ alkyl (preferably C₁₋₃ alkyl, for example, methyl or ethyl), and C₃₋₆ cycloalkyl (preferably C₃₋₅ cycloalkyl, for example, cyclopropyl or cyclobutyl); R^{e1} and R^{e2} are respectively independently selected from hydrogen, methyl, and ethyl; optionally substituted means that hydrogen on a substituted group is unsubstituted or one or more substitutable sites of the substituted group are independently substituted with R^{e3}; R^{e3} is independently selected from deuterium, halogen, hydroxy, amino, cyano, -R^{e4}, - OR^{e4}, -SO₂(R^{e4}), -C(O)R^{e4}, -N(R^{e4})(R^{e5}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen, or C₁₋₆ alkyl or C₃₋₆ membered cycloalkyl optionally substituted with one or more groups selected from hydrogen, halogen, hydroxy, amino, and cyano;
or two R^{e}, together with the atoms to which they are attached, form an optionally substituted 5-7 membered heterocyclyl, and optionally substituted means unsubstituted or substituted with one or more (for example, 2, 3, or 4) groups selected from deuterium, halogen, hydroxy, amino, nitro, mercapto, cyano, oxo, methyl, ethyl, methoxy, and cyclopropyl;
L₁ is independently selected from a bond;
m, n, o, p, q, and r are each independently 0, 1, 2, or 3.

In some embodiments, R^{e} is cyclopropyl, cyclobutyl, cyclopentyl, or cyclohexyl; preferably, R^{e} is cyclopropyl.

In some embodiments, wherein Ring D is independently selected from 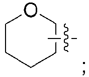
Ring E is selected from 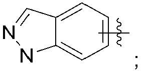
R^{a} is selected from fluoro, methyl, and cyclopropyl;
Rb is selected from methyl;
R^{c} is selected from H;
R^{d} is selected from methyl;
R^{e} is selected from halogen or the following optionally substituted groups: C₁₋₆ alkyl (preferably C₁₋₃ alkyl, for example, methyl or ethyl), and C₃₋₆ cycloalkyl (preferably C₃₋₅ cycloalkyl, for example, cyclopropyl or cyclobutyl); optionally substituted means that hydrogen on a substituted group is unsubstituted or one or more substitutable sites of the substituted group are independently substituted with R^{e3}. R^{e3} is independently selected from deuterium, halogen, hydroxy, amino, cyano, -R^{e4}, - OR^{e4}, -SO₂(R^{e4}), -C(O)R^{e4},
-N(R^{e4})(R^{e5}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen, or C₁₋₆ alkyl or C₃₋₆ membered cycloalkyl optionally substituted with one or more groups selected from hydrogen, halogen, hydroxy, amino, and cyano;
or two R^{e}, together with the atoms to which they are attached, form an optionally substituted 5-7 membered heterocyclyl, and optionally substituted means unsubstituted or substituted with one or more (for example, 2, 3, or 4) groups selected from deuterium, halogen, hydroxy, amino, nitro, mercapto, cyano, oxo, methyl, ethyl, methoxy, and cyclopropyl;
L₁ is independently selected from a bond;
m, n, o, p, q, and r are each independently 0, 1, 2, or 3;
provided that, when q is 2, R^{e} is F and methyl, respectively, and m is 3, 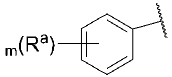 is not

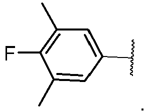

In some embodiments, the compound as shown in formula (III) is a compound as shown in formula (III-1): 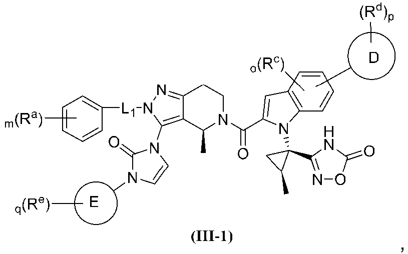
wherein Ring E, Ring D, R^{a}, R^{c}, R^{d}, R^{e}, L¹, m, o, p, and q are as defined for the compound of formula (III). In some embodiments, the compound as shown in formula (III) is a compound as shown in formula (III-2): 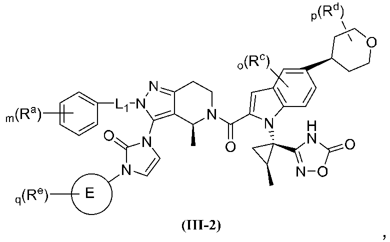
wherein Ring E, R^{a}, R^{c}, R^{d}, R^{e}, L¹, m, o, p, and q are as defined for the compound of formula (III).

In some embodiments, in the foregoing compounds (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula III-1, and formula III-2), the proviso is that, when L₄ is 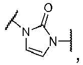 at least 1, for example, 1 or 2, of R^{a} or the substituent on Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene; or, when neither R^{a} nor the substituents on the Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene, two R^{e} (preferably two R^{e} located on different rings), together with atoms to which they are each attached, form optionally substituted 5-7 membered heterocyclyl, and the heterocyclyl (preferably heterocycloalkyl) contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; the R^{e3} is as defined for the compound of formula (I);
preferably, the C₃₋₅ cycloalkyl is selected from cyclopropyl, cyclobutyl, and cyclopentyl; further preferably cyclopropyl;
provided that the following compounds are not included:

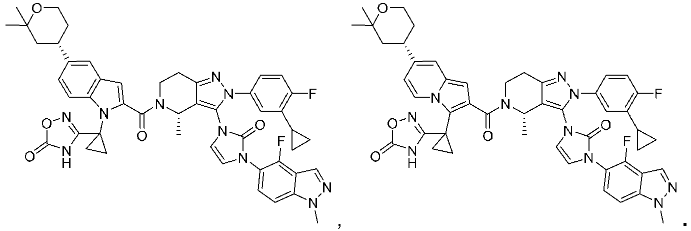

In some embodiments, in the foregoing compounds (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula III-1, and formula III-2), the proviso is that at least 1, for example, 1 or 2, of R^{a} or the substituent on Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene; or, when neither R^{a} nor the substituents on the Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene, two R^{e} (preferably two R^{e} located on different rings), together with atoms to which they are each attached, form optionally substituted 5-7 membered heterocyclyl, and the heterocyclyl (preferably heterocycloalkyl) contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; the R^{e3} is as defined for the compound of formula (I);
preferably, the C₃₋₅ cycloalkyl is selected from cyclopropyl, cyclobutyl, and cyclopentyl; further preferably cyclopropyl.

In some embodiments, two R^{e} (preferably two adjacent R^{e}; further preferably, two adjacent R^{e} located on different rings), together with the atoms to which they are respectively attached, form an optionally substituted 6-7 membered heterocyclyl, and the heterocyclyl (preferably heterocycloalkyl) contains 1-3 (for example, 1, 2, or 3) heteroatoms each independently selected from N, O, or S; preferably, the 6-7 membered heterocyclyl and Ring E form a fused tricyclic ring (i.e., the heterocyclyl and Ring E share 1 chemical bond (i.e., share 2 adjacent atoms), or 2 adjacent chemical bonds (i.e., share 3 adjacent atoms)); preferably, the 6-7 membered heterocyclyl is selected from 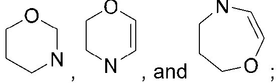 preferably, the 6-7 membered heterocyclyl is selected from 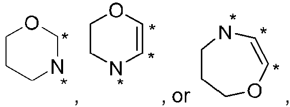 wherein * represents an atom shared with Ring E; preferably, the fused tricyclic ring formed by the 6-7 membered heterocyclyl and Ring E is selected from

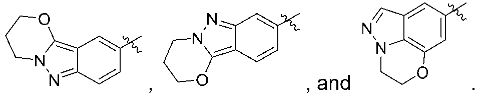

In some embodiments, wherein the compound as shown in formula (I), formula (II), or formulae (1-1) to (I-8) is a compound as shown in formula (IV): 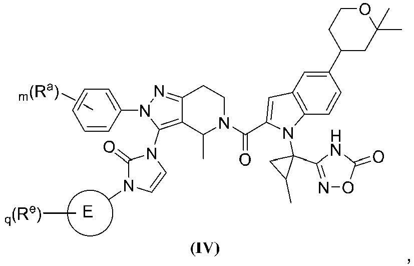
the Ring E is selected from 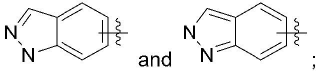
R^{a}, at each occurrence, is each independently selected from fluoro, methyl, and C₃₋₅ cycloalkyl;
m is 2 or 3;
q is 2 or 3;
R^{e}, at each occurrence, is each independently selected from fluoro or the following optionally substituted groups: C₁₋₃ alkyl (for example, methyl or ethyl) and C₃₋₅ cycloalkyl; provided that at least 1 (for example, 1 or 2) of R^{a} or R^{e} is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene;
or two R^{e} (preferably two adjacent R^{e}; further preferably, two R^{e} that are located on different rings and are adjacent), together with atoms to which they are each attached, form optionally substituted 5-7 membered heterocyclyl, and the heterocyclyl contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3};
R^{e3} is independently selected from deuterium, halogen (for example, fluoro or chloro), hydroxy, amino, oxo, cyano, -R^{e4}, -OR^{e4}, -S(O)₂R^{e4}, -C(O)R^{e4}, -N(R^{e4})(R^{e5}), -S(O)₂N(R^{e4})(R^{e5}), -N(R^{e4})S(O)₂R^{e5}, and - P(O)(R^{e4})R^{e5}; R^{e4} and R^{e5} are respectively independently selected from hydrogen or C₁₋₃ alkyl (for example, methyl, ethyl, and propyl) or C₃₋₆ membered cycloalkyl (preferably, 3-5 membered cycloalkyl, for example, cyclopropyl, cyclobutyl, and cyclopentyl) optionally substituted with one or more (for example, 2 or 3) of hydrogen, deuterium, halogen (for example, fluoro or chloro), hydroxy, amino, cyano, oxo, and methoxy;
preferably, the C₃₋₅ cycloalkyl is selected from cyclopropyl, cyclobutyl, and cyclopentyl; further preferably cyclopropyl.

In some embodiments, two R^{e} (preferably two adjacent R^{e}; further preferably, two adjacent R^{e} located on different rings), together with the atoms to which they are respectively attached, form an optionally substituted 6-7 membered heterocyclyl, and the heterocyclyl (preferably heterocycloalkyl) contains 1-3 (for example, 1, 2, or 3) heteroatoms each independently selected from N, O, or S; preferably, the 6-7 membered heterocyclyl and Ring E form a fused tricyclic ring (i.e., the heterocyclyl and Ring E share 1 chemical bond (i.e., share 2 adjacent atoms), or 2 adjacent chemical bonds (i.e., share 3 adjacent atoms)); preferably, the 6-7 membered heterocyclyl is selected from 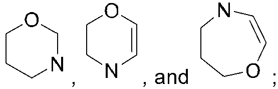 preferably, the 6-7 membered heterocyclyl is selected from 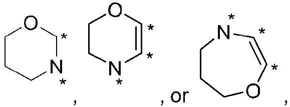 wherein * represents an atom shared with Ring E; preferably, the fused tricyclic ring formed by the 6-7 membered heterocyclyl and Ring E is selected from

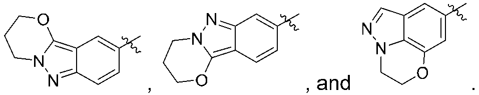

In some embodiments, the proviso is that the following compounds are not included:

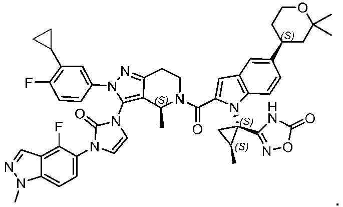

In some embodiments, wherein 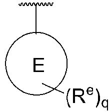 is 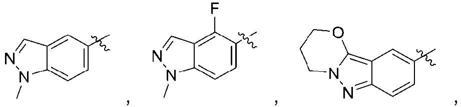 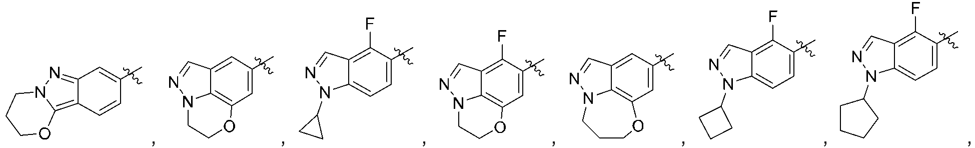 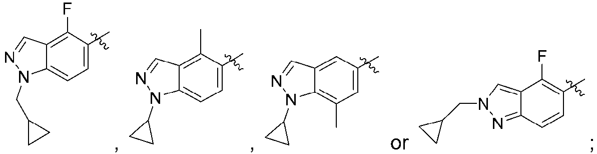 preferably, 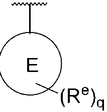 is

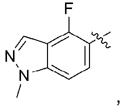

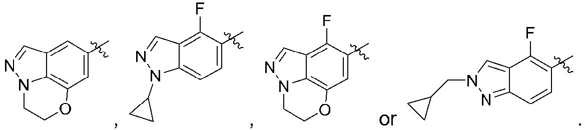

In some embodiments, wherein 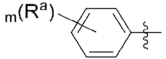 is

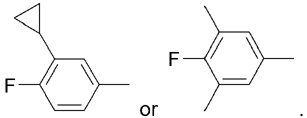

In some embodiments, wherein when 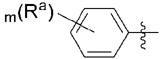 is 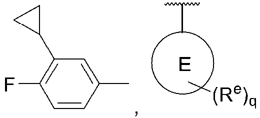 is 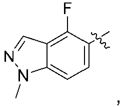 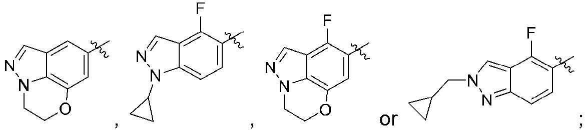 preferably, 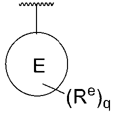 is 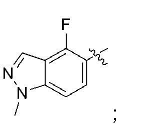 when 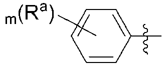 is 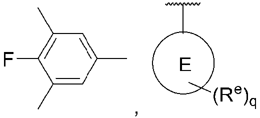 is 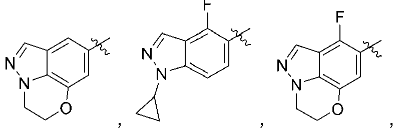 or

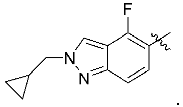

In some embodiments, wherein the compound as shown in formula (IV) is a compound as shown in formula (IV-1): 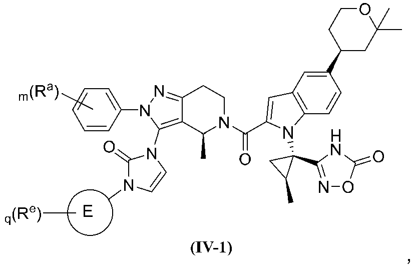 wherein Ring E, m, q, R^{a}, and R^{e} are as defined for the compound as shown in formula (IV).

In some embodiments, wherein the compound as shown in formula (I), formula (II), or formulae (1-1) to (I-8) is a compound as shown in formulae (V) to (VIII): 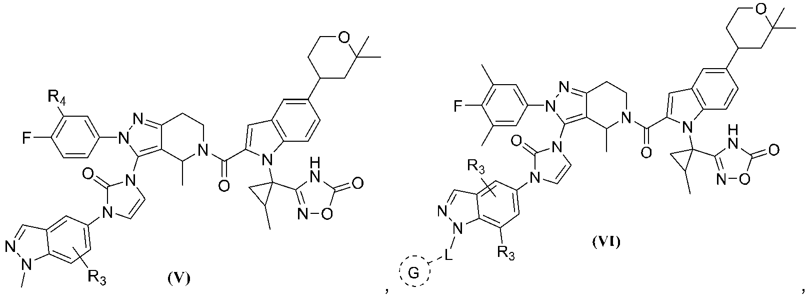 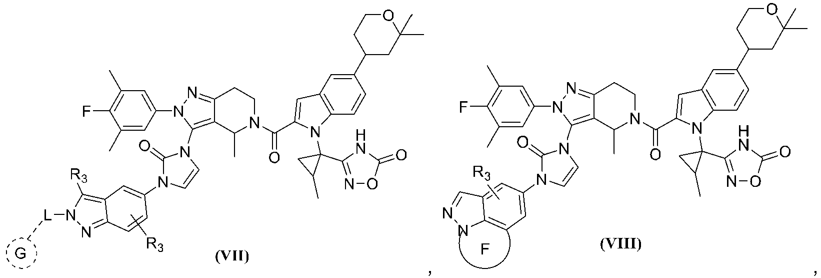 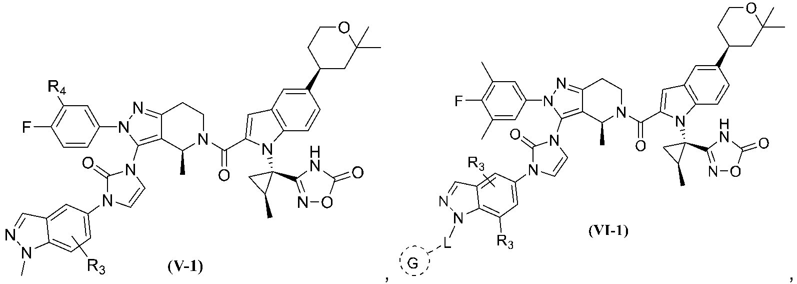 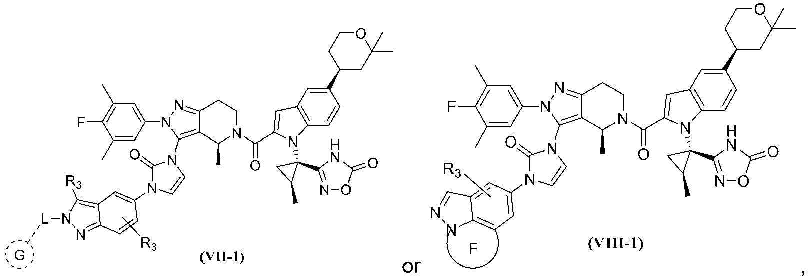
wherein 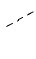 indicates that the bond is present or absent; 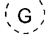
indicates that Ring G is present or absent; when the Ring G is present, the Ring G is C₃₋₅ cycloalkyl (for example, cyclopropyl, cyclobutyl, and cyclopentyl; preferably cyclopropyl);
L is a bond, C₁₋₃ alkylene (for example, methylene, ethylene, or propylene; preferably methylene), or C₁₋₃ alkyl (for example, methyl, ethyl, or propyl; preferably methyl);
or L cyclizes with R₃ adjacent thereto to form optionally substituted 5-7 membered heterocyclyl, and the heterocyclyl contains 1-3 (for example, 1, 2, or 3) heteroatoms each independently selected from N, O, or S, in which case the Ring G is absent;
R₃ is selected from H, F, or methyl;
R₄ is selected from C₃₋₅ cycloalkyl (for example, cyclopropyl, cyclobutyl, and cyclopentyl; preferably cyclopropyl);
Ring F is optionally substituted 5-7 membered heterocyclyl, and the heterocyclyl contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; R^{e3} is independently selected from deuterium, halogen (for example, fluoro or chloro), oxo, hydroxy, amino, cyano, -R^{e4}, -OR^{e4}, -SO₂(R^{e4}), -C(O)R^{e4}, -N(R^{e4})(R^{e5}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or C₁₋₃ alkyl (for example, methyl, ethyl, and propyl) or C₃₋₆ membered cycloalkyl (preferably, 3-5 membered cycloalkyl, for example, cyclopropyl, cyclobutyl, and cyclopentyl) optionally substituted with one or more (for example, 2 or 3) of hydrogen, halogen (for example, fluoro or chloro), hydroxy, amino, cyano, oxo, and methoxy.

In some embodiments, L cyclizes with adjacent R₃ to form an optionally substituted 6-7 membered heterocyclyl, wherein the heterocyclyl contains 1-3 (for example, 1, 2, or 3) heteroatoms each independently selected from N, O, or S; preferably, the 6-7 membered heterocyclyl is selected from 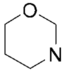 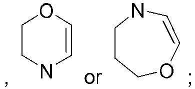 preferably, the 6-7 membered heterocyclyl is selected from 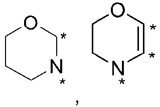 or 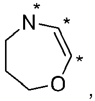 wherein * represents a shared atom.

In some embodiments, Ring F is an optionally substituted 6-7 membered heterocyclyl, wherein the heterocyclyl contains 1-3 (for example, 1, 2, or 3) heteroatoms each independently selected from N, O, or S; preferably, the 6-7 membered heterocyclyl is selected from 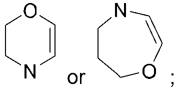 preferably, the 6-7 membered heterocyclyl is selected from 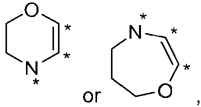 wherein * represents a shared atom.

In some embodiments, in formula (VIII) or formula (VIII-1), 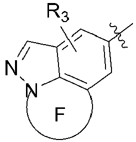 is

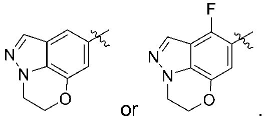

In some embodiments, wherein the compounds as shown in formulae (V) to (VIII) and formulae (V-1) to (VIII-1) are compounds as shown in formulae (V-2) to (VIII-2): 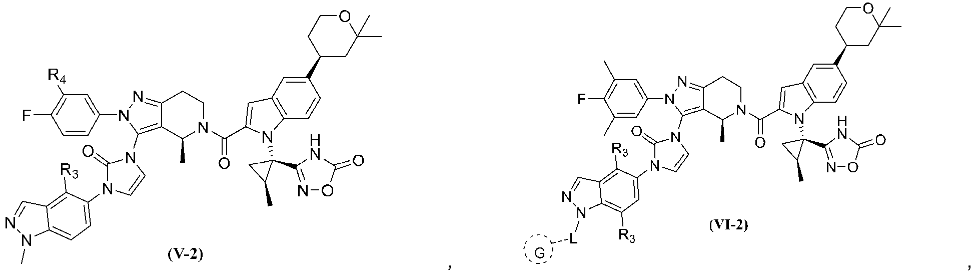 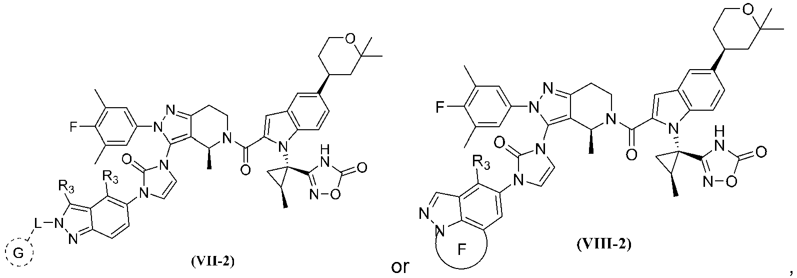 wherein R, R₃, L, and Ring F are as defined for the compounds as shown in formulae (V) to (VIII) and formulae (V-1) to (VIII-1).

On the basis of common general knowledge in the art, the above preferred conditions may be combined in any manner to obtain various preferred embodiments of the present invention.

In some embodiments, the present invention provides the following compounds, or tautomers, stereoisomers, or pharmaceutically acceptable salts thereof, wherein the compounds have the following structures:

| **Compound Structural Formula and No.** | **Compound Structural Formula and No.** |
|---|---|
| 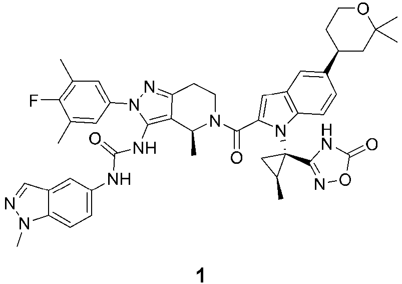 | 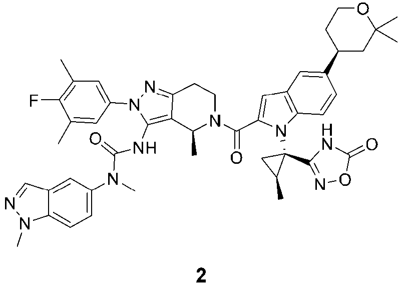 |
| 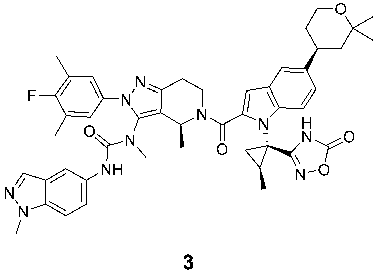 | 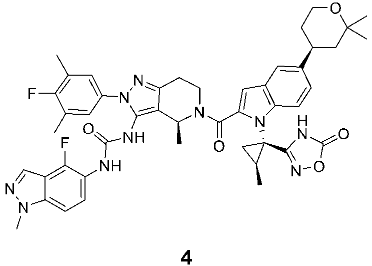 |
| 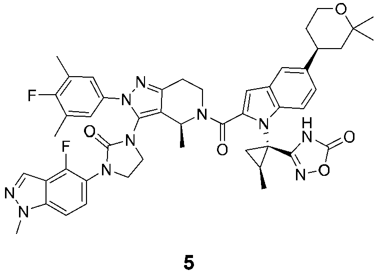 | 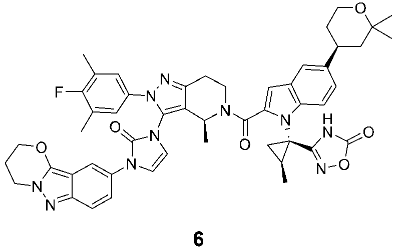 |
| 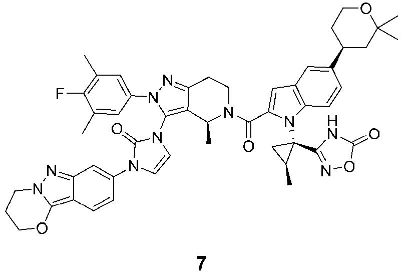 | 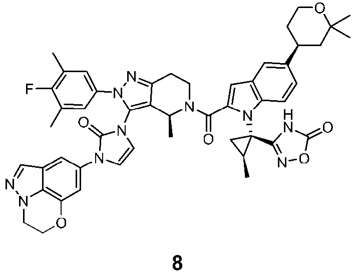 |
| 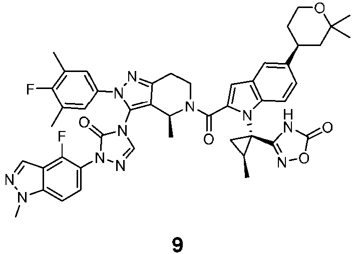 | 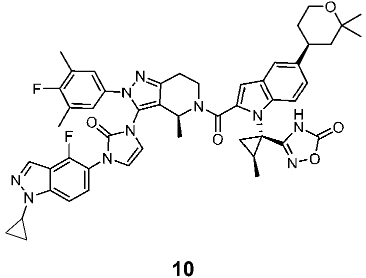 |
| 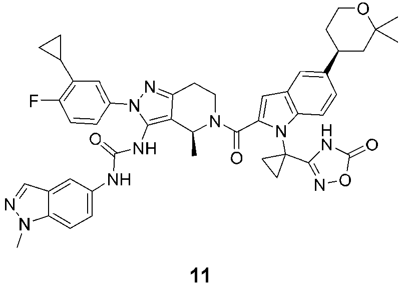 | 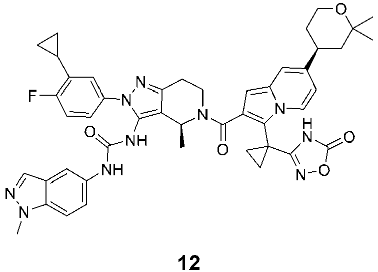 |
| 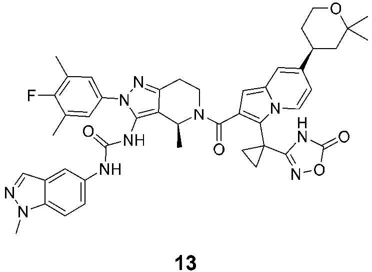 | 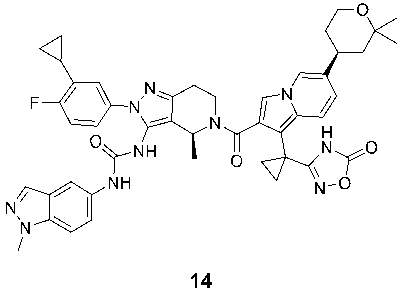 |
| 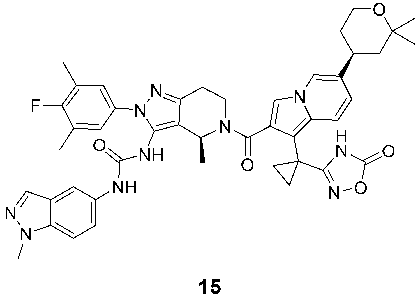 | 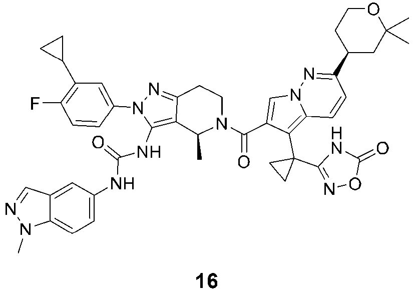 |
| 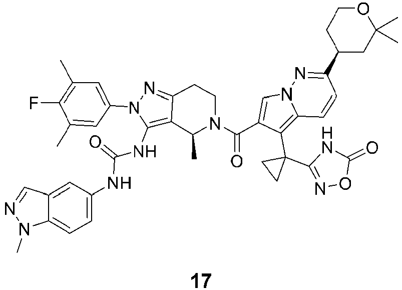 | 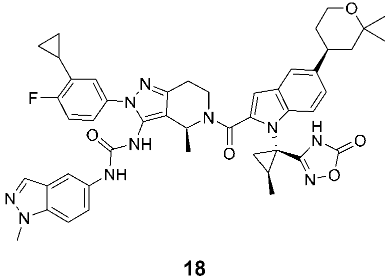 |
| 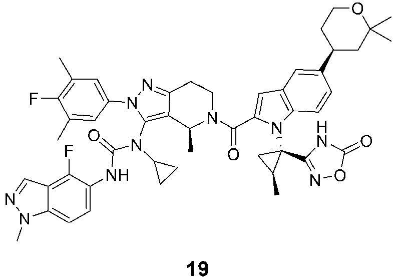 | 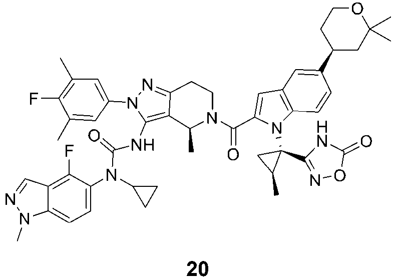 |
| 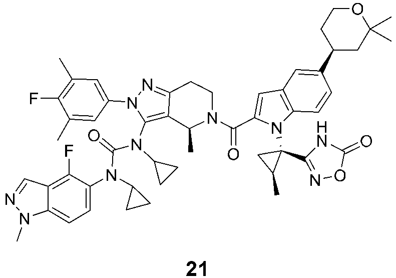 | 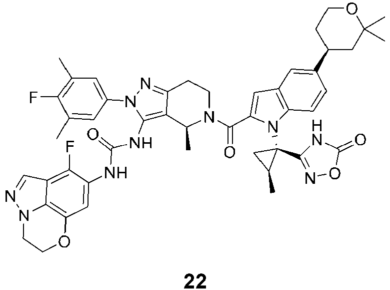 |
| 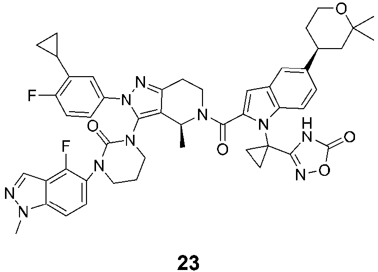 | 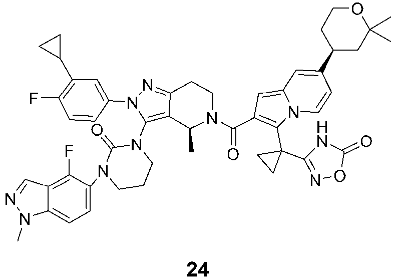 |
| 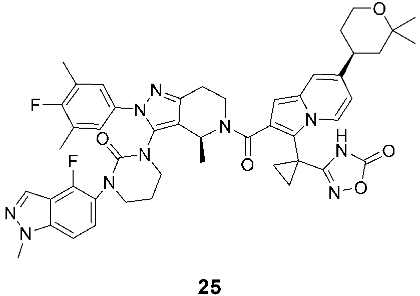 | 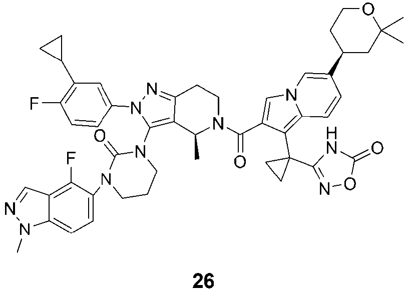 |
| 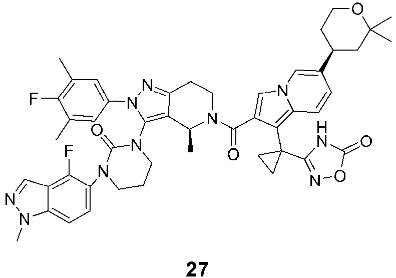 | 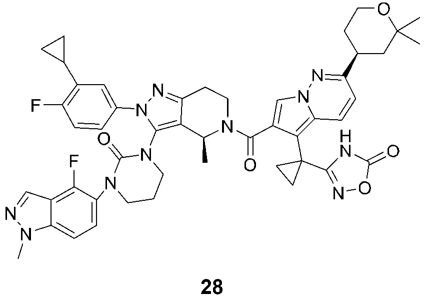 |
| 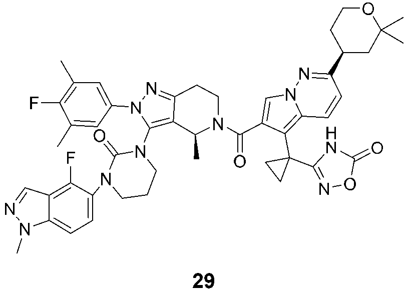 | 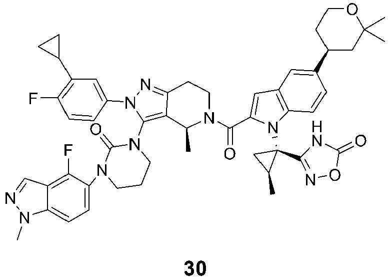 |
| 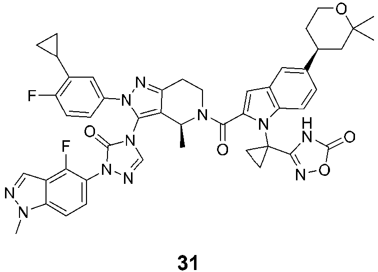 | 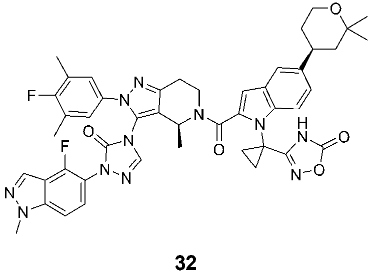 |
| 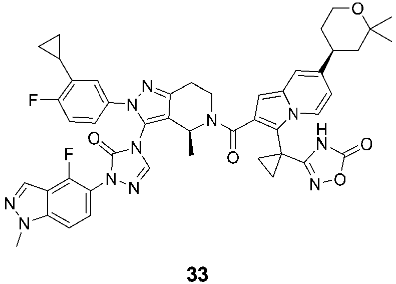 | 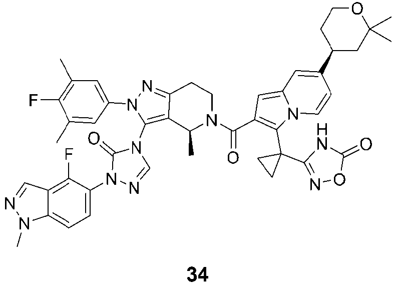 |
| 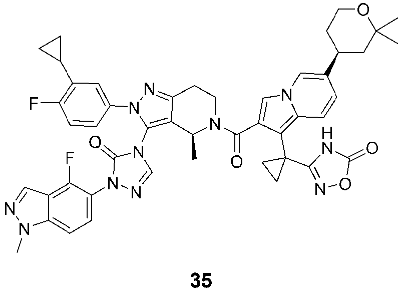 | 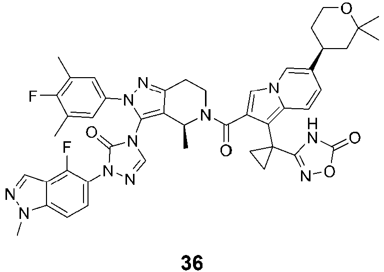 |
| 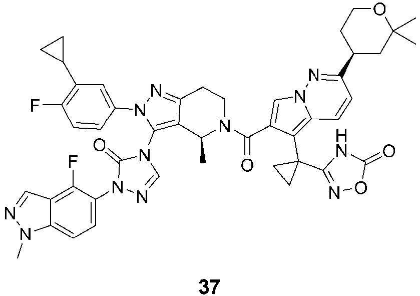 | 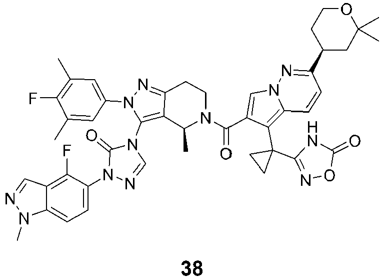 |
| 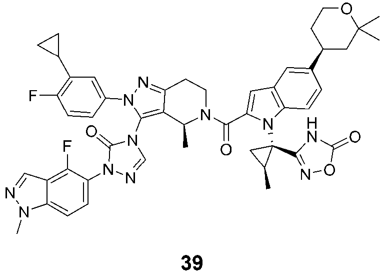 | 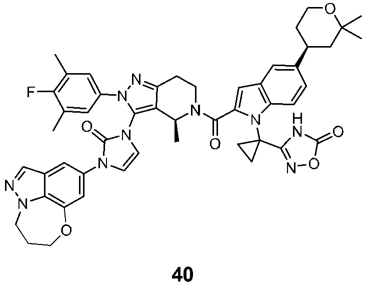 |
| 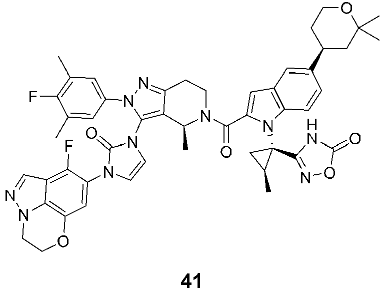 | 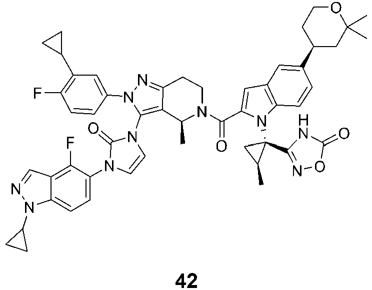 |
| 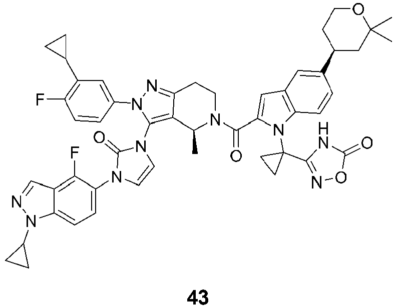 | 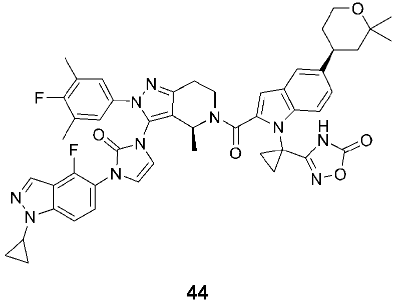 |
| 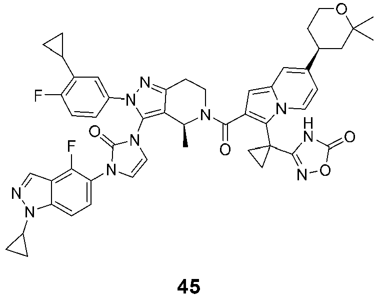 | 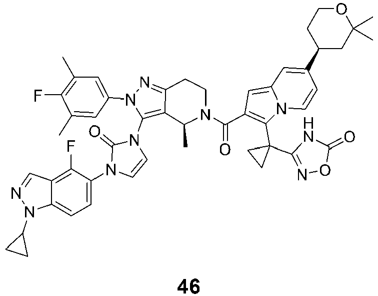 |
| 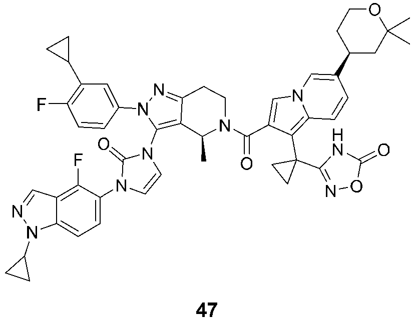 | 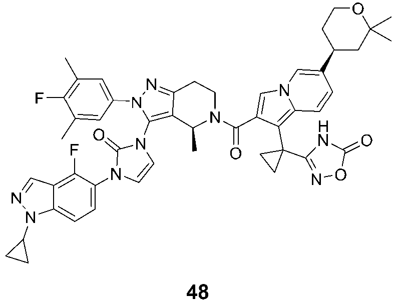 |
| 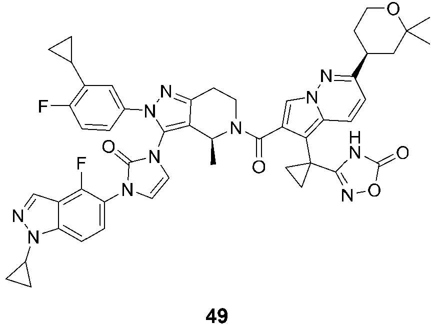 | 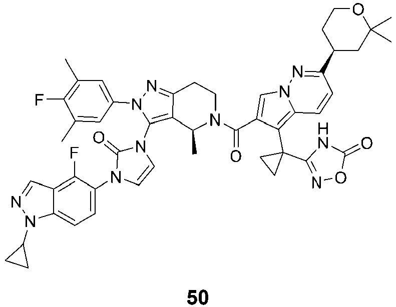 |
| 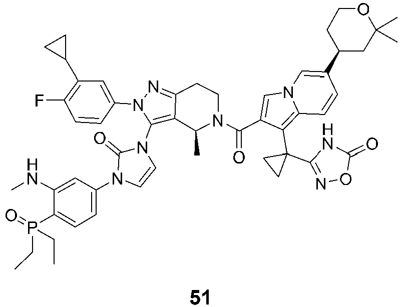 | 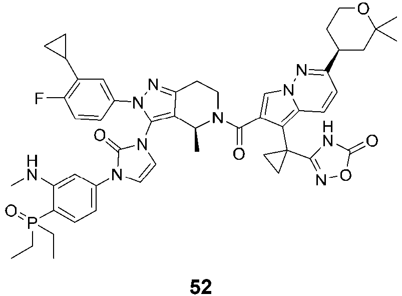 |
| 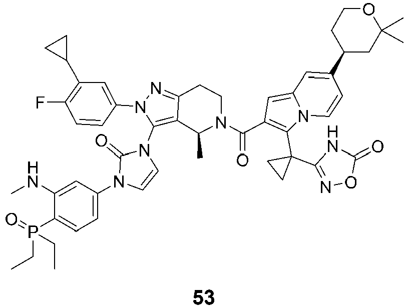 | 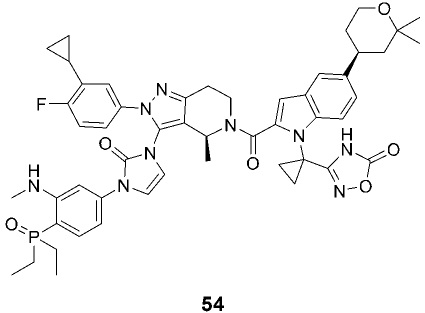 |
| 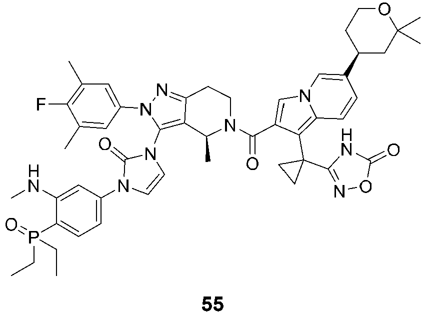 | 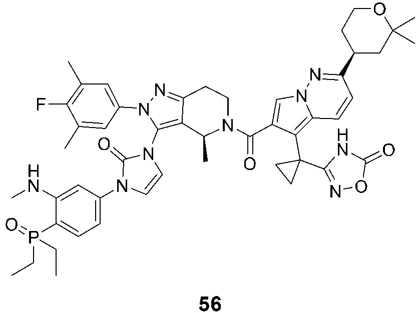 |
| 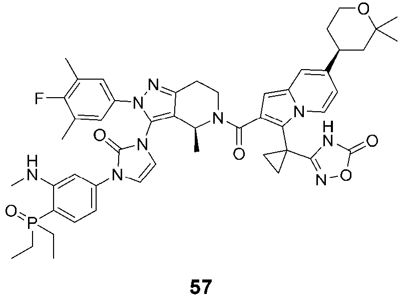 | 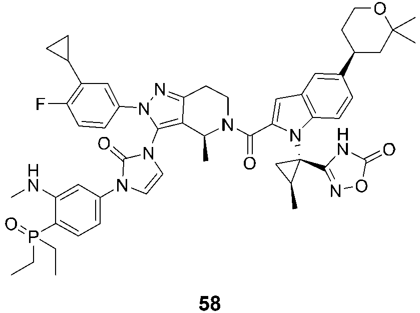 |
| 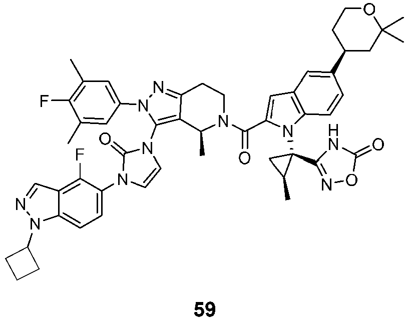 | 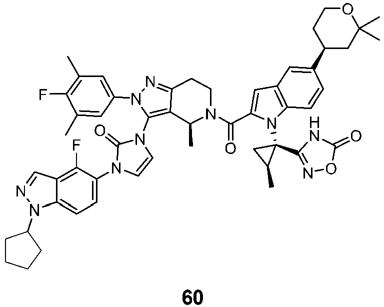 |
| 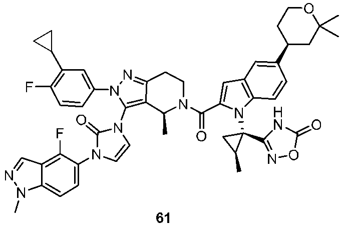 | 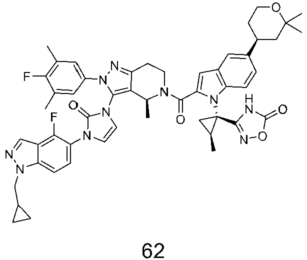 |
| 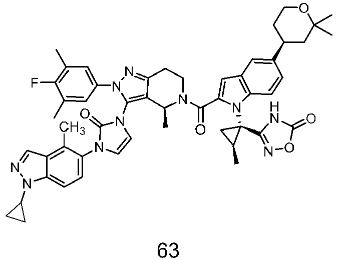 | 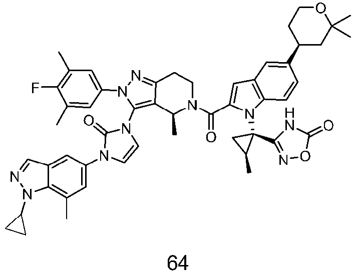 |
| 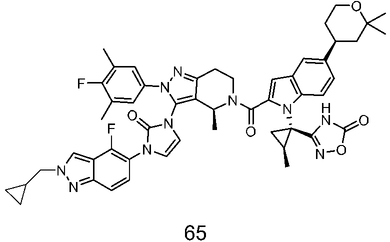 | |

In a second aspect, the present invention provides a method for preparing the compound as shown in formula (I), or tautomers, stereoisomers, or pharmaceutically acceptable salts thereof,
wherein the method may, for example, be carried out using the method shown in the following scheme, 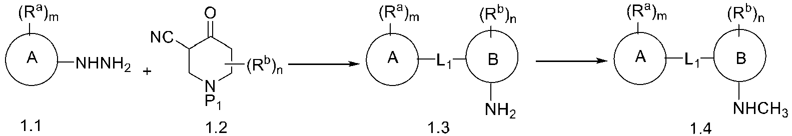 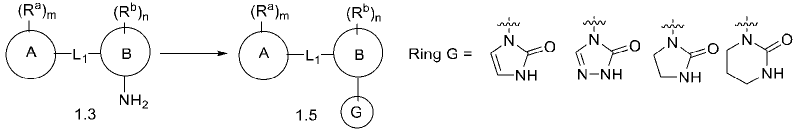 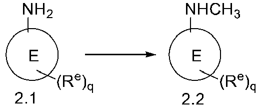 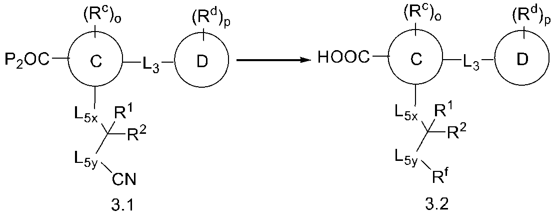 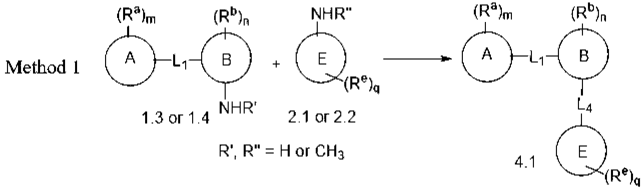 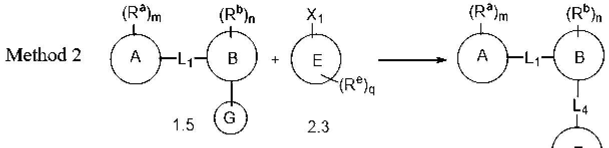 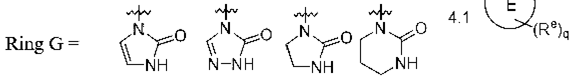
the hydrazino group of intermediate 1.1 is reacted with intermediate 1.2 to obtain intermediate 1.3, and intermediate 1.3 is alkylated to obtain intermediate 1.4; the amino group of intermediate 1.3 is reacted to obtain intermediate 1.5; intermediate 2.1 is alkylated to obtain intermediate 2.2; the cyano group of intermediate 3.1 is converted by reaction into intermediate 3.2; there are two methods for synthesizing intermediate 4.1:
   Method 1: the amino group of intermediate 1.3 or 1.4 is reacted with the amino group of intermediate 2.1 or 2.2 to obtain intermediate 4.1;
   Method 2: intermediate 1.5 and intermediate 2.3 are subjected to an Ullmann reaction to obtain intermediate 4.1;

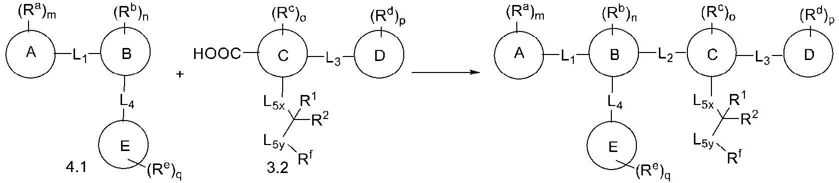

intermediate 4.1 is reacted with the carboxyl group of intermediate 3.2 to obtain the target compound; Note: some protection and deprotection reaction steps involved in this step are omitted;
P₁ is a protecting group (for example, Boc, Bn, Cbz, etc.), and X₁ is a leaving group, (for example, I, Cl, Br, etc.);
Ring A, Ring B, Ring C, Ring D, Ring E, R^{a}, R^{b}, R^{c}, R^{d}, R^{e}, R¹, R², L₁, L₂, L₃, R^{f}, R^{e}, L₄, L₅ₓ, L_{5y}, m, n, o, p, q, and the like are as defined for the compound of formula (I).

In a third aspect, the present invention provides a pharmaceutical composition comprising a compound of the present invention (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, and optionally further comprising a pharmaceutically acceptable excipient.

The compound of the present invention (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, may be administered in a pure form or in the form of a suitable pharmaceutical composition by any acceptable route of administration for drugs having similar uses. The pharmaceutical composition of the present invention may be prepared by combining the compound of the present invention with a suitable pharmaceutically acceptable excipient. The pharmaceutical composition of the present invention may be formulated as a solid, semi-solid, liquid, or gaseous formulation. Generally, the above pharmaceutical composition may be prepared by a conventional preparation method using conventional excipients in the field of formulations.

In a fourth aspect, the present invention provides use of the compound described above (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, or the pharmaceutical composition of the present invention, as a drug or in the manufacture of a drug; preferably, the drug is a GLP-1R agonist; further preferably, the drug is a drug for treating and/or preventing disorders related to GLP-1R; further preferably, the drug is a drug for treating and/or preventing disorders mediated with the involvement of GLP-1R; further preferably, the drug is a drug for treating and/or preventing disorders caused by reduced GLP-1R activity or mediated with the involvement of GLP-1R; further preferably, the drug is a drug for preventing and/or treating disorders by agonizing GLP-1R-mediated cascade signals; further preferably, the disorders comprise diabetes, overweight or obesity, non-alcoholic fatty liver disease, Alzheimer's disease, and the like; further preferably, the diabetes is type 2 diabetes.

In some embodiments, in the use, wherein the drug is a drug for treating and/or preventing diabetes, overweight or obesity, non-alcoholic fatty liver disease, or Alzheimer's disease; preferably, the drug is a drug for treating and/or preventing diabetes, overweight or obesity; further preferably, the diabetes is type 2 diabetes.

In a fifth aspect, the present invention provides the compound described above (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, or the pharmaceutical composition, for use in treating and/or preventing a disorder; preferably, the disorder is a GLP-1R-related disorder; further preferably, the disorder is a disorder mediated with the involvement of GLP-1R; further preferably, the disorder is a disorder caused by reduced GLP-1R activity or mediated with the involvement of GLP-1R; further preferably, the disorders comprise diabetes, overweight or obesity, non-alcoholic fatty liver disease, Alzheimer's disease, and the like; further preferably, the disorders comprise diabetes, overweight or obesity; further preferably, the diabetes is type 2 diabetes.

In a sixth aspect, the present application provides a method for treating a disorder, the method comprising administering to a subject in need thereof a therapeutically effective amount of the compound described above (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, or the pharmaceutical composition of the present invention; preferably, the disorder is a GLP-1R-related disorder; further preferably, the disorder is a disorder mediated with the involvement of GLP-1R; further preferably, the disorder is a disorder caused by reduced GLP-1R activity or mediated with the involvement of GLP-1R; further preferably, the disorders comprise diabetes, overweight or obesity, non-alcoholic fatty liver disease, Alzheimer's disease, and the like; further preferably, the disorders comprise diabetes, overweight or obesity; further preferably, the diabetes is type 2 diabetes.

In some embodiments, the present invention provides the use or method, wherein the compound of the present invention (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, or the pharmaceutical composition of the present invention is used in combination with one, two, or more other compounds or drugs having the same or similar indications.

The present invention further provides a composition comprising the compound of the present invention (formula I, formula I-1, formula I-2, formula I-3, formula I-4, formula I-5, formula I-6, formula I-7, formula I-8, formula I-9, formula I-10, formula I-11, formula I-12, formula I-13, formula I-14, formula I-15, formula I-16, formula I-17, formula I-18, formula I-19, formula I-20, formula II, formula II-1, formula II-2, formula III, formula IV, formula IV-1, formula V, formula VI, formula VII, formula VIII, formula V-1, formula VI-1, formula VII-1, formula VIII-1, formula V-2, formula VI-2, formula VII-2, formula VIII-2, or compounds 1-65), or tautomers, stereoisomers, or pharmaceutically acceptable salts thereof, or the pharmaceutical composition of the present invention, and one, two, or more other compounds having the same or similar indications.

### Definitions

Unless otherwise specified, the following terms used in the present invention have the following meanings: A specific term, in the absence of a special definition, should not be considered indefinite or unclear, but should be understood according to its ordinary meaning in the art. Where a trade name appears herein, it is intended to refer to the corresponding commercial product or its active ingredient.

Cₘ₋ₙ herein means that the moiety has an integer number of carbon atoms within the given range. For example, "C₁₋₆" means that the group may have 1 carbon atom, 2 carbon atoms, 3 carbon atoms, 4 carbon atoms, 5 carbon atoms, or 6 carbon atoms.

When any variable (for example R^{a}) occurs more than once in the composition or structure of a compound, its definition in each case is independent. Thus, for example, if a group, a site, or an atom is substituted with two R^{a}, each R^{a} has independent options.

"A plurality" herein refers to 2 to 10, for example, 2, 3, 4, 5, 6, 7, 8, 9, or 10, preferably 2, 3, 4, 5, 6, 7, 8, or 9; preferably 2, 3, 4, 5, 6, 7, or 8; preferably 2, 3, 4, or 5; preferably 2 or 3.

"R^{b}, at each occurrence, is independently selected from deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl" should be understood as "R^{b}, at each occurrence, is independently selected from deuterium, halogen, hydroxy, amino, nitro, mercapto, cyano, oxo, optionally substituted C₁₋₆ alkyl, optionally substituted C₁₋₆ alkoxy, optionally substituted C₁₋₆ alkylamino, optionally substituted C₂₋₆ alkenyl, optionally substituted C₂₋₆ alkynyl, optionally substituted C₃₋₁₀ cycloalkyl, optionally substituted 3-10 membered heterocyclyl, optionally substituted C₆₋₁₄ aryl, and optionally substituted 5-12 membered heteroaryl". Other similar expressions in the present invention should be understood with reference to the above manner of understanding.

The terms "optional", "optionally", "arbitrary", or "arbitrarily" mean that the subsequently described event or circumstance may, but need not, occur, and that the description includes instances in which the event or circumstance occurs and instances in which it does not occur.

The term "oxo" means that two hydrogen atoms at the same substitution position are replaced by the same oxygen atom to form a double bond.

Unless otherwise specified, the term "alkyl" refers to a monovalent saturated aliphatic hydrocarbon group, including a straight-chain or branched group containing 1 to 20 carbon atoms, preferably containing 1 to 10 carbon atoms (i.e., C₁₋₁₀ alkyl), further preferably containing 1 to 8 carbon atoms (i.e., C₁₋₈ alkyl), more preferably containing 1 to 6 carbon atoms (i.e., C₁₋₆ alkyl), more preferably containing 1 to 4 carbon atoms (i.e., C₁₋₄ alkyl), and more preferably containing 1 to 3 carbon atoms (i.e., C₁₋₃ alkyl). For example, "C₁₋₆ alkyl" means that the group is an alkyl group and the number of carbon atoms in the carbon chain is between 1 and 6, specifically 1, 2, 3, 4, 5, or 6. Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, and the like. Unless otherwise specified, the term "alkenyl" refers to a straight-chain or branched unsaturated aliphatic hydrocarbon group composed of carbon atoms and hydrogen atoms and having at least one double bond. The alkenyl may contain 2-20 carbon atoms, preferably 2-10 carbon atoms (i.e., C₂₋₁₀ alkenyl), further preferably 2-8 carbon atoms (i.e., C₂₋₈ alkenyl), more preferably 2-6 carbon atoms (i.e., C₂₋₆ alkenyl), 2-5 carbon atoms (i.e., C₂₋₅ alkenyl), 2-4 carbon atoms (i.e., C₂₋₄ alkenyl), 2-3 carbon atoms (i.e., C₂₋₃ alkenyl), or 2 carbon atoms (i.e., C₂ alkenyl). For example, "C₂₋₆ alkenyl" means that the group is an alkenyl group and the number of carbon atoms in the carbon chain is between 2 and 6 (specifically 2, 3, 4, 5, or 6). Nonlimiting examples of alkenyl include, but are not limited to, vinyl, 1-propenyl, 2-propenyl, 1-butenyl, isobutenyl, 1,3-butadienyl, and the like.

Unless otherwise specified, the term "alkynyl" refers to a straight-chain or branched unsaturated aliphatic hydrocarbon group composed of carbon atoms and hydrogen atoms and having at least one triple bond. The alkynyl may contain 2-20 carbon atoms, preferably 2-10 carbon atoms (i.e., C₂₋₁₀ alkynyl), further preferably 2-8 carbon atoms (i.e., C₂₋₈ alkynyl), more preferably 2-6 carbon atoms (i.e., C₂₋₆ alkynyl), 2-5 carbon atoms (i.e., C₂₋₅ alkynyl), 2-4 carbon atoms (i.e., C₂₋₄ alkynyl), 2-3 carbon atoms (i.e., C₂₋₃ alkynyl), or 2 carbon atoms (i.e., C₂ alkynyl). For example, "C₂₋₆ alkynyl" means that the group is an alkynyl group and the number of carbon atoms in the carbon chain is between 2-6 (specifically 2, 3, 4, 5, or 6). Nonlimiting examples of alkynyl include, but are not limited to, ethynyl, 1-propynyl, 2-propynyl, 1-butynyl, and the like.

Unless otherwise specified, the term "alkoxy" refers to -O-alkyl, wherein the alkyl is as defined above, i.e., containing 1-20 carbon atoms, preferably containing 1-10 carbon atoms, more preferably 1-8 carbon atoms, further preferably 1-6 carbon atoms (specifically 1, 2, 3, 4, 5, or 6), further preferably 1-4 carbon atoms, further preferably 1-3 carbon atoms, and further preferably 1-2 carbon atoms. Representative examples include, but are not limited to, methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, and the like.

Unless otherwise specified, the term "alkylamino" refers to -NR'R", wherein R' and R" are the same or different and may be H or alkyl as defined above, wherein the alkyl is as defined above, i.e., containing 1-20 carbon atoms, preferably containing 1-10 carbon atoms, more preferably 1-8 carbon atoms, further preferably 1-6 carbon atoms (specifically 1, 2, 3, 4, 5, or 6) further preferably 1-4 carbon atoms, further preferably 1-3 carbon atoms, and further preferably 1-2 carbon atoms. Representative examples include, but are not limited to, -NH(CH₃), -N(CH₃)(CH₃), -N(CH₂CH₃)(CH₃), -N(CH₂CH₃)[CH(CH₃)₂], and the like.

Unless otherwise specified, the term "halogen" or "halo" refers to F, Cl, Br, or I. The term "haloalkyl" refers the fact that one, two, or more hydrogen atoms, or all hydrogen atoms in alkyl as defined above are substituted with halogen. Representative examples of haloalkyl include CCl₃, CF₃, CHCl₂, CH₂Cl, CH₂Br, CH₂I, CH₂CF₃, CF₂CF₃, and the like.

Unless otherwise specified, the term "carbocyclyl" or "carbocycle" refers to a non-aromatic cyclic hydrocarbon group ("C₃₋₁₅ carbocyclyl") having 3-15 ring carbon atoms, and no heteroatoms exist in the non-aromatic ring system. In some embodiments, a carbocyclyl group has 3-15 ring carbon atoms ("C₃₋₁₅ carbocyclyl"), 3-14 ring carbon atoms ("C₃₋₁₄ carbocyclyl"), 3-12 ring carbon atoms ("C₃₋₁₂ carbocyclyl"), 4-12 ring carbon atoms ("C₄₋₁₂ carbocyclyl"), or 3-10 ring carbon atoms ("C₃₋₁₀ carbocyclyl"). In some embodiments, a carbocyclyl group has 3-8 ring carbon atoms ("C₃₋₈ carbocyclyl"). In some embodiments, a carbocyclyl group has 3-7 ring carbon atoms ("C₃₋₇ carbocyclyl"). In some embodiments, a carbocyclyl group has 3-6 ring carbon atoms ("C₃₋₆ carbocyclyl"). In some embodiments, a carbocyclyl group has 4-6 ring carbon atoms ("C₄₋₆ carbocyclyl"). In some embodiments, a carbocyclyl group has 5-10 ring carbon atoms ("C₅₋₁₀ carbocyclyl"), 5-7 ring carbon atoms ("C₅₋₇ carbocyclyl"), or 3-5 ring carbon atoms ("C₃₋₅ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As illustrated by the examples above, in certain embodiments, the carbocyclyl group is monocyclic ("monocyclic carbocyclyl") or is a fused ("fused carbocyclyl"), bridged ("bridged carbocyclyl"), or spiro-fused ("spirocyclyl") ring system, such as a bicyclic system ("bicyclic carbocyclyl"), and may be saturated or partially unsaturated. "Carbocyclyl" also includes ring systems in which the carbocyclyl ring as defined above is fused with one or more aryl groups, wherein the point of attachment is on the carbocyclyl or aryl ring, and in such cases, the number of members of the carbocyclyl ring system is the number of carbon atoms in the fused carbocyclic system. In certain embodiments, each instance of a carbocyclyl group is independently optionally substituted, e.g., unsubstituted (an "unsubstituted carbocyclyl") or substituted with one or more substituents (a "substituted carbocyclyl"). In certain embodiments, the carbocyclyl group is an unsubstituted C₃₋₁₀ carbocyclyl. In certain embodiments, the carbocyclyl group is a substituted C₃₋₁₀ carbocyclyl.

Unless otherwise specified, the term "cycloalkyl" refers to a monocyclic saturated aliphatic hydrocarbon group having a specified number of carbon atoms, preferably containing 3-12 carbon atoms (i.e., C₃₋₁₂ cycloalkyl), more preferably 3-10 carbon atoms (C₃₋₁₀ cycloalkyl), further preferably 3-7 carbon atoms (C₃₋₇ cycloalkyl), 3-6 carbon atoms (C₃₋₆ cycloalkyl), 3-5 carbon atoms (C₃₋₅ cycloalkyl), 3-4 carbon atoms (C₃₋₄ cycloalkyl), 4-6 carbon atoms (C₄₋₆ cycloalkyl), or 5-6 carbon atoms (C₅₋₆ cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, and the like.

Unless otherwise specified, the term "heterocyclyl" or "heterocycle" refers to a saturated or partially unsaturated monocyclic or polycyclic cyclic non-aromatic substituent having ring carbon atoms and 1-4 ring heteroatoms, containing 3-20 ring atoms, wherein 1, 2, 3, or more ring atoms are selected from N, O, or S, and the remaining ring atoms are C. Preferably, it contains 3-12 ring atoms (3-12 membered heterocyclyl), further preferably 3-10 ring atoms (3-10 membered heterocyclyl), or 3-8 ring atoms (3-8 membered heterocyclyl), or 3-6 ring atoms (3-6 membered heterocyclyl), or 4-6 ring atoms (4-6 membered heterocyclyl), or 4-5 ring atoms (4-5 membered heterocyclyl), or 3-5 ring atoms (3-5 membered heterocyclyl), or 5-7 ring atoms (5-7 membered heterocyclyl), or 5-6 ring atoms (5-6 membered heterocyclyl). The number of heteroatoms is preferably 1-4, more preferably 1-3, i.e., 1, 2, or 3. Examples of monocyclic heterocyclyl include pyrrolidinyl, imidazolidinyl, tetrahydrofuranyl, dihydropyrrolyl, piperidinyl, piperazinyl, pyranyl, and the like. Polycyclic heterocyclyl includes fused-ring, spiro-ring, and bridged-ring heterocyclyl. "Heterocyclyl" may be monocyclic ("monocyclic heterocyclyl") or a fused ("fused heterocyclyl"), bridged ("heterobridged ring group" or "bridged heterocyclyl"), or spiro ("heterospirocyclyl", "spiroheterocyclyl", or "spirocyclic heterocyclyl") ring system, such as a bicyclic ring system ("bicyclic heterocyclyl"), and may be saturated or partially unsaturated. A heterocyclyl bicyclic ring system may include one or more heteroatoms in one or both rings. "Heterocyclyl" also includes ring systems in which the heterocyclyl ring as defined above is fused or annulated with one or more carbocyclyl groups, wherein the point of attachment is on the carbocyclyl or heterocyclyl ring; "heterocyclyl" also includes ring systems in which the heterocyclyl ring as defined above is fused or annulated with one or more aryl or heteroaryl groups, or ring systems in which the cycloalkyl ring as defined above is fused or annulated with one or more heteroaryl groups, wherein the point of attachment is on the heterocyclyl ring, cycloalkyl ring, aryl ring, or heteroaryl ring, and in such cases, the number of members of the heterocyclyl ring system is the number of atoms in the fused ring system. In certain embodiments, each instance of heterocyclyl is independently optionally substituted, e.g., unsubstituted ("unsubstituted heterocyclyl") or substituted with one or more substituents (a "substituted heterocyclyl"). Exemplary 3-membered heterocyclyl groups containing 1 heteroatom include, but are not limited to, aziridinyl, oxiranyl, and thiorenyl. Exemplary 4-membered heterocyclyl groups containing 1 heteroatom include, but are not limited to, azetidinyl, oxetanyl, and thietanyl. Exemplary 5-membered heterocyclyl groups containing 1 heteroatom include, but are not limited to, tetrahydrofuranyl, dihydrofuranyl, tetrahydrothienyl, dihydrothienyl, pyrrolidinyl, dihydropyrrolyl, and pyrrolyl-2,5-dione. Exemplary 5-membered heterocyclyl groups containing 2 heteroatoms include, but are not limited to, dioxolanyl, oxathiolanyl, dithiolanyl, and oxazolidin-2-one. Exemplary 5-membered heterocyclyl groups containing 3 heteroatoms include, but are not limited to, triazolinyl, oxadiazolinyl, and thiadiazolinyl. Exemplary 6-membered heterocyclyl groups containing 1 heteroatom include, but are not limited to, piperidinyl, tetrahydropyranyl, dihydropyridinyl, and thianyl. Exemplary 6-membered heterocyclyl groups containing 2 heteroatoms include, but are not limited to, piperazinyl, morpholinyl, dithianyl, and dioxanyl. Exemplary 6-membered heterocyclyl groups containing 3 heteroatoms include, but are not limited to, triazinanyl, oxadiazinanyl, thiadiazinanyl, oxathiazinanyl, and dioxazinanyl. Exemplary 7-membered heterocyclyl groups containing 1 heteroatom include, but are not limited to, azepanyl, oxepanyl, and thiepanyl. Exemplary 8-membered heterocyclyl groups containing 1 heteroatom include, but are not limited to, azocanyl, oxocanyl, and thiocanyl. Exemplary 5-membered heterocyclyl groups fused to a C₆ aryl ring, also referred to herein as a 5,6-bicyclic heterocycle, include, but are not limited to, dihydroindolyl, isoindolinyl, dihydrobenzofuranyl, dihydrobenzothienyl, benzoxazolinonyl, and the like. Exemplary 6-membered heterocyclyl groups fused to an aryl ring, also referred to herein as a 6,6-bicyclic heterocycle, include, but are not limited to, tetrahydroquinolyl, tetrahydroisoquinolyl, and the like.

Unless otherwise specified, the term "fused ring" refers to a non-aromatic, saturated or partially unsaturated ring system formed by two or more cyclic structures sharing two adjacent atoms with each other, including fused carbocyclyl and fused heterocyclyl. "Non-aromatic" means that the entire ring system is non-aromatic. A fused carbocyclyl may contain 5-14 ring atoms, preferably 6-12 ring atoms, more preferably 7-10 ring atoms, e.g., 7 ring atoms, 8 ring atoms, 9 ring atoms, or 10 ring atoms. Fused carbocyclyl includes bicyclic, tricyclic, tetracyclic, or polycyclic fused carbocyclyl, preferably bicyclic, tricyclic, or tetracyclic fused carbocyclyl, more preferably bicyclic or tricyclic fused carbocyclyl. Exemplary examples of fused carbocyclyl include, but are not limited to, 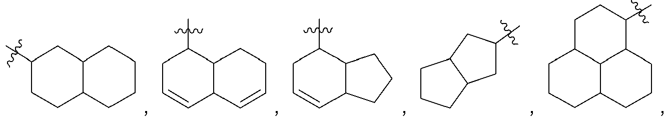 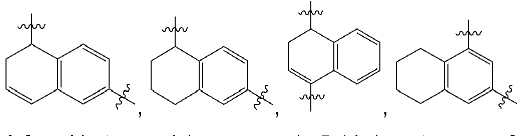 and the like.

A fused heterocyclyl may contain 5-14 ring atoms, preferably 6-12 ring atoms, more preferably 7-10 ring atoms, e.g., 7 ring atoms, 8 ring atoms, 9 ring atoms, or 10 ring atoms, and contains 1-4 ring heteroatoms, preferably 1-3, i.e., 1, 2, or 3, ring heteroatoms, wherein the heteroatoms are independently selected from N, O, and S. Fused heterocyclyl includes bicyclic, tricyclic, tetracyclic, or polycyclic fused heterocyclyl, preferably bicyclic, tricyclic, or tetracyclic fused heterocyclyl, more preferably bicyclic or tricyclic fused heterocyclyl. Exemplary examples of fused heterocyclyl include, but are not limited to, 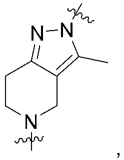 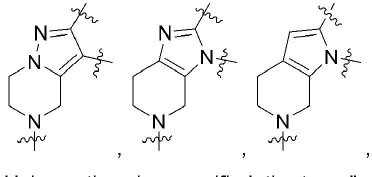 and the like.

Unless otherwise specified, the term "aryl" or "aryl ring group" refers to a monocyclic, bicyclic, or tricyclic aromatic carbocyclic ring system containing 6-16 carbon atoms, or 6-14 carbon atoms, or 6-12 carbon atoms, or 6-10 carbon atoms, or 6-8 carbon atoms, preferably 6-10 carbon atoms; the term "aryl" may be used interchangeably with the term "aromatic ring". Examples of aryl groups may include, but are not limited to, phenyl, naphthyl, anthracenyl, phenanthrenyl, pyrenyl, or the like.

Unless otherwise specified, the term "heteroaryl" or "heteroaryl ring group" refers to an aromatic monocyclic or polycyclic ring system containing 5-14 membered structures, preferably 5-12 membered structures, preferably 5-10 membered structures, preferably 5-9 membered structures, preferably 5-8 membered structures, and more preferably 5-6 membered structures, wherein 1, 2, 3, or more ring atoms are heteroatoms and the remaining atoms are carbon atoms; the heteroatoms are independently selected from O, N, or S, and the number of heteroatoms is preferably 1, 2, or 3. Polycyclic heteroaryl is fused heteroaryl. Examples of heteroaryl include, but are not limited to, furyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridyl, and the like.

Unless otherwise specified, the term "fused heteroaryl" refers to an aromatic ring system formed by two or more cyclic structures sharing two adjacent atoms with each other, wherein each ring in the fused heteroaryl is an unsaturated aromatic ring, which may contain 5-20 ring atoms, preferably 6-14 ring atoms, more preferably 7-10 ring atoms, for example, 7 ring atoms, 8 ring atoms, 9 ring atoms, or 10 ring atoms, and contains 1-4 ring heteroatoms, preferably 1-3, i.e., 1, 2, or 3, ring heteroatoms, wherein the heteroatoms are independently selected from N, O, and S. Fused heteroaryl includes bicyclic, tricyclic, tetracyclic, or polycyclic fused heteroaryl, preferably bicyclic, tricyclic, or tetracyclic fused heteroaryl, more preferably bicyclic or tricyclic fused heteroaryl. Exemplary examples of fused heteroaryl include, but are not limited to, 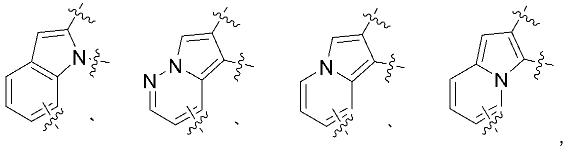 and the like.

Herein, 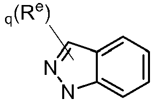 means that q Re groups may be substituted at any position of the bicyclic system, provided that a stable structure can be formed; for example, 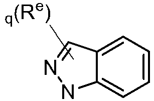 may be represented as 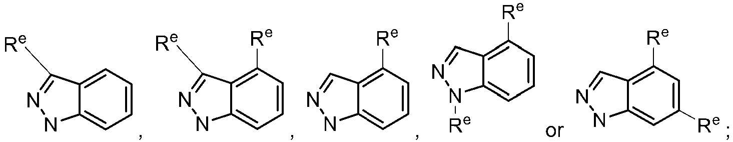 This rule applies to any ring system of the present invention and is used herein for illustrative purposes.

In various parts of the present invention, linking substituents (for example, L₄ₓ, L_{4y}, and the like) are involved. Those skilled in the art can understand that, when a linking group is clearly required in the compound structure, the Markush variables listed for that group should be understood as linking groups. For example, if the structure requires a linking group and the Markush group definition for that variable lists "alkyl" or "aryl", it should be understood that the "alkyl" or "aryl" represents a linking alkylene group or arylene group, respectively. Therefore, when used as a linking group, a "*yl" and a "*ylene" have equivalent definitions; for example, "alkyl" and "alkylene" have equivalent definitions.

Unless otherwise specified, the term "pharmaceutically acceptable salt" or "pharmaceutically usable salt" refers to a salt that, within the scope of reasonable medical judgment, is suitable for contact with the tissues of mammals, particularly humans, without undue toxicity, irritation, allergic response, and the like, and is commensurate with a reasonable benefit/risk ratio. For example, medically acceptable salts of amines, carboxylic acids, and other types of compounds are well known in the art. The salt may be prepared in situ during the final isolation and purification of the compound of the present invention, or may be separately prepared by reacting a free base or free acid with a suitable reagent.

Unless otherwise specified, the term "stereoisomer" refers to compounds having the same chemical constitution but differing in the spatial arrangement of atoms or groups. Stereoisomers include enantiomers, diastereomers, conformational isomers (rotamers), geometric (cis/trans) isomers, atropisomers, rotamers, and the like. Any resulting mixture of stereoisomers may be separated into pure or substantially pure geometric isomers, enantiomers, and diastereomers based on differences in the physicochemical properties of the components, for example, by chromatography and/or fractional crystallization. Unless otherwise specified, the term "geometric (cis/trans) isomer" may include a carbon-carbon double bond or carbon-nitrogen double bond in the E or Z configuration, wherein the term "E" denotes higher-priority substituents on opposite sides of the carbon-carbon or carbon-nitrogen double bond, and the term "Z" denotes higher-priority substituents on the same side of the carbon-carbon or carbon-nitrogen double bond, as determined using the Cahn-Ingold-Prelog priority rules. The compounds of the present invention may also exist as mixtures of "E" and "Z" isomers.

Unless otherwise specified, the term "tautomer" refers to structural isomers having different energies that are interconvertible through a low energy barrier. If tautomerism is possible (for example, in solution), a chemical equilibrium of the tautomers may be reached. For example, proton tautomers (also known as prototropic tautomers) include interconversion through migration of a proton, such as keto-enol isomerization and imine-enamine isomerization. Valence tautomers include interconversion through reorganization of some bonding electrons.

Unless otherwise indicated, the structural formulas described in the present invention include all isomeric forms, such as enantiomeric, diastereomeric, and geometric isomeric or conformational isomeric forms, including, for example, R and S configurations containing an asymmetric center, (Z) and (E) isomers of double bonds, and (Z) and (E) conformational isomers. Therefore, individual stereochemical isomers of the compounds of the present invention, or mixtures of enantiomers, diastereomers, or geometric isomers (or conformational isomers) thereof, are all within the scope of the present invention.

The compounds of the present invention also include isotopic derivatives thereof. Unless otherwise specified, the term "isotopic derivative" means that the compounds of the present invention may exist in isotopically labeled or enriched form and contain one or more atoms having an atomic weight or mass number different from that of the most abundant atom found in nature. The isotopes may be radioactive or non-radioactive isotopes. Isotopes commonly used as isotopic labels are: hydrogen isotopes, ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and¹⁵N; oxygen isotopes: ¹⁵O, ¹⁷O, and ¹⁸O; and sulfur isotope: ³⁵S. These isotopically labeled compounds may be used to study the distribution of medicinal molecules in tissues. In particular, ³H and ¹³C are more widely used because they are easy to label and convenient to detect. Substitution of certain heavy isotopes, such as deuterium (²H), can enhance metabolic stability and prolong half-life, thereby providing therapeutic advantages by reducing dosage. Isotopically labeled compounds are generally synthesized from labeled starting materials using known synthetic techniques, in the same manner as non-isotopically labeled compounds. The isotopic derivative is preferably a derivative in a deuterated form.

The compounds of the present invention also include solvates or solvated forms thereof. Unless otherwise specified, the terms "solvate" and "solvated form" refer to a physical association of a compound of the present invention with one or more solvent molecules, whether organic or inorganic. The physical association includes hydrogen bonding. In certain circumstances, for example, when one or more solvent molecules are incorporated into the crystal lattice of a crystalline solid, the solvate can be isolated. The solvent molecules in the solvate may be present in an ordered and/or disordered arrangement. The solvate may contain stoichiometric or non-stoichiometric amounts of solvent molecules. "Solvate" encompasses both solution-phase and isolable solvates. Exemplary solvates include, but are not limited to, hydrates, ethanolates, methanolates, and isopropanolates. Solvation methods are well known in the art.

The compounds of the present invention also include co-crystals thereof. Unless otherwise specified, the term "co-crystal" is used to describe a situation in which neutral molecular components are present in a crystalline compound in a defined stoichiometric ratio. The preparation of pharmaceutical co-crystals enables changes to be made to the crystalline form of an active pharmaceutical ingredient, which in turn can alter its physicochemical properties without compromising its desired biological activity (see Pharmaceutical Salts and Co-crystals, edited by J. Wouters and L. Quere, RSC Publishing, 2012).

The compounds of the present invention also include polymorphs thereof. Unless otherwise specified, the term "polymorph" refers to different arrangements of chemical drug molecules, generally manifested as the form in which a drug raw material exists in the solid state. A drug may exist in various crystalline states, and different crystalline forms of the same drug may differ in dissolution and absorption in vivo, thereby affecting dissolution and release of the formulation.

The compounds of the present invention also include metabolites thereof. Unless otherwise specified, the term "metabolite" refers to a product obtained by metabolism of a specific compound or a salt thereof in vivo. Metabolites of a compound may be identified by techniques well known in the art, and their activity may be characterized by test methods as described in the present invention. Such products may be obtained after the administered compound undergoes oxidation, reduction, hydrolysis, amidation, deamidation, esterification, deesterification, enzymatic cleavage, and the like. Accordingly, the present invention includes metabolites of the compounds, including metabolites produced by contacting a compound of the present invention with a mammal for a sufficient period of time.

The compounds of the present invention also include prodrugs thereof. Unless otherwise specified, the term "prodrug" refers to a drug that is converted in vivo into the parent drug. Prodrugs are often useful as they can improve certain defined undesirable physical or biological properties. Physical properties are usually related to solubility, whether excessive or insufficient lipid or aqueous solubility, or stability, whereas problematic biological properties include overly rapid metabolism or poor bioavailability, which themselves may be related to physicochemical properties. For example, they may be bioavailable by oral administration whereas the parent compound is not. Compared with the parent drug, a prodrug may also have improved solubility in a pharmaceutical composition One example of a prodrug, without limitation, may be any compound of the present invention administered as an ester ("prodrug") to facilitate passage across a cell membrane, where aqueous solubility is detrimental to transport but beneficial once inside the cell, after which the ester is metabolically hydrolyzed to the carboxylic acid, i.e., the active entity. Another example of a prodrug may be a short peptide (a polyamino acid) bound to an acid group, wherein the peptide is metabolized to reveal the active moiety.

The words "comprise" or "include", and English variants thereof, such as "comprises" or "comprising", should be understood in an open, non-exclusive sense, namely "including but not limited to".

The term "individual", also referred to as an "object" or "subject", refers to a cell or animal, wherein the animal includes, but is not limited to, a mammal, such as a laboratory animal or a human.

Some compounds of the present invention are optically active. The compounds of the present application may be racemates, optical isomers, or mixtures thereof. Optical isomers of the compounds of the present invention may be synthesized from optically isomeric starting materials or prepared by resolving racemates. The above embodiments represent exemplary embodiments of the present invention, but the present invention is not limited to the above embodiments. In addition, the various technical features in the above embodiments of the present invention may be combined with one another to form one or more new technical solutions, and such new technical solutions also fall within the scope of the present invention, provided that they are technically feasible.

Unless otherwise specified, the term "treating" encompasses any treatment of a disease, disorder, or condition in a patient, including: (a) inhibiting symptoms of the disease, disorder, or condition, i.e., arresting their development; or (b) alleviating symptoms of the disease, disorder, or condition, i.e., causing regression of the disease or symptoms; or (c) ameliorating or eliminating the disease, disorder, or condition, or one or more symptoms associated with the disease.

The abbreviations used in the Preparative Examples, Embodiments, and elsewhere herein are:
- DCM: dichloromethane
- DCE: 1,2-dichloroethane
- DIPEA: N,N-diisopropylethylamine
- DMF: N,N-dimethylformamide
- HATU: 2-(7-azobenzotriazole)-N,N,N',N'-tetramethyluronium hexafluorophosphate
- NMP: N-methylpyrrolidinone
- THF: tetrahydrofuran

The present application provides a class of compounds having novel structures, thereby providing a new direction for treating diabetes, non-alcoholic fatty liver disease, Alzheimer's disease, and other diseases, as well as for losing weight.

**The beneficial effects of the present invention are as follows:**
The compounds of the present invention achieve one or more of the following beneficial effects:
(1) Enzymatic tests show that the compounds of the present application have a strong agonistic action on GLP-1R;
(2) In vivo pharmacokinetic tests in mice and/or rats show that the compounds of the present application have good oral properties.
(3) In vivo pharmaceutical effects in mice show that the compounds of the present application have excellent weight-loss effects.
(4) In vivo pharmaceutical effects in mice show that the compounds of the present application have excellent blood glucose-lowering efficacy.
(5) Safety assessment tests show that the compounds of the present application have no off-target risk or a very low off-target risk, and have good safety.

### Brief Description of the Drawings

FIG. 1: spectrum of nuclear magnetic hydrogen spectroscopy (temperature: 60°C) of Embodiment 10 (Compound 10).
FIG. 2: spectrum of nuclear magnetic hydrogen spectroscopy (temperature: 80°C) of Embodiment 8 (Compound 8).
FIG. 3: spectrum of nuclear magnetic hydrogen spectroscopy (temperature: 80°C) of Embodiment 15 (Compound 65).
FIG. 4: chart of body weight gain rates of Test Example 4.

### Detailed Description of Embodiments

The present invention is further described below with reference to specific embodiments. It should be understood that these examples are merely used to illustrate the present invention and are not intended to limit the scope of the present invention. For experimental methods in the following examples for which specific conditions are not indicated, conventional conditions are generally followed, or conditions recommended by the manufacturer are followed. Unless otherwise defined, all technical and scientific terms used herein have the same meanings as commonly understood by those skilled in the art. In addition, any methods and materials similar or equivalent to content described herein may be applied to the methods of the present invention. The preferred implementation methods and materials shown herein are for illustrative purposes only.

The following are preparation examples of exemplary compounds of the present application.

The structures of the compounds of the present invention were determined by nuclear magnetic resonance (NMR), liquid chromatography-mass spectrometry (LC-MS), and/or high-performance liquid chromatography (HPLC), and/or high-resolution mass spectrometry (HRMS). The instruments used for NMR assays were Bruker Avance neo 400 MHz (temperature: 60°C) and/or Bruker AVANCEIII 600; the instruments used for LC-MS were Agilent, 1260 Infinity II-6120/6125MSD and/or Waters ACQUITY QDa; the instrument used for HPLC was Waters e2695-2998 (chromatographic column: Waters XBridge C18 4.6*250 mm,5 µm; column temperature: 40°C; flow rate: 1 mL/min; wavelength: 216 nm; mobile phase A: water (containing 0.1% trifluoroacetic acid); mobile phase B: methanol); the instrument used for HRMS was Agilent Accurate-Mass Q-TOF 6530.

The starting materials in the examples of the present invention are known and commercially available, or may be synthesized using or following methods known in the art.

### Preparation Example 1: Synthesis of tert-butyl (S)-2-(4-fluoro-3,5-dimethylphenyl)-4-methyl-3-(methylamino)-2,4,6,7-tetrahydro-5H-pyrazolo[4,3-c]pyridine-5-carboxylate (Intermediate 1.4-A)

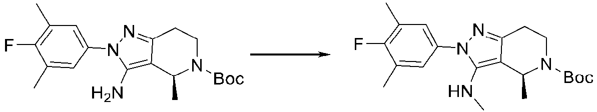

tert-Butyl (S)-3-amino-2-(4-fluoro-3,5-dimethylphenyl)-4-methyl-2,4,6,7-tetrahydro-5H-pyrazolo[4,3-c]pyridine-5-carboxylate (150 mg) was dissolved in THF (5 mL), followed by the addition of methyl iodide (62 mg) and sodium hydride (32 mg), and the mixture was stirred at room temperature for two hours. Water (40 mL) was added, and the mixture was extracted with ethyl acetate (30 mL*3). The organic phases were combined, dried over anhydrous sodium sulfate, then filtered and concentrated, and subjected to column chromatography isolation (n-hexane: ethyl acetate = 1:1, volume ratio) to obtain Intermediate 1.4-A (115 mg), ESI-MS (m/z): 389.2 [M+H]⁺.

### Preparation Example 2: Synthesis of tert-butyl (S)-3-(3-(4-fluoro-1-methyl-1H-indazol-5-yl)-2-oxoimidazolin-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-A)

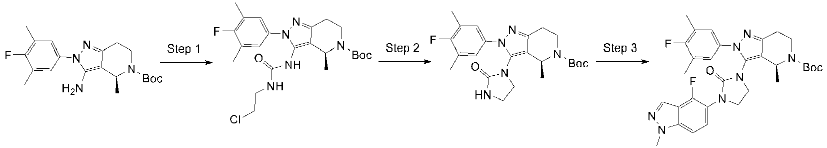

### Step 1: Synthesis of tert-butyl (S)-3-(3-(2-chloroethyl)ureido)-2-(4-fluoro-3,5-dimethylphenyl)-4-methyl-2,4,6,7-tetrahydro-5H-pyrazolo[4,3-c]pyridine-5-carboxylate

tert-Butyl (S)-3-amino-2-(4-fluoro-3,5-dimethylphenyl)-4-methyl-2,4,6,7-tetrahydro-5*H*-pyrazolo[4,3-c]pyridine-5-carboxylate (2 g), chloroethyl isocyanate (734 mg), and tetrahydrofuran (40 mL) were added into a flask, triethylamine (1.6 g) was added, and the mixture was heated to 60°C and stirred for 8 hours. After completion of the reaction, the mixture was cooled, added into water (40 mL), and extracted with ethyl acetate (30 mL*3). The organic phases were combined, dried over anhydrous sodium sulfate, and then filtered and concentrated. The crude product was purified by column chromatography isolation (n-hexane:ethyl acetate = 3:1) to obtain the product (700 mg), ESI-MS (m/z): 480.2 [M+H]⁺;

### Step 2: Synthesis of tert-butyl (S)-2-(4-fluoro-3,5-dimethylphenyl)-4-methyl-3-(2-oxoimidazolin-1-yl)-2,4,6,7-tetrahydro-5H-pyrazolo[4,3-c]pyridine-5-carboxylate

The product from Step 1 (700 mg) and N,N-dimethylformamide (10 mL) were added into a flask, NaH (88 mg) was added, and the mixture was stirred at room temperature for 4 hours. After completion of the reaction, water (40 mL) was added into the mixture, and the mixture was extracted with ethyl acetate (30 mL*3). The organic phases were combined, dried over anhydrous sodium sulfate, and then filtered and concentrated. The crude product was purified by column chromatography isolation (n-hexane:ethyl acetate = 1:1) to obtain the product (300 mg), ESI-MS (m/z): 444.2 [M+H]⁺;

### Step 3: Synthesis of Intermediate 1.5-A

The product from Step 2 (60 mg), 5-bromo-4-fluoro-1-methyl-1*H*-indazole (62 mg), copper(I) iodide (24 mg), (1S,2S)-(+)-N,N-dimethylcyclohexanediamine (18 mg), potassium phosphate (61 mg), and dioxane (5 mL) were added into a flask, and the mixture was heated to 120°C and stirred for 4 hours under a nitrogen atmosphere. After completion of the reaction, the mixture was cooled, water (40 mL) was added, and the mixture was extracted with ethyl acetate (30 mL*3). The organic phases were combined, dried over anhydrous sodium sulfate, and then filtered and concentrated. The crude product was separated and purified by thin-plate chromatographic separation (n-hexane:ethyl acetate = 1:2) to obtain Intermediate 1.5-A (50 mg), ESI-MS (m/z): 592.3 [M+H]⁺.

### Preparation Example 3: Synthesis of tert-butyl (S)-3-(3-(3,4-dihydro-2H-[1,3]oxazino[3,2-b]indazol-9-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-B)

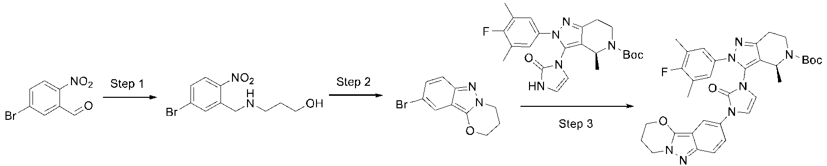

### Step 1: Synthesis of 3-((5-bromo-2-nitrobenzyl)amino)propan-1-ol

5-bromo-2-nitrobenzaldehyde (100 mg) and 3-amino-1-propanol (144 mg) were dissolved in ethanol (10 mL), tetraisopropyl titanate (1 g) was then added, and the mixture was stirred at room temperature for 1 hour. Sodium borohydride (50 mg) was then added, and the mixture was stirred at room temperature for two hours. Aqueous ammonia (6 mL) was added to quench the reaction, a reaction liquid was filtered, and the filtrate was dried and concentrated. The resulting crude product was purified by column chromatography (ethyl acetate) to obtain the product (78 mg), ESI-MS (m/z): 290.1 [M+H]⁺;

### Step 2: Synthesis of 9-bromo-3,4-dihydro-2H-[1,3]oxazino[3,2-b]indazole

The product from Step 1 (78 mg) was dissolved in a mixed solvent of tert-butanol and water (15 mL/5 mL), potassium hydroxide (61 mg) was added, and the reaction was carried out at 85°C for 5 hours. The mixture was diluted with water (40 mL) and extracted with ethyl acetate (30 mL*3). The organic phase was dried over anhydrous sodium sulfate, filtered, and concentrated. The crude product was purified by column chromatography (ethyl acetate) to obtain the product (54 mg), ESI-MS (m/z): 253.2 [M+H]⁺;

### Step 3: Synthesis of Intermediate 1.5-B

The product from Step 2 (54 mg), tert-butyl (S)-2-(4-fluoro-3,5-dimethylphenyl)-4-methyl-3-(2-oxo-2,3-dihydro-1H-imidazol-1-yl)-2,4,6,7-tetrahydro-5H-pyrazolo[4,3-c]pyridine-5-carboxylate (47 mg), copper(I) iodide (8 mg), (1S,2S)-(+)-N,N'-dimethylcyclohexane-1,2-diamine (12 mg), and potassium carbonate (87 mg) were dissolved in N-methylpyrrolidone (2 mL), and the mixture was stirred at 130°C for 4 hours under nitrogen protection. After completion of the reaction, the reaction liquid was directly concentrated, and the crude product was purified by column chromatography (petroleum ether:ethyl acetate = 5:1) to obtain Intermediate 1.5-B (27 mg), ESI-MS (m/z): 614.4 [M+H]⁺.

### Preparation Example 4: Synthesis of tert-butyl (S)-3-(3-(3,4-dihydro-2H-[1,3]oxazino[3,2-b]indazol-8-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-C)

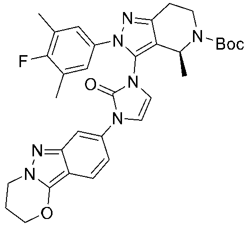

With reference to Intermediate 1.5-B, 4-bromo-2-nitrobenzaldehyde (100 mg) was used as the raw material to obtain Intermediate 1.5-C (24 mg), ESI-MS (m/z): 614.4 [M+H]⁺.

### Preparation Example 5: Synthesis of tert-butyl (S)-3-(3-(2,3-dihydro-[1,4]oxazino[2,3,4-hi]indazol-8-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-D)

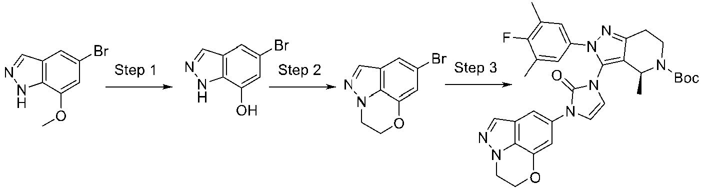

### Step 1: Synthesis of 5-bromo-7-hydroxy-1H-indazole

5-bromo-7-methoxy-1H-indazole (200 mg) was dissolved in dichloromethane (10 mL), boron tribromide (440 mg) was added dropwise at 0°C, and the mixture was stirred at room temperature for 1 hour. Water (40 mL) was added to quench the reaction, the mixture was extracted with dichloromethane (30 mL*3), dried over anhydrous sodium sulfate, and filtered, and the filtrate was dried and concentrated. The resulting crude product was purified by column chromatography (n-hexane:ethyl acetate = 5:1) to obtain 5-bromo-7-hydroxy-1H-indazole (150 mg), ESI-MS (m/z): 213.1 [M+H]⁺.

### Step 2: Synthesis of 8-bromo-2,3-dihydro-[1,4]oxazino[2,3,4-hi]indazole

5-bromo-7-hydroxy-1H-indazole (150 mg) was dissolved in N,N-dimethylformamide (20 mL), 1-bromo-2-chloroethane (102 mg) was added, and the mixture was reacted at room temperature for 4 hours, then heated to 70°C, and continuously reacted for 2 hours. Water (100 mL) was added for dilution, the mixture was extracted with ethyl acetate (30 mL*3), and the organic phase was dried over anhydrous sodium sulfate, filtered, and concentrated. The crude product was purified by column chromatography (n-hexane:ethyl acetate = 10:1) to obtain 8-bromo-2,3-dihydro-[1,4]oxazino[2,3,4]indazole (100 mg), ESI-MS (m/z): 239.2 [M+H]⁺.

### Step 3: Synthesis of Intermediate 1.5-D

With reference to Intermediate 1.5-B, 8-bromo-2,3-dihydro-[1,4]oxazino[2,3,4-***hi***]indazole (100 mg) was used as the raw material to obtain Intermediate 1.5-D (30 mg), ESI-MS (m/z): 600.4 [M+H]⁺.

### Preparation Example 6: Synthesis of tert-butyl (S)-3-(1-(4-fluoro-1-methyl-1H-indazol-5-yl)-5-oxo-1,5-dihydro-4H-1,2,4-triazol-4-yl)-2-(4-fluoro-3,5-dimethylphenyl)-4-methyl-2,4,6,7-tetrahydro-5H-pyrazolo[4,3-c]pyridine-5-carboxylate (Intermediate 1.5-E)

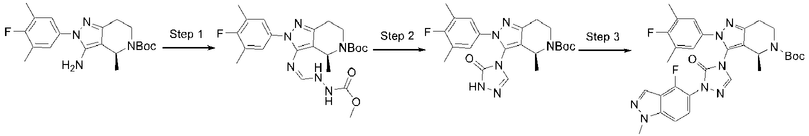

### Step 1: Synthesis of tert-butyl (S)-2-(4-fluoro-3,5-dimethylphenyl)-3-(((2-(methoxycarbonyl)hydrazino)methylene)amino)-4-methyl-2,4,6,7-tetrahydro-5H-pyrazolo[4,3-c]pyridine-5-carboxylate

tert-Butyl (S)-3-amino-2-(4-fluoro-3,5-dimethylphenyl)-4-methyl-2,4,6,7-tetrahydro-5H-pyrazolo[4,3-c]pyridine-5-carboxylate (5 g) and triethyl orthoformate (2.57 g) were added into a flask. The mixture was reacted at 90°C for 1 hour. After completion of the reaction, the mixture was cooled, the solvent was removed under reduced pressure, methyl carbazate (2.4 g) and p-toluenesulfonic acid (634 mg) were added, and the mixture was stirred at 50°C for 2 hours. After completion of the reaction, the mixture was cooled, water (100 mL) was added, the mixture was extracted with ethyl acetate (50 mL*3), the solvent was removed under reduced pressure, and column chromatography (n-hexane:ethyl acetate = 1:1) was performed to obtain the product (5.4 g), ESI-MS (m/z): 475.2 [M+H]⁺.

### Step 2: Synthesis of tert-butyl (S)-2-(4-fluoro-3,5-dimethylphenyl)-4-methyl-3-(5-oxo-1,5-dihydro-4H-1,2,4-triazol-4-yl)-2,4,6,7-tetrahydro-5H-pyrazolo[4,3-c]pyridine-5-carboxylate

The product from Step 1 (5.4 g) and methanol (100 mL) were added into a flask, a methanolic solution of sodium methoxide (5 M, 4.5 mL) was added dropwise, and the mixture was stirred at room temperature for 12 hours. After completion of the reaction, most of the methanol was removed under reduced pressure, water (100 mL) was added, and the mixture was filtered and dried to obtain the product (crude product, 4.2 g). ESI-MS (m/z): 443.2 [M+H]⁺;

### Step 3: Synthesis of Intermediate 1.5-E

With reference to Intermediate 1.5-A, the product from Step 2 (100 mg) was used as the raw material to obtain Intermediate 1.5-E (42 mg), ESI-MS (m/z): 591.3 [M+H]⁺.

### Preparation Example 7: Synthesis of tert-butyl (S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-F)

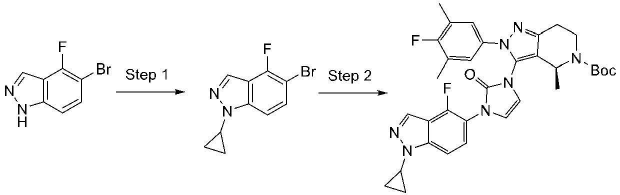

### Step 1: Synthesis of 5-bromo-1-cyclopropyl-4-fluoro-1H-indazole

5-bromo-4-fluoro-1H-indazole (1 g), cyclopropylboronic acid (0.8 g), anhydrous copper acetate (0.85 g), bipyridine (0.73 g), sodium carbonate (1 g), and 1,2-dichloroethane (20 mL) were added into a flask, and the mixture was heated to 70°C and stirred for 2 hours. The mixture was filtered, the filtrate was diluted with dichloromethane, washed with water (40 mL), concentrated under reduced pressure, and purified by column chromatography (n-hexane:ethyl acetate = 5:1) to obtain the product (0.8 g), ESI-MS (m/z): 254.9 [M+H]⁺;

### Step 2: Synthesis of Intermediate 1.5-F

With reference to Intermediate 1.5-A, the product from Step 1 (220 mg) and tert-butyl (S)-2-(4-fluoro-3,5-dimethylphenyl)-4-methyl-3-(2-oxo-2,3-dihydro-1H-imidazol-1-yl)-2,4,6,7-tetrahydro-5H-pyrazolo[4,3-c]pyridine-5-carboxylate (200 mg) were used as raw materials to obtain Intermediate 1.5-F (250 mg), ESI-MS (m/z): 616.3 [M+H]⁺;

### Preparation Example 8: Synthesis of tert-butyl (S)-2-(3-cyclopropyl-4-fluorophenyl)-3-(3-(4-fluoro-1-methyl-1H-indazol-5-yl)-2-oxo-2,3-dihydro-1H-imidazol-1-yl)-4-methyl-2,4,6,7-tetrahydro-5H-pyrazolo[4,3-c]pyridine-5-carboxylate (Intermediate 1.5-G)

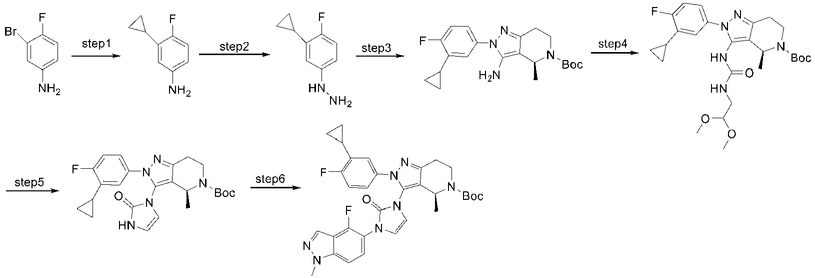

### Step 1: Synthesis of 3-cyclopropyl-4-fluoroaniline

3-bromo-4-fluoroaniline (10 g), cyclopropylboronic acid (5.9 g), potassium phosphate (39.1 g), palladium acetate (1.2 g), triphenylphosphine (1.4 g), toluene (200 mL), and water (20 mL) were added into a flask, and the mixture was heated to 100°C and stirred for 15 hours under a nitrogen atmosphere. After completion of the reaction, water (200 mL) was added, the mixture was extracted with ethyl acetate (150 mL*3), concentrated under reduced pressure, and purified by column chromatography (n-hexane:ethyl acetate = 10:1) to obtain the product (6.9 g), ESI-MS (m/z): 152.1 [M+H]⁺.

### Step 2: Synthesis of (3-cyclopropyl-4-fluorophenyl)hydrazine

The product from Step 1 (900 mg) and concentrated hydrochloric acid (30 mL) were added into a flask, an aqueous solution of sodium nitrite (0.12 M, 60 mL) was added dropwise at 0°C, and the mixture was stirred at 0°C for 1 hour. A solution of stannous chloride in concentrated hydrochloric acid (0.33 M, 50 mL) was then added dropwise at 0°C, and the mixture was stirred at 0°C for 1 hour. After completion of the reaction, water (150 mL) was added, a saturated aqueous sodium hydroxide solution was added to adjust the pH to 8, ethyl acetate (200 mL) was added, insoluble matter was removed by filtration, the layers were separated, and the ethyl acetate layer was concentrated under reduced pressure to obtain a crude product, which was finally purified by column chromatography (n-hexane:ethyl acetate = 1:1) to obtain the product (210 mg), ESI-MS (m/z): 167.1 [M+H]⁺.

### Step 3: Synthesis of tert-butyl (S)-3-amino-2-(3-cyclopropyl-4-fluorophenyl)-4-methyl-2,4,6,7-tetrahydro-5H-pyrazolo[4,3-c]pyridine-5-carboxylate

The product from Step 2 (190 mg), tert-butyl (2S)-3-cyano-2-methyl-4-oxopiperidine-1-carboxylate (227 mg), ethanol (5 mL), and aqueous hydrochloric acid solution (2 M, 100 µL) were added into a flask, and the mixture was stirred at 50°C. After completion of the reaction, the mixture was concentrated under reduced pressure and purified by column chromatography (n-hexane:ethyl acetate = 3:2) to obtain the product (258 mg), ESI-MS (m/z): 387.2 [M+H]⁺.

### Step 4: Synthesis of tert-butyl (S)-2-(3-cyclopropyl-4-fluorophenyl)-3-(3-(2,2-dimethoxyethyl)ureido)-4-methyl-2,4,6,7-tetrahydro-5H-pyrazolo[4,3-c]pyridine-5-carboxylate

The product from Step 3 (87 mg) and dichloromethane (2 mL) were added into a flask. Triphosgene (22 mg) and N,N-diisopropylethylamine (58 mg) were dissolved in dichloromethane (2 mL) to be then added dropwise to the flask, and the mixture was stirred at room temperature for 1 hour. 2,2-Dimethoxyethylamine (94 mg) was added dropwise, and the mixture was stirred at room temperature. After completion of the reaction, water (20 mL) was added, the mixture was extracted with dichloromethane (20 mL*3), concentrated under reduced pressure, and purified by column chromatography (n-hexane:ethyl acetate = 1:1) to obtain the product (85 mg), ESI-MS (m/z): 518.3 [M+H]⁺.

### Step 5: Synthesis of tert-butyl (S)-2-(3-cyclopropyl-4-fluorophenyl)-4-methyl-3-(2-oxo-2,3-dihydro-1H-imidazol-1-yl)-2,4,6,7-tetrahydro-5H-pyrazolo[4,3-c]pyridine-5-carboxylate

The product from Step 4 (80 mg), trifluoromethanesulfonic acid (35 mL), and tetrahydrofuran (2 mL) were added into a flask, and the mixture was stirred at 60°C for 1 hour. The reaction liquid was cooled to room temperature, triethylamine (47 mg) and di-tert-butyl dicarbonate (51 mg) were added, and the mixture was stirred at room temperature. After completion of the reaction, the mixture was concentrated under reduced pressure and purified by column chromatography (n-hexane:ethyl acetate = 1:1) to obtain the product (65 mg), ESI-MS (m/z): 454.2 [M+H]⁺.

### Step 6: Synthesis of Intermediate 1.5-G

With reference to the final reaction step of Intermediate 1.5-A, 5-bromo-1-methyl-4-fluoro-1H-indazole (39 mg) and the product from Step 5 (50 mg) were used as raw materials to obtain Intermediate 1.5-G (40 mg), ESI-MS (m/z): 602.3 [M+H]⁺.

### Preparation Example 9: Synthesis of tert-butyl (S)-3-(3-(1-cyclopropylmethyl-4-fluoro-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-H)

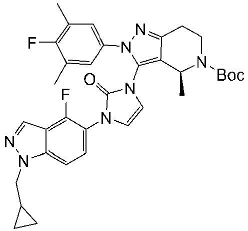

With reference to the synthesis of Intermediate 1.5-F, bromomethylcyclopropane (200 mg) was used as the raw material to obtain Intermediate 1.5-H (90 mg), ESI-MS (m/z): 630.3 [M+H]⁺.

### Preparation Example 10: Synthesis of tert-butyl (S)-3-(3-(1-cyclopropyl-4-methyl-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-I)

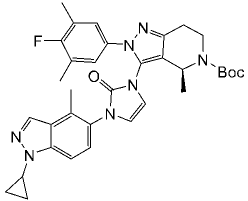

With reference to the synthesis of Intermediate 1.5-F, 5-bromo-4-methyl-1H-indazole (140 mg) was used as the raw material to obtain Intermediate 1.5-I (48 mg), ESI-MS (m/z): 612.2 [M+H]⁺.

### Preparation Example 11: Synthesis of tert-butyl (S)-3-(3-(1-cyclopropyl-7-methyl-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-J)

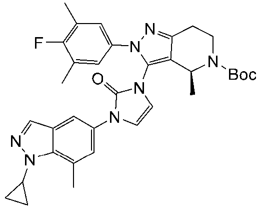

With reference to the synthesis of Intermediate 1.5-F, 5-bromo-7-methyl-1H-indazole (150 mg) was used as the raw material to obtain Intermediate 1.5-J (280 mg), ESI-MS (m/z): 612.2 [M+H]⁺.

### Preparation Example 12: Synthesis of tert-butyl (S)-3-(3-(2-cyclopropylmethyl-4-fluoro-2H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-K)

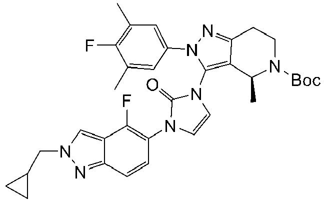

With reference to the synthesis of Intermediate 1.5-F, (3.15 g) was used as the raw material to obtain Intermediate 1.5-K (4.20 g), ESI-MS (m/z): 630.3 [M+H]⁺.

### Preparation Example 13: Synthesis of 4-fluoro-1-methyl-1H-indazol-5-amine (Intermediate 2.1-A)

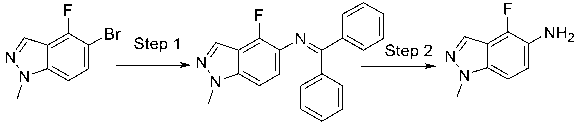

### Step 1: Synthesis of N-(4-fluoro-1-methyl-1H-indazol-5-yl)-1,1-diphenylmethanimine

5-bromo-4-fluoro-1-methyl-1H-indazole (150 mg), benzophenone imine (142 mg), bis(dibenzylideneacetone)palladium (60 mg), 4,5-bis(diphenylphosphino)-9,9-dimethylxanthene (152 mg), and cesium carbonate (429 mg) were added into a flask, toluene (20 mL) was added, and the mixture was heated to 130°C and stirred for 4 hours. After completion of the reaction, the mixture was cooled and added into water (40 mL) and extracted with ethyl acetate (30 mL*3), and the organic phases were combined, dried over anhydrous sodium sulfate, filtered, and concentrated. The product (120 mg) was obtained by column chromatography (n-hexane:ethyl acetate = 5:1) isolation, ESI-MS (m/z): 330.1 [M+H]⁺;

### Step 2: Synthesis of Intermediate 2.1-A

The product from Step 1 (120 mg) was added into a flask, tetrahydrofuran and water (10 mL each) were added, citric acid (200 mg) was added in an ice bath, and the mixture was stirred at room temperature overnight. After completion of the reaction, the pH was adjusted to 9-10 with an aqueous sodium bicarbonate solution, the mixture was extracted with ethyl acetate (30 mL*3), the organic phases were combined, dried over anhydrous sodium sulfate, filtered, and concentrated, and column chromatography (n-hexane:ethyl acetate = 5:1) isolation was performed to obtain Intermediate 2.1-A (42 mg), ESI-MS (m/z): 166.1 [M+H]⁺.

### Preparation Example 14: Synthesis of N,1-dimethyl-1H-indazol-5-amine (Intermediate 2.2-A)

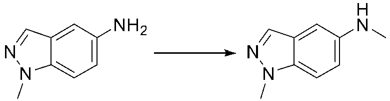

1-methyl-1H-indazol-5-amine (150 mg) was dissolved in THF (5 mL), followed by the addition of methyl iodide (160 mg) and sodium hydride (81 mg), and the mixture was stirred at room temperature for two hours. After completion of the reaction, water (40 mL) was added, and the mixture was extracted with ethyl acetate (30 mL*3). The organic phases were combined, dried over anhydrous sodium sulfate, filtered, concentrated, and subjected to column chromatography (n-hexane:ethyl acetate = 5:1) isolation to obtain Intermediate 2.2-A (103 mg), ESI-MS (m/z): 162.1 [M+H]⁺.

### Embodiment 1: Synthesis of 1-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-3-(1-methyl-1H-indazol-5-yl)urea (Compound 1)

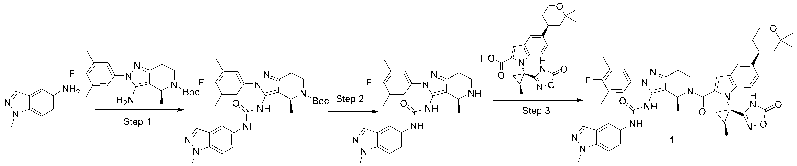

### Step 1: Synthesis of tert-butyl (S)-2-(4-fluoro-3,5-dimethylphenyl)-4-methyl-3-(3-(1-methyl-1H-indazol-5-yl)ureido)-2,4,6,7-tetrahydro-5H-pyrazolo[4,3-c]pyridine-5-carboxylate

1-methyl-1H-indazol-5-amine (50 mg) was dissolved in dichloromethane (5 mL), triphosgene (30 mg) and triethylamine (68 mg) were added, and the mixture was stirred at room temperature for two hours. tert-Butyl (S)-3-amino-2-(4-fluoro-3,5-dimethylphenyl)-4-methyl-2,4,6,7-tetrahydro-5H-pyrazolo[4,3-c]pyridine-5-carboxylate (127 mg, 0.34 mmol) was then added, and the reaction was stirred at room temperature overnight. After completion of the reaction, water (40 mL) was added, and the mixture was extracted with dichloromethane (30 mL*3). The organic phases were combined, dried over anhydrous sodium sulfate, filtered, concentrated, and subjected to thin-layer chromatography (dichloromethane:methanol = 30:1, v/v) isolation to obtain the product (153 mg), ESI-MS (m/z): 548.3 [M+H]⁺;

### Step 2: Synthesis of (S)-1-(2-(4-fluoro-3,5-dimethylphenyl)-4-methyl-4,5,6,7-tetrahydro-2H-pyrazolo[4,3-c]pyridin-3-yl)-3-(1-methyl-1H-indazol-5-yl)urea

The product from Step 1 (153 mg) was dissolved in a solution of hydrochloric acid in dioxane (4 M, 10 mL), and the mixture was stirred at room temperature for two hours. After completion of the reaction, the mixture was directly concentrated to obtain the product (crude product, 105 mg), ESI-MS (m/z): 448.2 [M+H]⁺;

### Step 3: Synthesis of 1-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-3-(1-methyl-1H-indazol-5-yl)urea

The product from Step 2 (50 mg) and 5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carboxylic acid (46 mg) were dissolved in anhydrous DMF (2 mL), HATU (85 mg) and DIPEA (29 mg) were then added, and the reaction was stirred at room temperature overnight. After completion of the reaction, water (40 mL) was added, and the mixture was extracted with ethyl acetate (30 mL*3). The organic phases were combined, dried over anhydrous sodium sulfate, filtered, concentrated, and subjected to thin-layer chromatography (dichloromethane:methanol = 20:1) isolation to obtain Compound 1 (65 mg), ESI-MS (m/z): 841.4 [M+H]⁺; HRMS (m/z): [M+H]⁺ Calcd. for C₄₆H₄₉N₁₀O₅F: 841.3944. Found: 841.3950. HPLC retention time: 18.820 min. The structure was confirmed by nuclear magnetic hydrogen spectroscopy (¹H NMR (600 MHz, DMSO-d₆)) to be correct.

### Embodiment 2: Synthesis of 3-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-1-methyl-1-(1-methyl-1H-indazol-5-yl)urea (Compound 2)

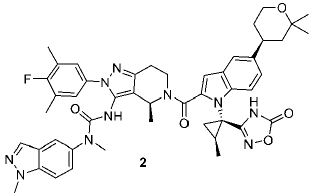

With reference to the method of Embodiment 1, N,1-dimethyl-1H-indazol-5-amine (Intermediate 2.2-A) (50 mg) was used as the raw material to obtain Compound 2 (60 mg), ESI-MS (m/z): 855.4 [M+H]⁺; HRMS (m/z): [M+H]⁺ Calcd. for C₄₇H₅₁N₁₀O₅F: 855.4101. Found: 855.4098. HPLC retention time: 19.002 min.

### Embodiment 3: Synthesis of 1-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-1-methyl-3-(1-methyl-1H-indazol-5-yl)urea (Compound 3)

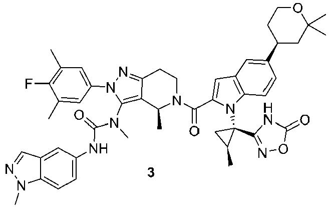

With reference to the method of Embodiment 1, tert-butyl (S)-2-(4-fluoro-3,5-dimethylphenyl)-4-methyl-3-(methylamino)-2,4,6,7-tetrahydro-5H-pyrazolo[4,3-c]pyridine-5-carboxylate (Intermediate 1.4-A) (50 mg) was used as the raw material to obtain Compound 3 (63 mg), ESI-MS (m/z): 855.4 [M+H]⁺; HRMS (m/z): [M+H]⁺ Calcd. for C₄₇H₅₁N₁₀O₅F: 855.4101. Found: 855.4100. HPLC retention time: 19.101 min.

### Embodiment 4: Synthesis of 1-((S)-5-(5-((S)-2,2-dimethyltetrahydro-2H-pyran-4-yl)-1-((1S,2S)-2-methyl-1-(5-oxo-4,5-dihydro-1,2,4-oxadiazol-3-yl)cyclopropyl)-1H-indole-2-carbonyl)-2-(4-fluoro-3,5-dimethylphenyl)-4-methyl-4,5,6,7-tetrahydro-2H-pyrazolo[4,3-c]pyridin-3-yl)-3-(4-fluoro-1-methyl-1H-indazol-5-yl)urea (Compound 4)

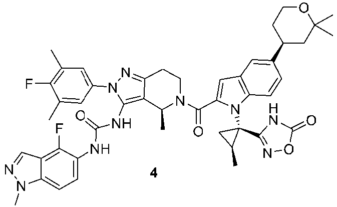

With reference to the method of Embodiment 1, 4-fluoro-1-methyl-1H-indazol-5-amine (Intermediate 2.1-A) (50 mg) was used as the raw material to obtain Compound 4 (53 mg), ESI-MS (m/z): 859.4 [M+H]⁺; HRMS (m/z): [M+H]⁺ Calcd. for C₄₆H₄₈N₁₀O₅F₂: 859.3850. Found: 859.3843. HPLC retention time: 18.912 min.

### Embodiment 5: Synthesis of 3-((1S,2S)-1-(5-((S)-2,2-dimethyltetrahydro-2H-pyran-4-yl)-2-((S)-3-(3-(4-fluoro-1-methyl-1H-indazol-5-yl)-2-oxoimidazolin-1-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one (Compound 5)

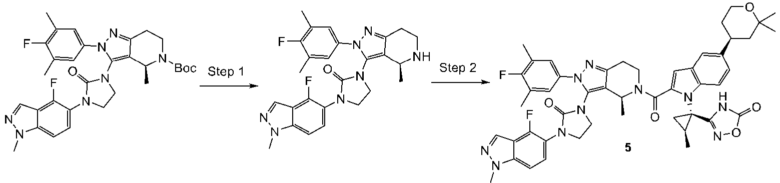

### Step 1: Synthesis of (S)-1-(4-fluoro-1-methyl-1H-indazol-5-yl)-3-(2-(4-fluoro-3,5-dimethylphenyl)-4-methyl-4,5,6,7-tetrahydro-2H-pyrazolo[4,3-c]pyridin-3-yl)imidazolin-2-one

tert-Butyl (S)-3-(3-(4-fluoro-1-methyl-1*H*-indazol-5-yl)-2-oxoimidazolin-1-yl)-2-(4-fluoro-3,5-dimethylphenyl)-4-methyl-2,4,6,7-tetrahydro-5*H*-pyrazolo[4,3-*c*]pyridine-5-carboxylate (Intermediate 1.5-A) (66 mg) and dichloromethane (10 mL) were added into a flask, trifluoroacetic acid (3 mL) was added, and the mixture was stirred at room temperature. After completion of the reaction, the solvent was concentrated, and the crude product was purified by column chromatography (dichloromethane:methanol = 10:1) isolation to obtain the product (50 mg), ESI-MS (m/z): 492.2 [M+H]⁺;

### Step 2: Synthesis of Compound 5

5-((S)-2,2-Dimethyltetrahydro-2*H*-pyran-4-yl)-1-((1S,2S)-2-methyl-1-(5-oxo-4,5-dihydro-1,2,4-oxadiazol-3-yl)cyclopropyl)-1*H*-indole-2-carboxylic acid (93 mg) and N,N-dimethylformamide (5 mL) were added into a flask, HATU (125 mg), triethylamine (56 mg), and the product from Step 1 (54 mg) were added, and the mixture was stirred at room temperature for 6 hours. After completion of the reaction, water (40 mL) was added into the mixture, and the mixture was extracted with ethyl acetate (30 mL*3). The organic phases were combined, dried over anhydrous sodium sulfate, filtered, and concentrated, and the crude product was purified by column chromatography (dichloromethane:methanol = 20:1) isolation to obtain Compound 5 (22 mg), ESI-MS (m/z): 885.4 [M+H]⁺; HRMS (m/z): [M+H]⁺ Calcd. for C₄₈H₅₀N₁₀O₅F₂: 885.4006. Found: 885.4001; ¹H NMR (600 MHz, DMSO-d₆) δ 11.80 (s, 1H), 8.25 (s, 1H), 7.58 (d, *J* = 8.8 Hz, 1H), 7.54 (s, 1H), 7.50-7.44 (m, 1H), 7.40 (d, *J* = 8.4 Hz, 1H), 7.31 (d, *J =* 5.7 Hz, 2H), 7.27 (d, *J* = 8.0 Hz, 1H), 6.94 (s, 1H), 5.68-5.63 (m, 1H), 4.39-4.33 (m, 1H), 4.09 (s, 3H), 4.06-4.02 (m, 1H), 3.96-3.86 (m, 2H), 3.66-3.60 (m, 2H), 3.18-3.10 (m, 1H), 3.08-3.00 (m, 2H), 2.90-2.80 (m, 2H), 2.32 (s, 6H), 1.75-1.62 (m, 4H), 1.55-1.52 (m, 3H), 1.29-1.22 (m, 5H), 1.21-1.16 (m, 7H). HPLC retention time: 20.381 min.

### Embodiment 6: Synthesis of 3-((1S,2S)-1-(2-((S)-3-(3-(3,4-dihydro-2H-[1,3]oxazino[3,2-b]indazol-9-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 6)

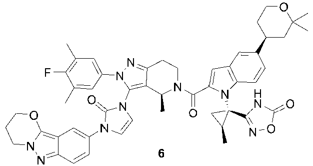

With reference to the method of Embodiment 5, (S)-3-(3-(3,4-dihydro-2*H*-[1,3]oxazino[3,2-b]indazol-9-yl)-2-oxo-2,3-dihydro-1*H*-imidazol-1-yl)-2-(4-fluoro-3,5-dimethylphenyl)-4-methyl-2,4,6,7-tetrahydro-5H-pyrazolo[4,3-*c*]pyridine-5-carboxylate tert-butyl ester (Intermediate 1.5-B) (27 mg) was used as the raw material to obtain Compound 6 (43 mg), ESI-MS (m/z): 907.4 [M+H]⁺; HRMS (m/z): [M+H]⁺ Calcd. for C₅₀H₅₁N₁₀O₆F: 907.4050. Found: 907.4058. HPLC retention time: 22.250 min.

### Embodiment 7: Synthesis of 3-((1S,2S)-1-(2-((S)-3-(3-(3,4-dihydro-2H-[1,3]oxazino[3,2-b]indazol-8-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 7)

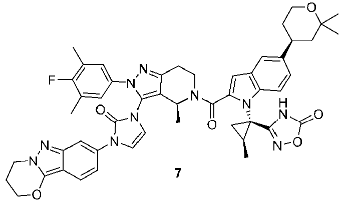

With reference to the method of Embodiment 5, tert-butyl (S)-3-(3-(3,4-dihydro-2H-[1,3]oxazino[3,2-b]indazol-8-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-C) (24 mg) was used as the raw material to obtain Compound 7 (10 mg), ESI-MS (m/z): 907.4 [M+H]⁺; HRMS (m/z): [M+H]⁺ Calcd. for C₅₀H₅₁N₁₀O₆F: 907.4050. Found: 907.4044. HPLC retention time: 22.262 min.

### Embodiment 8: Synthesis of 3-((1S,2S)-1-(2-((S)-3-(3-(2,3-dihydro-[1,4]oxazino[2,3,4-hi]indazol-8-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 8)

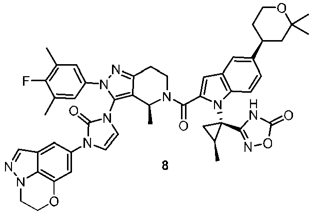

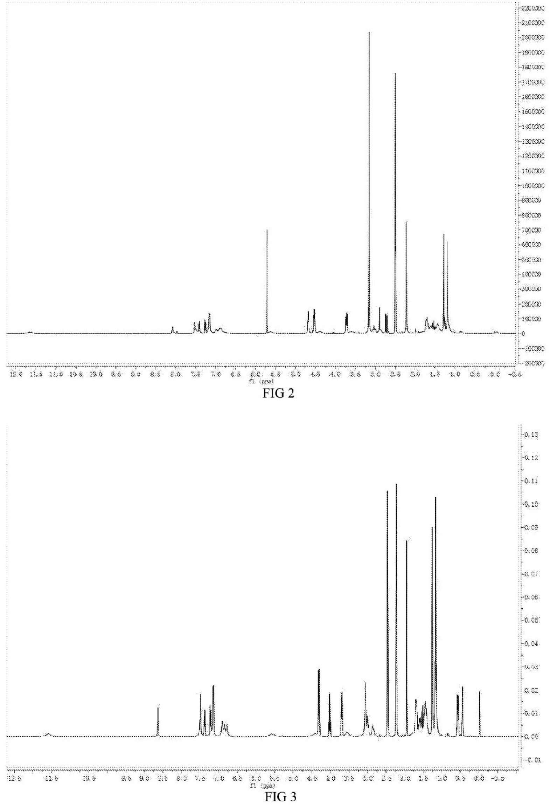

With reference to the method of Embodiment 5, tert-butyl (S)-3-(3-(2,3-dihydro-[1,4]oxazino[2,3,4-***hi***]indazol-8-yl)-2-oxo-2,3-dihydro-1*H*-imidazol-1-yl)-2-(4-fluoro-3,5-dimethylphenyl)-4-methyl-2,4,6,7-tetrahydro-5*H*-pyrazolo[4,3-c]pyridine-5-carboxylate (Intermediate 1.5-D) (30 mg) was used as the raw material to obtain Compound 8 (42 mg), ESI-MS (m/z): 893.4 [M+H]⁺; HRMS (m/z): [M+H]⁺ Calcd. for C₄₉H₄₉N₁₀O₆F: 893.3893. Found: 893.3884. HPLC retention time: 20.210 min. ¹H NMR (400 MHz, DMSO-d6, 80°C): see FIG. 2.

### Embodiment 9: Synthesis of 3-((1S,2S)-1-(5-((S)-2,2-dimethyltetrahydro-2H-pyran-4-yl))-2-((S)-3-(1-(4-fluoro-1-methyl-1H-indazol-5-yl)-5-oxo-1,5-dihydro-4H-1,2,4-triazol-4-yl)-2-(4-fluoro-3,5-dimethylphenyl)-4-methyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one (Compound 9)

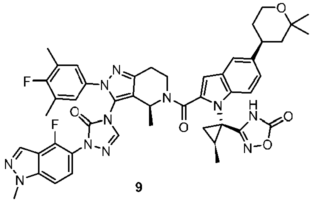

With reference to the method of Embodiment 5, tert-butyl (S)-3-(1-(4-fluoro-1-methyl-1H-indazol-5-yl)-5-oxo-1,5-dihydro-4*H*-1,2,4-triazol-4-yl)-2-(4-fluoro-3,5-dimethylphenyl)-4-methyl-2,4,6,7-tetrahydro-5*H-*pyrazolo[4,3-c]pyridine-5-carboxylate (Intermediate 1.5-E) (42 mg) was used as the raw material to obtain Compound 9 (13 mg), ESI-MS (m/z): 884.4 [M+H]⁺; HRMS (m/z): [M+H]⁺ Calcd. for C₄₇H₄₇N₁₁O₅F₂: 884.3802. Found: 884.3812. HPLC retention time: 20.090 min.

### Embodiment 10: Synthesis of 3-((1S,2S)-1-(2-((S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 10)

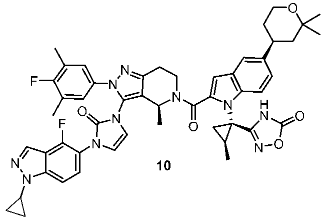

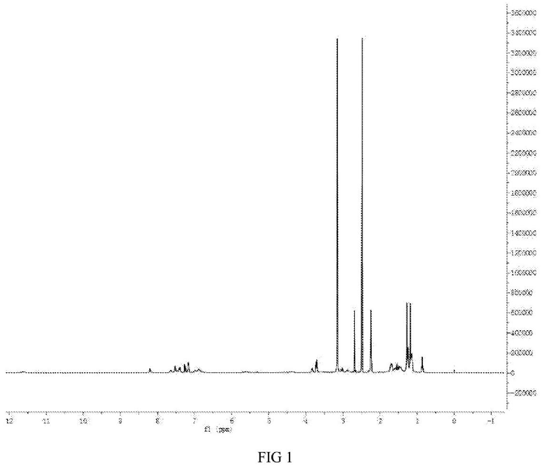

With reference to the method of Embodiment 5, tert-butyl (S)-3-(3-(1-cyclopropyl-4-fluoro-1*H*-indazol-5-yl)-2-oxo-2,3-dihydro-1*H*-imidazol-1-yl)-2-(4-fluoro-3,5-dimethylphenyl)-4-methyl-2,4,6,7-tetrahydro-5*H-*pyrazolo[4,3-*c*]pyridine-5-carboxylate (Intermediate 1.5-F) (250 mg) was used as the raw material to obtain Compound 10 (196 mg), ESI-MS (m/z): 909.4 [M+H]⁺; HRMS (m/z): [M+H]⁺ Calcd. for C₅₀H₅₀N₁₀O₅F₂: 909.4006. Found: 909.4008. The ¹H NMR (600 MHz, DMSO-d₆, 60°C) results are shown in FIG. 1. HPLC retention time: 21.060 min.

### Embodiment 11: Synthesis of 3-((1S,2S)-1-(5-((4S)-2,2-dimethyloxan-4-yl)-2-((4S)-2-(4-fluoro-3-cyclopropylphenyl)-3-(3-(4-fluoro-1-cyclopropylindazol-5-yl)-2-oxoimidazol-1-yl)-4-methyl-6,7-dihydro-4H-pyrazolo[4,3-c]pyridine-5-carbonyl)indol-1-yl)-2-methylcyclopropyl)-4H-1,2,4-oxadiazol-5-one (Compound 61)

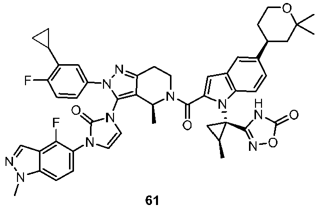

With reference to the method of Embodiment 5, tert-butyl (S)-2-(3-cyclopropyl-4-fluorophenyl)-3-(3-(4-fluoro-1-methyl-1*H*-indazol-5-yl)-2-oxo-2,3-dihydro-1*H*-imidazol-1-yl)-4-methyl-2,4,6,7-tetrahydro-5*H-*pyrazolo[4,3-c]pyridine-5-carboxylate (Intermediate 1.5-G) (40 mg) was used as the raw material to obtain Compound 61 (41 mg), ESI-MS (m/z): 895.3 [M+H]⁺; HRMS (m/z): [M+H]⁺ Calcd. for C₄₉H₄₈N₁₀O₅F₂: 895.3850. Found: 895.3846. HPLC retention time: 19.834 min.

### Embodiment 12: Synthesis of 3-((1S,2S)-1-(2-((S)-3-(3-(1-cyclopropylmethyl-4-fluoro-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 62)

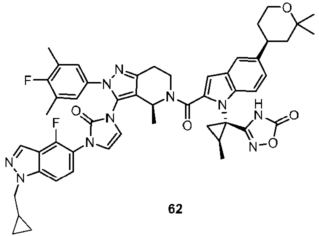

With reference to the method of Embodiment 5, tert-butyl (S)-3-(3-(1-cyclopropylmethyl-4-fluoro-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-H) (50 mg) was used as the raw material to obtain Compound 62 (40 mg), ESI-MS (m/z): 923.4 [M+H]⁺; HRMS (m/z): [M+H]⁺ Calcd. for C₅₁H₅₂N₁₀O₅F₂: 923.4163. Found: 923.4170. HPLC retention time: 21.515 min.

### Embodiment 13: Synthesis of 3-((1S,2S)-1-(2-((S)-3-(3-(1-cyclopropyl-4-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 63)

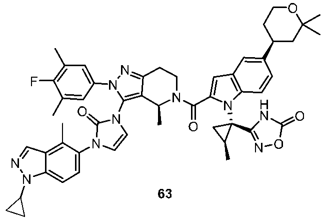

With reference to Embodiment 5, tert-butyl (S)-3-(3-(1-cyclopropyl-4-methyl-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-I) (83 mg) was used as the raw material to obtain Compound 63 (82 mg), ESI-MS (m/z): 905.4 [M+H]⁺; HRMS (m/z): [M+H]⁺ Calcd. for C₅₁H₅₃N₁₀O₅F₂: 905.4257. Found: 905.4266. HPLC retention time: 20.586 min.

### Embodiment 14: Synthesis of 3-((1S,2S)-1-(2-((S)-3-(3-(1-cyclopropyl-7-methyl-1H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 64)

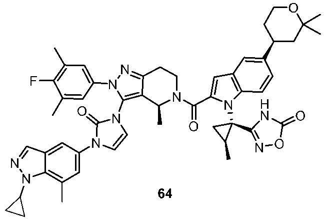

With reference to Embodiment 5, tert-butyl (S)-3-(3-(1-cyclopropyl-7-methyl-1H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-J) (140 mg) was used as the raw material to obtain Compound 64 (130 mg), ESI-MS (m/z): 905.3 [M+H]⁺; HRMS (m/z): [M+H]⁺ Calcd. for C₅₁H₅₃N₁₀O₅F: 905.4257. Found: 905.4251. HPLC retention time: 21.285 min.

### Embodiment 15: Synthesis of 3-((1S,2S)-1-(2-((S)-3-(3-(2-cyclopropylmethyl-4-fluoro-2H-indazol-5-yl)-2-oxo-2,3-dihydro-1H-imidazol-1-yl)-2-(4-fluoro-3,5-dimethylphenyl)-4-methyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 65)

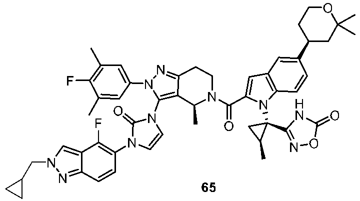

With reference to the method of Embodiment 5, tert-butyl (S)-3-(3-(2-cyclopropylmethyl-4-fluoro-2H-indazol-5-yl)-2-oxo-2,3-dihydro-1H-imidazol-1-yl)-2-(4-fluoro-3,5-dimethylphenyl)-4-methyl-2,4,6,7-tetrahydro-5H-pyrazolo[4,3-c]pyridine-5-carboxylate (Intermediate 1.5-K) (3.71 g) was used as the raw material to obtain Compound 65 (5.22 g), ESI-MS (m/z): 923.3 [M+H]⁺; HRMS (m/z): [M+H]⁺ Calcd. for C₅₁H₅₂N₁₀O₅F₂: 923.4163. Found: 923.4169. HPLC retention time: 20.656 min. ¹H NMR (400 MHz, DMSO-d6, 80°C): see FIG. 3.

With reference to the preparation methods of the above relevant examples and/or in combination with preparation methods described in the literature, the following compounds were prepared, and their structures and characterization data are as follows:

Specifically, with reference to Embodiment 1 and/or in combination with preparation methods described in the literature, the following compounds were prepared, and their structures and characterization data are as follows:

| Number | Structural formula | ESI-MS(m/z) |
|---|---|---|
| **11** | 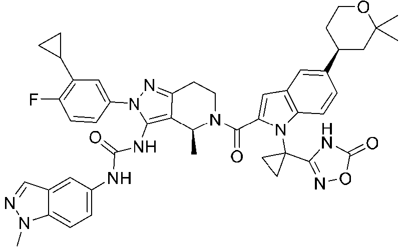 | 839.4[M+H]⁺ |
| **12** | 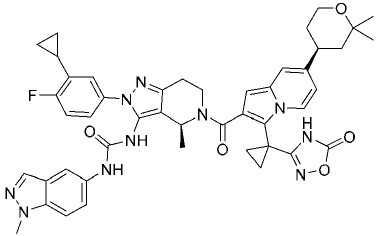 | 839.4[M+H]⁺ |
| **13** | 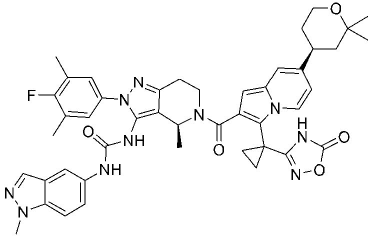 | 827.4[M+H]⁺ |
| 14 | 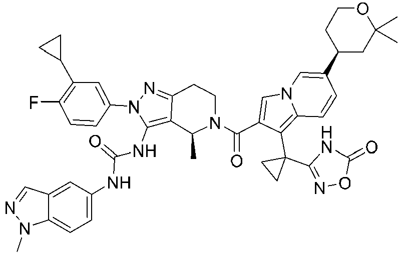 | 839.4[M+H]⁺ |
| 15 | 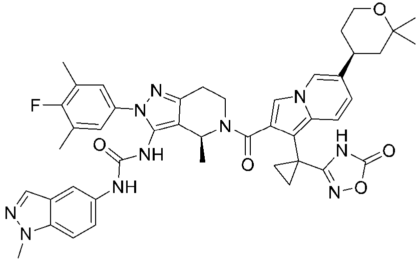 | 827.4[M+H]⁺ |
| 16 | 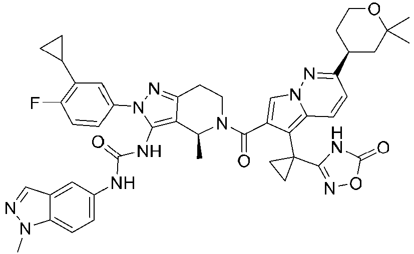 | 840.4[M+H]⁺ |
| 17 | 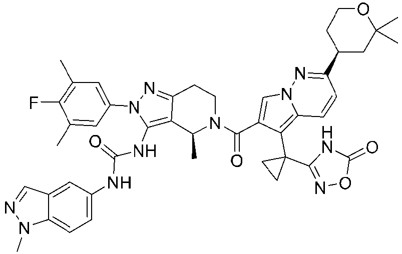 | 828.4[M+H]⁺ |
| 18 | 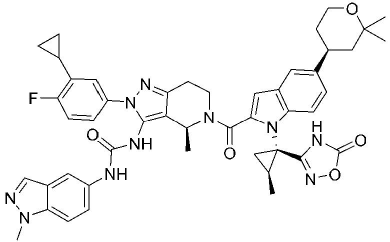 | 853.4[M+H]⁺ |
| **19** | 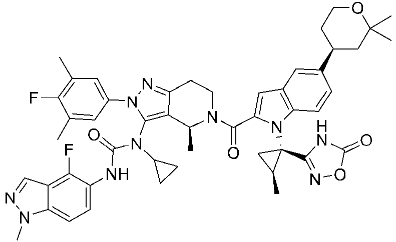 | 899.4[M+H]⁺ |
| **20** | 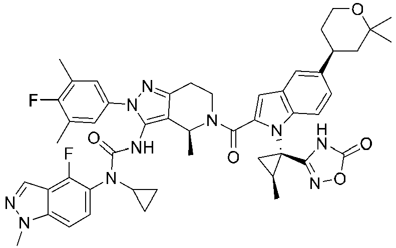 | 899.4[M+H]⁺ |
| **21** | 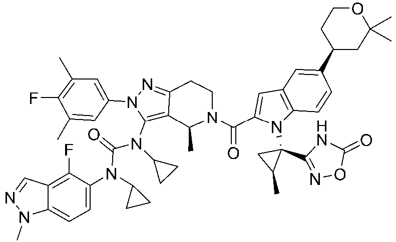 | 939.4[M+H]⁺ |
| **22** | 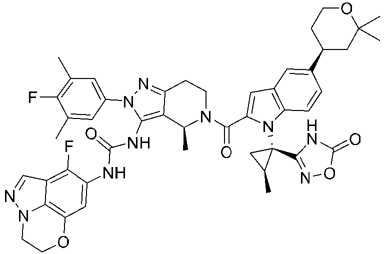 | 887.4[M+H]⁺ |

Specifically, with reference to Embodiment 5 and/or in combination with preparation methods described in the literature, the following compounds were prepared, and their structures and characterization data are as follows:

| Number | Structural formula | ESI-MS(m/z) |
|---|---|---|
| **23** | 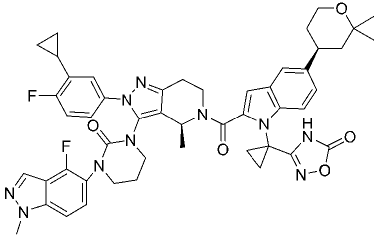 | 897.4[M+H]⁺ |
| **24** | 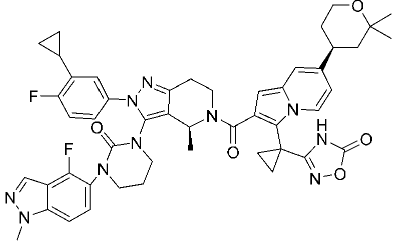 | 897.4[M+H]⁺ |
| **25** | 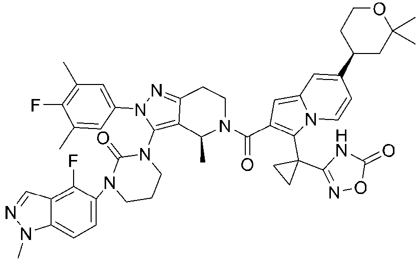 | 885.4[M+H]⁺ |
| **26** | 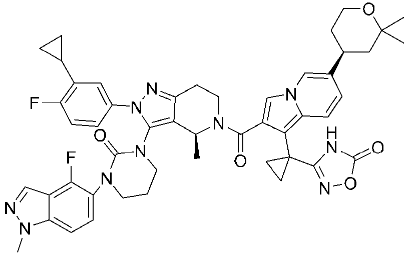 | 897.4[M+H]⁺ |
| **27** | 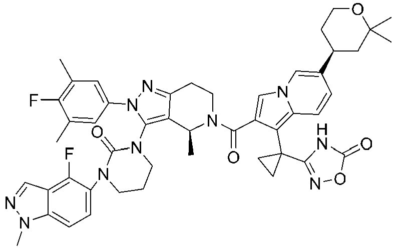 | 885.4[M+H]⁺ |
| **28** | 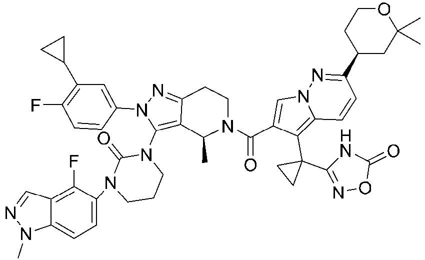 | 898.4[M+H]⁺ |
| **29** | 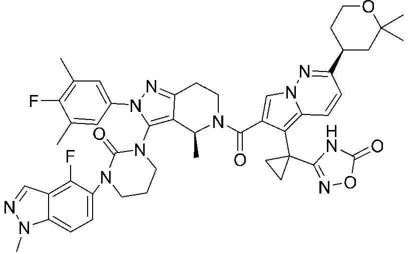 | 886.4[M+H]⁺ |
| **30** | 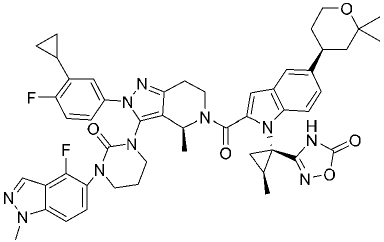 | 911.4[M+H]⁺ |

Specifically, with reference to Embodiment 9 and/or in combination with preparation methods described in the literature, the following compounds were prepared, and their structures and characterization data are as follows:

| Number | Structural formula | ESI-MS(m/z) |
|---|---|---|
| **31** | 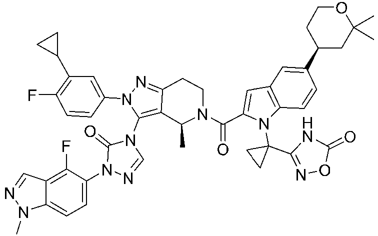 | 882.4[M+H]⁺ |
| **32** | 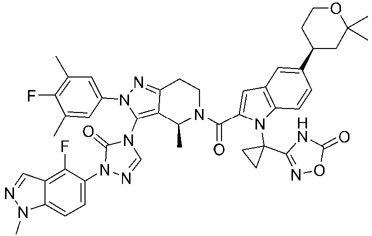 | 870.4[M+H]⁺ |
| **33** | 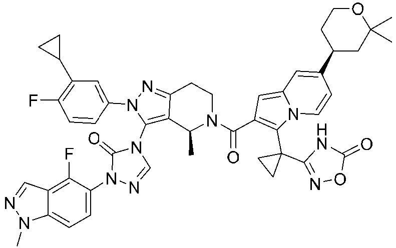 | 882.4[M+H]⁺ |
| **34** | 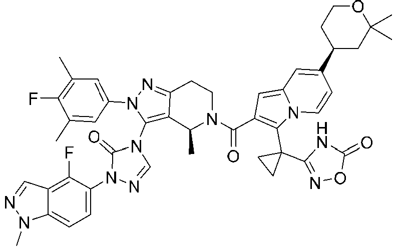 | 870.4[M+H]⁺ |
| **35** | 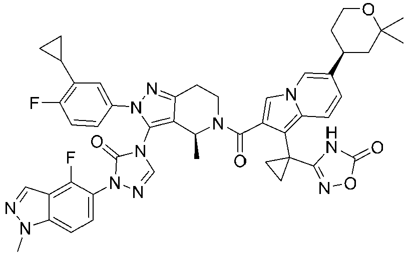 | 882.4[M+H]⁺ |
| **36** | 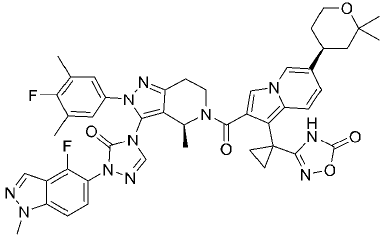 | 870.4[M+H]⁺ |
| **37** | 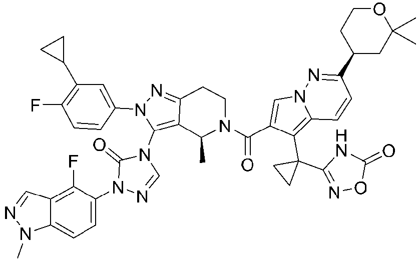 | 883.4[M+H]⁺ |
| **38** | 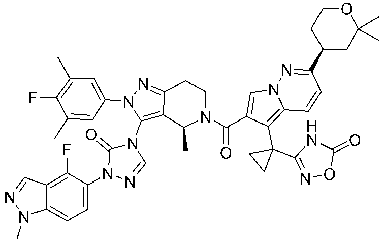 | 871.4[M+H]⁺ |
| **39** | 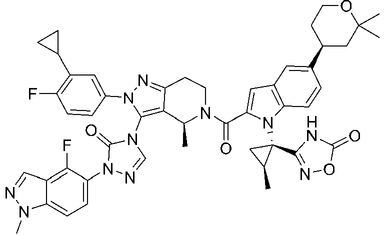 | 896.4[M+H]⁺ |

Specifically, with reference to Embodiment 8 and/or in combination with preparation methods described in the literature, the following compounds were prepared, and their structures and characterization data are as follows:

| Number | Structural formula | ESI-MS(m/z) |
|---|---|---|
| **40** | 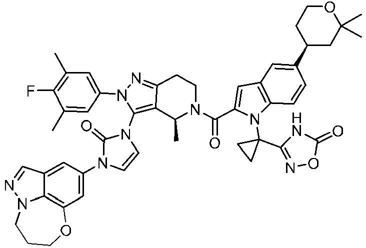 | 893.4[M+H]⁺ |
| **41** | 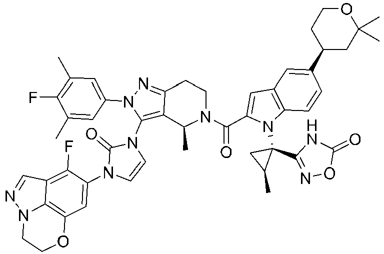 | 911.4[M+H]⁺ |

Specifically, with reference to Embodiment 10 and/or in combination with preparation methods described in the literature, the following compounds were prepared, and their structures and characterization data are as follows:

| Number | Structural formula | ESI-MS(m/z) |
|---|---|---|
| **42** | 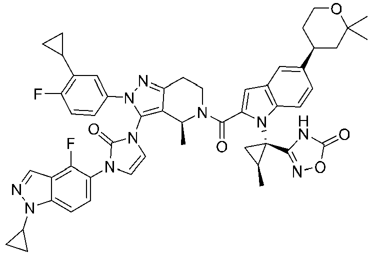 | 921.4[M+H]⁺ |
| **43** | 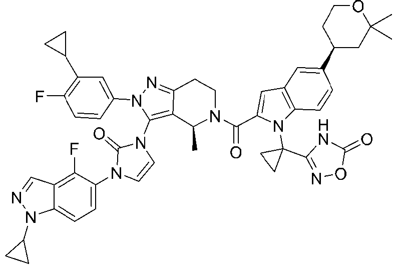 | 907.4[M+H]⁺ |
| **44** | 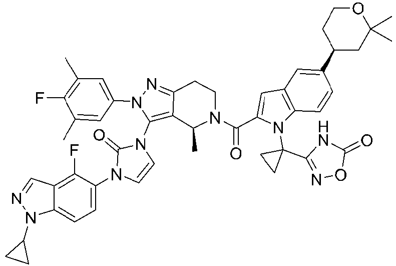 | 895.4[M+H]⁺ |
| **45** | 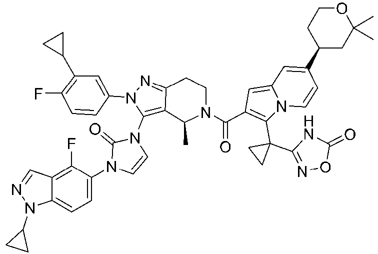 | 907.4[M+H]⁺ |
| **46** | 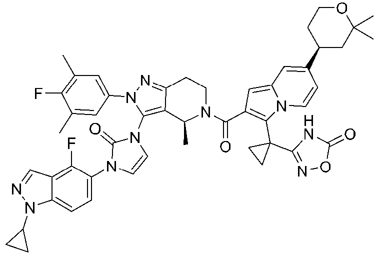 | 895.4[M+H]⁺ |
| **47** | 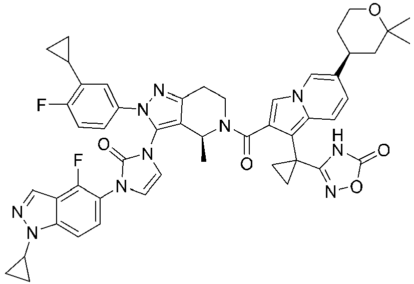 | 907.4[M+H]⁺ |
| **48** | 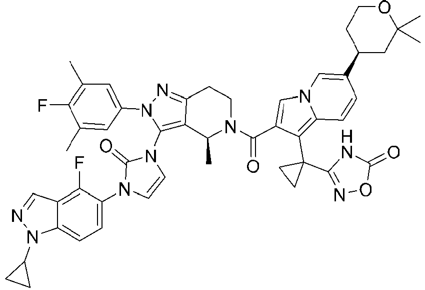 | 895.4[M+H]⁺ |
| **49** | 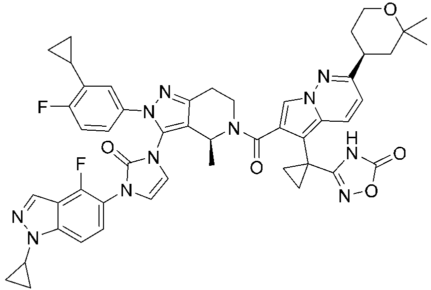 | 908.4[M+H]⁺ |
| **50** | 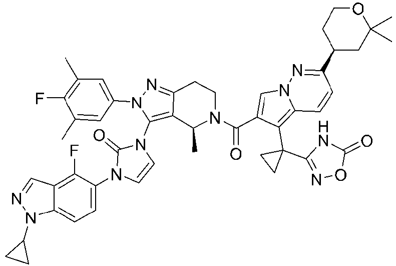 | 896.4[M+H]⁺ |
| **51** | 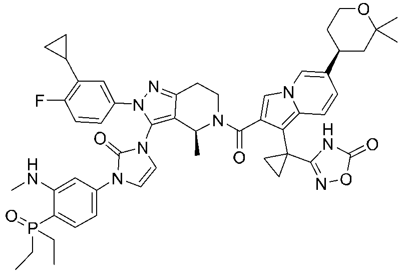 | 942.4[M+H]⁺ |
| **52** | 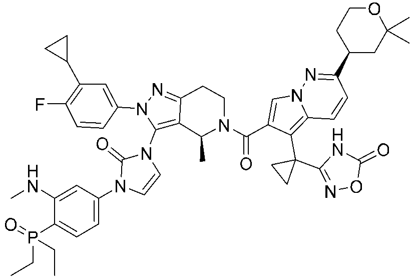 | 943.4[M+H]⁺ |
| **53** | 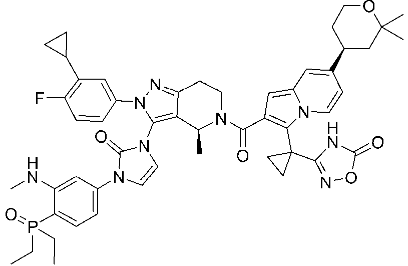 | 942.4[M+H]⁺ |
| **54** | 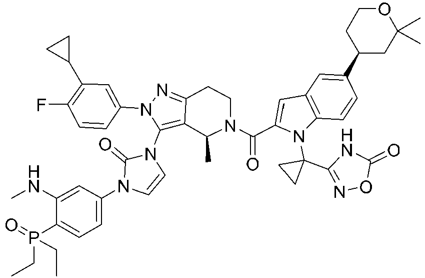 | 942.4[M+H]⁺ |
| **55** | 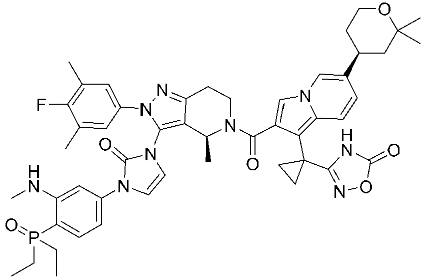 | 930.4[M+H]⁺ |
| **56** | 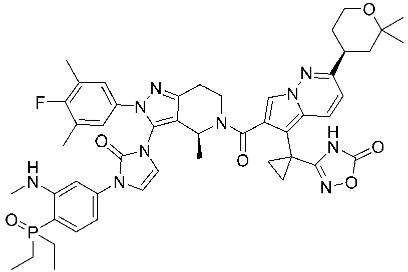 | 931.4[M+H]⁺ |
| **57** | 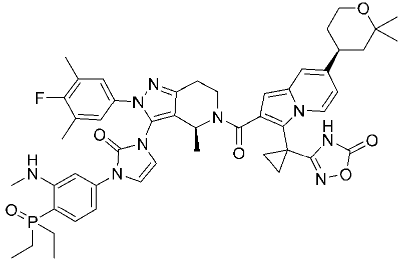 | 930.4[M+H]⁺ |
| **58** | 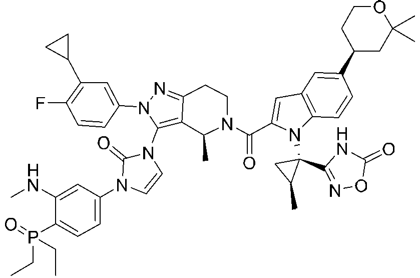 | 956.4[M+H]⁺ |
| **59** | 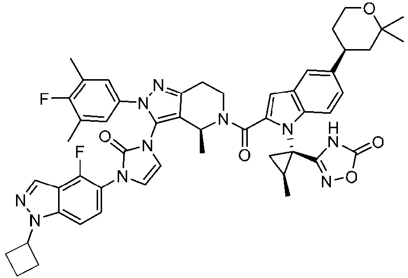 | 923.4[M+H]⁺ |
| **60** | 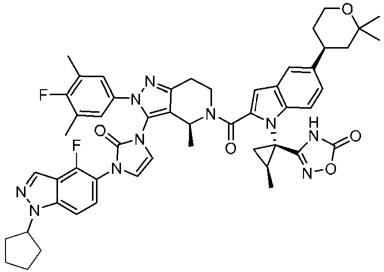 | 937.4[M+H]⁺ |

### Test Example 1: In Vitro Enzymatic Assay

GLP1R-cAmp-Luc cells were used to determine the agonistic activity of the test compounds on GLP-1R. 1) The test compounds were dissolved in DMSO and mixed thoroughly until the test compounds were completely dissolved. The test compounds were dissolved in DMSO and prepared into solutions at a mother liquor concentration of 10 mM. All test compounds were diluted with DMEM medium (high-glucose, Puromycin⁺, Hygromycin B⁺) to a starting concentration of 1250 nM, and then diluted at a 5-fold ratio to give a total of 7 concentration gradients. GLP1R-cAmp-Luc cells were seeded in a white-walled 96-well cell culture plate at a density of 1 × 10⁵/mL, with 90 µL of cell suspension per well; The test compounds were added at 10 µL per well for all 7 concentration gradients. The actual starting working concentration of the test compounds was 125 nM, and the cells were placed in a 37°C, 5% CO₂ cell culture incubator for 4 h; 2) A potassium luciferin solution diluted with PBS to 300 µg/mL was added at 100 µL per well, giving a working concentration of 150 µg/mL, followed by incubation at room temperature for 30 min; 3) The firefly luciferin signal was measured using a multifunctional microplate reader (PerkinElmer, Nivo); The fluorescence values were processed using GraphPad Prism software, and EC₅₀ values were calculated.

The compounds of the present invention have good agonistic activity on GLP-1R. Preferred compounds have an agonistic activity (EC₅₀) of less than or equal to 20 nM; more preferred compounds have an agonistic activity (EC₅₀) of less than or equal to 10 nM; more preferred compounds have an agonistic activity (EC₅₀) of less than or equal to 5 nM; more preferred compounds have an agonistic activity (EC₅₀)) of less than or equal to 2 nM; and more preferred compounds have an agonistic activity (EC₅₀) of less than or equal to 1 nM. The activity results of exemplary compounds are shown in the table below.

| **Compound No.** | **Agonistic activity** (EC₅₀: nM) | **Compound No.** | **Agonistic activity** (EC₅₀: nM) |
|---|---|---|---|
| 1 | A | 6 | A |
| 2 | A | 7 | A |
| 3 | A | 8 | A |
| 4 | A | 9 | A |
| 5 | A | 10 | A |
| 61 | A | 62 | A |
| 63 | A | 64 | A |
| 65 | A | LY35029770 | A |

| | | | |
|---|---|---|---|
| Note: A represents EC₅₀ ≤ 10 nM. | | | |

### Test Example 2: In Vivo Pharmacokinetic Study in Mice

Test animals: ICR mice (female, 22-25 g) were purchased from Beijing Vital River Laboratory Animal Technology Co., Ltd.

Test steps: ICR mice were administered the test compound by tail vein injection or intragastric administration. Whole blood samples of the mice were collected at 5 min after administration (intravenous only), 15 min, 0.5 h, 1 h, 2 h, 4 h, 8 h, 24 h, 36 h, 48 h, and 72 h. Plasma was separated by centrifugation, compound concentrations were determined by LC-MS/MS, and pharmacokinetic parameters including plasma clearance (Cl), elimination half-life (T_{1/2}), peak time (Tmax), peak concentration (Cmax), and area under the concentration-time curve (AUC) were calculated using Winnolin software.

Intravenous dose: 2 mg/kg; Intragastric administration dose: 10 mg/kg.

Determination method: after pretreatment by protein precipitation, quantitative analysis was performed using an LC-MS/MS system.

The compounds of the present invention have excellent oral administration properties. The PK parameters of exemplary compounds are respectively listed in the table below. Compared with LY 3502970, the compounds have better pharmacokinetic properties, in particular better oral absorption properties.

| Compound | iv (2 mg/kg) | po (10 mg/kg) | | |
|---|---|---|---|---|
| | AUC_{inf} (h*ng/mL) | Cₘₐₓ (ng/mL) | Tₘₐₓ (h) | AUC_{inf} (h*ng/mL) |
| 8 | 7706 | 5377 | 1.33 | 41197 |

| | | | | |
|---|---|---|---|---|
| Note: Detection results at 24 h. | | | | |

| Compound | iv (2 mg/kg) | | | po (10 mg/kg) | | | | |
|---|---|---|---|---|---|---|---|---|
| | Cl (mL/h /kg) | AUC_{inf} (h*ng /mL) | T_{1/2} (h) | Cₘₐₓ (ng/ mL) | Tₘₐₓ (h) | T_{1/2} (h) | AUC_{inf} (h*ng/ mL) | F (%) |
| 10 | 92.7 | 21599 | 10 | 8197 | 1.67 | 9.56 | 103132 | 95.5 |

| | | | | | | | | |
|---|---|---|---|---|---|---|---|---|
| Note: Detection results at 72 h. | | | | | | | | |

| Compound | iv (2 mg/kg) | | | po (10 mg/kg) | | | | |
|---|---|---|---|---|---|---|---|---|
| | Cl (mL/h/ kg) | AUC_{inf} (h*ng /mL) | T_{1/2} (h) | Cₘₐₓ (ng/ mL) | Tₘₐₓ (h) | T_{1/2} (h) | AUC_{inf} (h*ng/ mL) | F (%) |
| 10 | 102 | 19736 | 7.14 | 8197 | 1.67 | 7.77 | 95629 | 96.9 |

| | | | | | | | | |
|---|---|---|---|---|---|---|---|---|
| Note: Detection results at 24 h. | | | | | | | | |

| Compound | iv (2 mg/kg) | po (10 mg/kg) | | |
|---|---|---|---|---|
| | AUC_{inf} (h*ng/mL) | Cₘₐₓ (ng/mL) | Tₘₐₓ (h) | AUC_{inf} (h*ng/mL) |
| 61 | 7077 | 5103 | 1.00 | 42906 |

| | | | | |
|---|---|---|---|---|
| Note: Detection results at 24 h. | | | | |

| Compound | iv (2 mg/kg) | po (10 mg/kg) | | |
|---|---|---|---|---|
| | AUC_{inf} (h*ng/mL) | Cₘₐₓ (ng/mL) | Tₘₐₓ (h) | AUC_{inf} (h*ng/mL) |
| 41 | 7207 | 4557 | 1.67 | 43702 |

| | | | | |
|---|---|---|---|---|
| Note: Detection results at 24 h. | | | | |

| Compound | iv (2 mg/kg) | po (10 mg/kg) | | | |
|---|---|---|---|---|---|
| | AUC_{inf} (h*ng/mL) | Cₘₐₓ (ng/mL) | Tₘₐₓ (h) | AUC_{inf} (h*ng/mL) | F (%) |
| 65 | 17341 | 7807 | 1.67 | 85040 | 98.1 |

| | | | | | |
|---|---|---|---|---|---|
| Note: Detection results at 24 h. | | | | | |

| Compound | iv (2 mg/kg) | po (10 mg/kg) | | | |
|---|---|---|---|---|---|
| | AUC_{inf} (h*ng/mL) | Cₘₐₓ (ng/mL) | Tₘₐₓ (h) | AUC_{inf} (h*ng/mL) | F (%) |
| LY3502970 | 10268 | 2660 | 1.17 | 23822 | 46.4 |

| | | | | | |
|---|---|---|---|---|---|
| Note: Detection results at 24 h. | | | | | |

### Test Example 3: In Vivo Pharmacokinetic Study in Rats

Experimental animals: SD rats (female, 20-25 g) were purchased from Beijing Vital River Laboratory Animal Technology Co., Ltd.

Test steps: SD rats were administered the test compound by tail vein injection or intragastric administration. Whole blood samples of the rats were collected before administration, at 15 min, 0.5 h, 1 h, 2 h, 4 h, 8 h, 24 h. Plasma was separated by centrifugation, compound concentrations were determined by LC-MS/MS, and pharmacokinetic parameters including plasma elimination half-life (T1/2), peak time (Tmax), peak concentration (Cmax), and area under the concentration-time curve (AUC) were calculated using Winnolin software.

Intravenous dose: 2 mg/kg; intragastric administration dose: 10 mg/kg.

The compounds of the present invention have excellent oral administration properties. The oral PK parameters of exemplary compounds are respectively listed in the table below. Compared with LY 3502970, the compounds have better pharmacokinetic properties, in particular better oral absorption properties.

| Compound | Cₘₐₓ (ng/mL) | Tₘₐₓ (h) | T_{1/2} (h) | AUC_{inf} (h*ng/mL) |
|---|---|---|---|---|
| 8 | 2947 | 2.0 | 3.86 | 24500 |
| 65 | 3190 | 3.33 | 7.47 | 31480 |
| LY3502970 | 1006 | 2.0 | 6.86 | 9190 |

### Test Example 4: Study on Weight-Loss Drug Efficacy in DIO C57BL/6 hGLP1R Mice

1. Test objective: To compare, at the same dose, the weight-loss drug efficacy of the test compound and LY 3502970 administered to obese mice by intragastric administration at a dose of 0.3 mpk.
2. Test animals
After purchased, animals (species: C57BL/6 humanized transgenic (hGLP1R) mice; Mouse age: 6 weeks old; Sex: male) were fed with a high-fat diet (60 kcal% fat) for high-fat induction for not less than 12 weeks, until the body weights of the animals reached more than 40 g.
3. Test grouping

| Group | Drug | Number of animals per group | Dose | Dosing concentration | Dosing volume | Route of administration | Dosing frequency | Vehicle |
|---|---|---|---|---|---|---|---|---|
| | | | mg/kg | mg/mL | mL/kg | | | |
| G1 | Vehicle | 5 | 0 | 0 | 5 | i.g. | qd | 10% DMSO + 10% EL35 + 15% PEG400 + 65% (100 mM glycine-NaOH buffer, pH = 10) |
| G2 | 10 | 5 | 0.3 | 0.06 | | i.g. | qd | |
| G3 | LY3502970 | 5 | 0.3 | 0.06 | | i.g. | qd | |

| | | | | | | | | |
|---|---|---|---|---|---|---|---|---|
| Note: i.g. represents intragastric administration; qd represents once daily. | | | | | | | | |

4. Test method
Before administration, the mice were weighed, and the food intake of each mouse over one day (i.e., one complete night plus daytime period) was measured. The mice were evenly assigned to groups based on the body weight and food intake.
The mouse body weight was measured at a fixed time point each day, and the body weight change rate was calculated. After measurement, the mice were administered the corresponding drugs according to their grouping and current body weight.
The body weight of the mice was recorded daily, the body weight change rate was calculated, and the data were recorded and plotted.

5. Test results: after 18 days of continuous administration, the body weight change rates of mice in each group were statistically analyzed. Compared with the Vehicle group, Compound 10 of the present invention achieved a significantly better weight-loss effect (an average body weight gain rate of Compound 10 was - 20.2%, p < 0.001); compared with the LY3502970 group (an average body weight gain rate of LY3502970 was -15.1%), Compound 10 of the present invention achieved a significantly better weight-loss effect (p < 0.05).

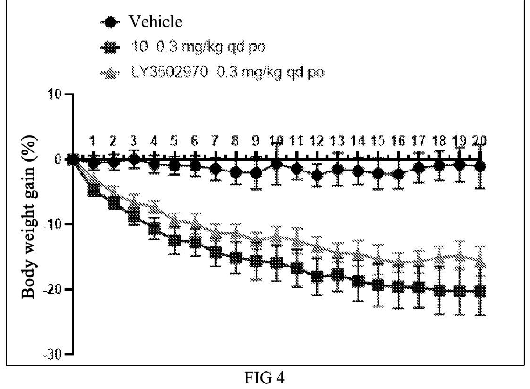

After 20 days of continuous administration, the body weight change rates of mice in each group were statistically analyzed. Compared with the Vehicle group, Compound 10 of the present invention achieved a significantly better weight-loss effect (the average body weight gain rate of Compound 10 was -20.3%, p < 0.001); compared with the LY3502970 group (an average body weight gain rate of LY3502970 was - 15.7%), Compound 10 of the present invention achieved a significantly better weight-loss effect (p < 0.05). The graph of the body weight gain rates is shown in FIG. 4.

### Test Example 5: Drug Efficacy Study in an Intraperitoneal Glucose Tolerance Test (IPGTT) in hGLP-1R Transgenic Mice

1. Test objective: To investigate the effects on blood glucose at different time points after glucose loading in hGLP-1R transgenic mice following single intragastric administration of different doses of the test compound.
2. Test animals: C57BL/6JSmoc-GIp1r^{em2(hGLP1R)Smoc} mice, male, approximately 23 g.
3. Test grouping: before the start of the test, the mice were fasted without water deprivation for approximately 16 h, and basal blood glucose levels of mice were measured. Animals were evenly assigned to groups based on basal blood glucose and mouse body weight using a randomization grouping method, with 7 mice in each group.
4. Test method: each group was administered the corresponding test compound by intragastric administration, with an administration volume of 10 mL/kg. The vehicle control group was administered, at the same frequency and the same administration volume, 2.5% DMSO + 10% Kolliphor^{®} EL + 15% PEG400 + 72.5% (100 mM glycine-NaOH buffer, pH = 10). At 60 min after administration, a glucose solution was intraperitoneally injected (i.p.) at 2 g/kg based on the mouse body weight, with an injection volume of 10 mL/kg, and the time of glucose administration was set as time 0. Blood glucose levels were measured before glucose administration and at 15 min, 30 min, 60 min, and 120 min after glucose administration.
5. Specific grouping was as follows:

| **Group** | **Drug** | **Number of animals** | **Dose** | **Dosing concentration** | **Dosing volume** | **Route of administration** | **Dosing frequency** |
|---|---|---|---|---|---|---|---|
| | | | **mg/kg** | **mg/mL** | **mL/kg** | | |
| G1 | vehicle | 6 | 0 | - | 10 | i.g. | Single |
| G4 | Compound 10 | 6 | 0.05 | 0.005 | | i.g. | Single |

6. Test results:
Blood glucose test results showed that, at a dose of 0.05 mg/kg, blood glucose levels in the Compound 10 group showed a decreasing trend at 15 min, 30 min, 60 min, and 120 min after glucose loading and were significantly lower than those in the Vehicle group (P < 0.05, P < 0.01, P < 0.001). Calculation of the area under the blood glucose curve showed that, compared with the Vehicle group, AUC_{0-120min Glu} after glucose loading of mice in the Compound 10 group was significantly reduced (P < 0.001). The results are shown in the table below.

| Mean blood glucose AUC (mmol-min/L) (Mean ± SD, n = 5) | | |
|---|---|---|
| Time point (min) | G1 (Vehicle) | G4 (Compound 10) 0.05 mg/kg |
| 0-120 | 1790.5±176.4 | 949.6±87.6 |

### Test Example 6: Safety Assessment Test of Off-Target Effects on 44 Targets

1. Test objective: This study mainly used radioisotope binding methods, calcium flux assays, and enzymatic fluorescence or chemiluminescence techniques to detect, at the in vitro cellular or molecular level, the off-target effects of the test compound on 44 selected targets, thereby evaluating the relevant safety associated with binding or activity inhibition effects of these targets by the test compound.
2. Test method: Test Compound 10 (free acid) was tested at a single concentration of 10 µM (the test concentration for Eta agonism was 12 µM) to assess binding, inhibition, or agonistic effects on 44 targets.
3. Test steps: Radioligand competition assays were used to detect the binding effects of the test compound on 23 GPCRs (ADORA2A, Alpha1A, Alpha2A, Beta1, Beta2, CB1, CB2, CCKa, D1, D2L, H1, H2, op-delta, op-kappa, op-mu, M1, M2, M3, 5-HT1A, 5-HT1B, 5-HT2A, 5-HT2B, and V1A), 8 ion channels (nAChR-Alpha7, Cav1.2 (Dihydropyridine Site), GABAA (α1β3γ2), hERG, NMDA (MK-801), 5-HT3, Na⁺, and Kv), 3 transporters (DAT, NET, and 5-HTT), and 2 nuclear receptors (AR and GR). Calcium flux tests were used to detect the agonistic and antagonistic effects of the test compound on the Eta receptor. The effects of the test compound on 7 enzyme targets were tested using an in vitro biochemical platform.
4. Test results:
   At a test concentration of 10 µM, Compound 10 showed no obvious binding or inhibition effect (inhibition rate < 50%) or agonistic effect (agonism rate < 50%) on any of the 44 targets at a single concentration of 10 µM (the test concentration for Eta agonistic effect was 12 µM).

At the test concentration of 10 µM, LY3502970 showed mild inhibition effects on Ca²⁺-L, 5HT2B, and op-delta (inhibition rate > 50%). This may give rise to off-target risks associated with the relevant targets. The implementations of the present invention have been described above. However, the present invention is not limited to the above-described implementations. Any modifications, equivalent substitutions, improvements, and the like made within the spirit and principles of the present invention shall fall within the protection scope of the present invention.

## Claims

1. A compound as shown in formula (I), or stereoisomers, tautomers or pharmaceutically acceptable salts thereof: 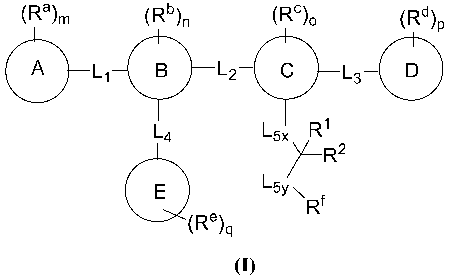 wherein,
Ring A is selected from C₃₋₁₅ carbocyclyl, 3-12 membered heterocyclyl, C₆₋₁₄ aryl, and 5-12 membered heteroaryl;
Ring B is selected from 3-12 membered heterocyclyl, C₆₋₁₄ aryl, and 5-12 membered heteroaryl;
Ring C is selected from 9-membered bicyclic heteroaryl;
Ring D is selected from C₃₋₁₅ carbocyclyl, 3-12 membered heterocyclyl, C₆₋₁₄ aryl, and 5-12 membered heteroaryl;
Ring E is selected from C₃₋₁₅ carbocyclyl, 3-12 membered heterocyclyl, C₆₋₁₄ aryl, and 5-12 membered heteroaryl;
R^{a}, at each occurrence, is independently selected from hydrogen, deuterium, halogen, hydroxy, amino, nitro, mercapto, cyano, oxo, -OR^{a1}, -S(O)R^{a1}, -SO₂(R^{a1}), -C(O)R^{a1}, -C(O)OR^{a1}, -OC(O)R^{a1}, -N(R^{a1})(R^{a2}), - C(O)N(R^{a1})(R^{a2}), -N(R^{a1})C(O)R^{a2}, -S(O)N(R^{a1})(R^{a2}), -SO₂N(R^{a1})(R^{a2}), -N(R^{a1})S(O)R^{a2}, -N(R^{a1})S(O)₂R^{a2}, and the following groups optionally substituted with one or more (for example, 2, 3, 4, 5, 6, 7, or 8) R^{a3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{a}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{a1} and R^{a2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{a3}, at each occurrence, is independently selected from hydrogen, deuterium, halogen, hydroxy, amino, nitro, mercapto, cyano, oxo, -R^{a4}, -OR^{a4}, -SR^{a4}, -S(O)R^{a4}, -S(O)₂R^{a4}, -C(O)R^{a4}, -C(O)OR^{a4}, -OC(O)R^{a4}, - N(R^{a4})(R^{a5}), -C(O)N(R^{a4})(R^{a5}), -N(R^{a4})C(O)R^{a5}, -S(O)N(R^{a4})(R^{a5}), -S(O)₂N(R^{a4})(R^{a5}), -N(R^{a4})S(O)R^{a5}, - N(R^{a4})S(O)₂R^{a5};
R^{a4} and R^{a5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{b}, at each occurrence, is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or one or more (for example, 2, 3, 4, 5, 6, 7, or 8) substitutable sites of a substituted group each independently substituted with R^{b1};
or two R^{b}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{b1}, at each occurrence, is independently selected from deuterium, halogen, hydroxy, amino, nitro, mercapto, cyano, oxo, -R^{b2}, -OR^{b2}, -SR^{b2}, -S(O)R^{b2}, -S(O)₂R^{b2}, -C(O)R^{b2}, -C(O)OR^{b2}, -OC(O)R^{b2}, - N(R^{b2})(R^{b3}), -C(O)N(R^{b2})(R^{b3}), -N(R^{b2})C(O)R^{b3}, -S(O)N(R^{b2})(R^{b3}), -S(O)₂N(R^{b2})(R^{b3}), -N(R^{b2})S(O)R^{b3}, - N(R^{b2})S(O)₂R^{b3};
R^{b2} and R^{b3}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
R^{c}, at each occurrence, is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or one or more (for example, 2, 3, 4, 5, 6, 7, or 8) substitutable sites of a substituted group each independently substituted with R^{c1};
or two R^{c}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{c1}, at each occurrence, is independently selected from deuterium, halogen, hydroxy, amino, nitro, mercapto, cyano, oxo, -R^{c2}, -OR^{c2}, -SR^{c2}, -S(O)R^{c2}, -S(O)₂R^{c2}, -C(O)R^{c2}, -C(O)OR^{c2}, -OC(O)R^{c2}, - N(R^{c2})(R^{c3}), -C(O)N(R^{c2})(R^{c3}), -N(R^{c2})C(O)R^{c3}, -S(O)N(R^{c2})(R^{c3}), -S(O)₂N(R^{c2})(R^{c3}), -N(R^{c2})S(O)R^{c3}, - N(R^{c2})S(O)₂R^{c3};
R^{c2} and R^{c3}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) groups selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
R^{d}, at each occurrence, is independently selected from hydrogen, deuterium, halogen, hydroxy, amino, nitro, mercapto, cyano, oxo, -OR^{d1}, -S(O)R^{d1}, -S(O)₂R^{d1}, -C(O)R^{d1}, -C(O)OR^{d1}, -OC(O)R^{d1}, -N(R^{d1})(R^{d2}), - C(O)N(R^{d1})(R^{d2}), -N(R^{d1})C(O)R^{d2}, -S(O)N(R^{d1})(R^{d2}), -S(O)₂N(R^{d1})(R^{d2}), -N(R^{d1})S(O)R^{d2}, -N(R^{d1})S(O)₂R^{d2}, and the following groups optionally substituted with one or more (for example, 2, 3, 4, 5, 6, 7, or 8) R^{d3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{d}, together with atoms to which they are attached, form an optionally substituted 3-10 membered heterocyclyl, an optionally substituted 3-7 membered cycloalkyl, or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{d1} and R^{d2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{d3}, at each occurrence, is independently selected from hydrogen, deuterium, halogen, hydroxy, amino, nitro, mercapto, cyano, oxo, -R^{d4}, -OR^{d4}, -SR^{d4}, -S(O)R^{d4}, -S(O)₂R^{d4}, -C(O)R^{d4}, -C(O)OR^{d4}, -OC(O)R^{d4}, - N(R^{d4})(R^{d5}), -C(O)N(R^{d4})(R^{d5}), -N(R^{d4})C(O)R^{d5}, -S(O)N(R^{d4})(^{Rd5}), -S(O)₂N(R^{d4})(R^{d5}), -N(R^{d4})S(O)R^{d5},-N(R^{d4})S(O)₂R^{d5};
R^{d4} and R^{d5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{e}, at each occurrence, is independently selected from hydrogen, deuterium, halogen, hydroxy, amino, nitro, mercapto, cyano, oxo, -OR^{e1}, -SR^{e1}, -S(O)R^{e1}, -S(O)₂R^{e1}, -C(O)R^{e1}, -C(O)OR^{e1}, -OC(O)R^{e1}, - N(R^{e1})(R^{e2}), -C(O)N(R^{e1})(R^{e2}), -N(R^{e1})C(O)R^{e2}, -S(O)N(R^{e1})(R^{e2}), -S(O)₂N(R^{e1})(R^{e2}), -N(R^{e1})S(O)R^{e2}, - N(R^{e1})S(O)₂R^{e2}, -C(O)N(R^{e1})S(O)₂N(R^{e1})(R^{e2}), -C(O)N(R^{e1})S(O)₂R^{e2}, -P(O)(R^{e1})R^{e2}, and the following groups optionally substituted with one or more (for example, 2, 3, 4, 5, 6, 7, or 8) R^{e3}: C₁₋₆ alkyl, C₁₋₆ alkoxy, C₁₋₆ alkylamino, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl;
or two R^{e}, together with atoms to which they are attached, form an optionally substituted 3-10 membered heterocyclyl or an optionally substituted 5-12 membered heteroaryl, and optionally substituted means unsubstituted or substituted with one or more (for example, 2, 3, 4, 5, 6, 7, or 8) groups selected from deuterium, halogen, hydroxy, amino, nitro, mercapto, cyano, oxo, C₁₋₆ alkyl, C₁₋₆ alkoxy, C₃₋₁₀ cycloalkyl, 3-10 membered heterocyclyl, C₆₋₁₄ aryl, and 5-12 membered heteroaryl;
R^{e1} and R^{e2}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from hydrogen, halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R^{e3}, at each occurrence, is independently selected from hydrogen, deuterium, halogen, hydroxy, amino, nitro, mercapto, cyano, oxo, -R^{e4}, -OR^{e4}, -SR^{e4}, -S(O)R^{e4}, -S(O)₂R^{e4}, -C(O)R^{e4}, -C(O)OR^{e4}, -OC(O)R^{e4}, - N(R^{e4})(R^{e5}), -C(O)N(R^{e4})(R^{e5}), -N(R^{e4})C(O)R^{e5}, -S(O)N(R^{e4})(R^{e5}), -S(O)₂N(R^{e4})(R^{e5}), -N(R^{e4})S(O)R^{e5}, - N(R^{e4})S(O)₂R^{e5}, -C(O)N(R^{e4})S(O)₂N(R^{e4})(R^{e5}), -C(O)N(R^{e4})S(O)₂R^{e5}, -P(O)(R^{e4})R^{e5};
R^{e4} and R^{e5}, at each occurrence, are respectively independently selected from hydrogen, deuterium, and the following optionally substituted groups: C₁₋₆ alkyl, C₁₋₆ alkoxy, C₂₋₆ alkenyl, C₂₋₆ alkynyl, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₇ cycloalkyl, 3-7 membered heterocyclyl, phenyl, and 5-6 membered heteroaryl;
R^{f} is selected from -C(O)OR^{f1}, -C(O)N(R^{f1})(R^{f2}), 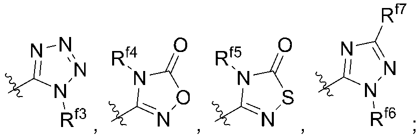
R^{f1}, R^{f2}, R^{f3}, R^{f4}, R^{f5}, R^{f6} and R^{f7}, at each occurrence, are respectively independently selected from hydrogen, deuterium, and the following optionally substituted groups: C₁₋₆ alkyl, C₁₋₆ alkoxy, C₃₋₁₀ cycloalkyl, 3-10 membered heterocyclyl, C₆₋₁₄ aryl, and 5-12 membered heteroaryl; optionally substituted means unsubstituted or substituted with one or more (for example, 2, 3, 4, 5, 6, 7, or 8) substituents selected from halogen, hydroxy, amino, nitro, mercapto, cyano, oxo, C₁₋₆ alkyl, C₁₋₆ alkoxy, C₂₋₆ alkenyl, C₂₋₆ alkynyl, C₃₋₆ cycloalkyl, 3-6 membered heterocyclyl, phenyl, and 5-6 membered heteroaryl;
R¹ and R², together with atoms to which they are attached, form C₃₋₁₅ carbocyclyl; the C₃₋₁₅ carbocyclyl is optionally substituted with one or more (for example, 2, 3, 4, 5, 6, 7, or 8) R³; R³ is independently selected from hydrogen, deuterium, halogen, hydroxy, or the following optionally substituted groups: C₁₋₆ alkyl, C₁₋₆ alkoxy, C₁₋₆ alkylamino, C₃₋₁₀ cycloalkyl, and 3-10 membered heterocyclyl; optionally substituted means unsubstituted or one or more (for example, 2, 3, 4, 5, 6, 7, or 8) substitutable sites of a substituted group each independently substituted with deuterium, halogen, hydroxy, amino, cyano, oxo, C₃₋₁₀ cycloalkyl, and 3-10 membered heterocyclyl;
L₁ is selected from a bond, -C(R^{L1})₂-, -O-, -C(R^{L1})₂O-, -S-, -C(O)-, -C(O)O-, -OC(O)-, -N(R^{L1})C(O)-, - C(O)N(R^{L1})-, or -N(R^{L1})-; R^{L1} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₂ is independently selected from a bond, -C(R^{L2})₂-, -O-, -C(R^{L2})₂O-, -S-, -C(O)-, -C(O)O-, -OC(O)-, - N(R^{L2})C(O)-, -C(O)N(R^{L2})-, or -N(R^{L2})-; R^{L2} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₃ is independently selected from a bond, -C(R^{L3})₂-, -O-, -S-, -C(O)-, -C(O)O-, -OC(O)-, -N(R^{L3})C(O)-, -C(O)N(R^{L3})-, or -N(R^{L3})-; R^{L3} is independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl, and optionally substituted means unsubstituted or substituted with one or more (for example, 2, 3, 4, 5, 6, 7, or 8) groups selected from deuterium, halogen, hydroxy, amino, nitro, mercapto, cyano, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₄ is independently selected from a bond, -L₄ₓ-C(R^{L4a}) (R^{L4b})-L_{4y}-, -L₄ₓ-O-L_{4y}-, -L₄ₓ-S-L_{4y}-, -L₄ₓ-C(O)-L_{4y}-, -L₄ₓ-C(O)O-L_{4y}-, -L₄ₓ-OC(O)-L_{4y}-, -L₄ₓ-N(R^{L4a})C(O)-L_{4y}-, -L₄ₓ-C(O)N(R^{L4a})-L_{4y}-, -L₄ₓ-N(R^{L4a})-L_{4y}-, -L₄ₓ-N(R^{L4a})C(O)N(R^{L4b})-L_{4y}-, -L₄ₓ-N(R^{L4a})C(S)N(R^{L4b})-L_{4y}-, or 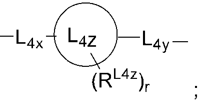
L₄ₓ and L_{4y} are respectively independently selected from a bond or optionally substituted C₁₋₁₀ alkylene; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
R^{L4a} and R^{L4b} are respectively independently selected from hydrogen, deuterium, or the following optionally substituted groups: C₁₋₆ alkyl, C₁₋₆ alkoxy, C₂₋₆ alkenyl, C₂₋₆ alkynyl, C₃₋₁₀ cycloalkyl, and 3-10 membered heterocyclyl; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
Ring L_{4Z} is selected from C₃₋₁₅ carbocyclyl, 3-12 membered heterocyclyl, C₆₋₁₄ aryl, and 5-12 membered heteroaryl;
R^{L4z} is independently selected from hydrogen, deuterium, halogen, hydroxy, amino, nitro, mercapto, cyano, oxo, or the following optionally substituted groups: C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl; optionally substituted means unsubstituted or one or more substitutable sites of a substituted group independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
L₅ₓ and L_{5y} are respectively independently selected from a bond or optionally substituted C₁₋₁₀ alkylene; optionally substituted means unsubstituted or one or more (for example, 2, 3, 4, 5, 6, 7, or 8) substitutable sites of a substituted group each independently substituted with deuterium, halogen, hydroxy, amino, nitro, mercapto, cyano, oxo, C₁₋₆ alkyl, C₁₋₆ alkoxy, C₁₋₆ alkylamino, C₂₋₆ alkenyl, C₂₋₆ alkynyl, C₃₋₁₀ cycloalkyl, and 3-10 membered heterocyclyl;
m, n, o, p, q, and r are each independently 0, 1, 2, or 3;
unless otherwise stated, heteroatoms in the above-mentioned heterocyclyl and heteroaryl are independently selected from O, N or S, and the number of the heteroatoms is 1, 2, 3 or 4.

2. The compound according to claim 1, or stereoisomers, tautomers or pharmaceutically acceptable salts thereof, wherein
Ring A is selected from phenyl;
Ring B is selected from 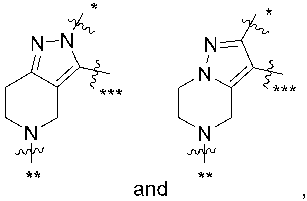 wherein a "*" end is an end connected to L₁, a "**" end is an end connected to L₂, and a "***" end is an end connected to L₄;
Ring C is selected from the following group: 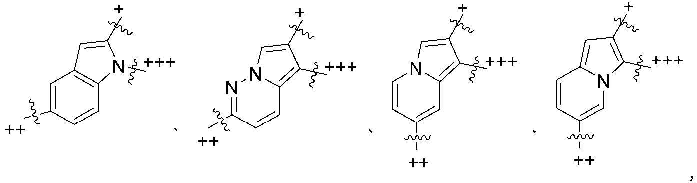 wherein a "+" end is an end connected to L₂, a "++" end is an end connected to L₃, and a "+++" end is an end connected to L₅ₓ;
Ring D is 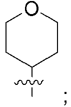
Ring E is 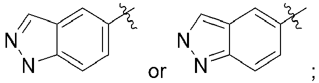
L₁ is selected from a bond;
L₂ is selected from -C(O)-;
L₃ is selected from a bond;
L₄ is selected from -NHC(O)NH-, -N(methyl)C(O)NH-, -N(cyclopropyl)C(O)NH-, - N(cyclopropyl)C(O)N(cyclopropyl)-, and 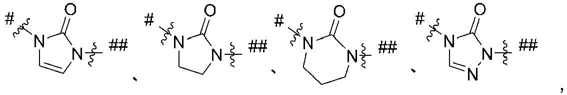 wherein a "#" end is an end connected to the Ring B, and a "##" end is an end connected to the Ring E;
L₅ₓ and L_{5y} are respectively independently selected from a bond;
R^{a}, at each occurrence, is independently selected from halogen, methyl, and C₃₋₅ cycloalkyl-(CH₂)_{w}-;
R^{b}, at each occurrence, is independently selected from fluoro, chloro, oxo, and methyl;
R^{c}, at each occurrence, is independently selected from hydrogen, fluoro, chloro, oxo, methyl, ethyl, n-propyl, isopropyl, and n-butyl;
R^{d}, at each occurrence, is independently selected from optionally substituted C₁₋₃ alkyl and optionally substituted C₁₋₃ alkoxy; optionally substituted means unsubstituted or substituted with one or more (for example, 2, 3, 4, or 5) substituents selected from hydrogen, deuterium, halogen, hydroxy, and amino;
R^{e}, at each occurrence, is each independently selected from fluoro or the following optionally substituted groups: C₁₋₃ alkyl (for example, methyl or ethyl), C₃₋₅ cycloalkyl (for example, cyclopropyl, cyclobutyl, and cyclopentyl), and C₃₋₅ cycloalkyl-(CH₂)_{w}-; provided that, when L₄ is 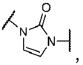 at least 1 (for example, 1 or 2) of R^{a} or substituents on the Ring E (or R^{e}) is C₃₋₅ cycloalkyl-(CH₂)_{w}-;
or two R^{e} (preferably two adjacent R^{e}; further preferably, two adjacent R^{e} located on different rings), together with atoms to which they are each attached, form optionally substituted 5-7 membered heterocyclyl (for example, 5-membered, 6-membered, or 7-membered heterocyclyl), and the heterocyclyl contains 1-3 (for example, 1, 2, or 3) heteroatoms each independently selected from N, O, or S; optionally substituted means that hydrogen on a substituted group is unsubstituted or one or more substitutable sites of the substituted group are independently substituted with R^{e3};
a carbocycle formed by R¹ and R² together with atoms to which they are attached is independently selected from 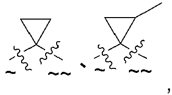 wherein a "~" end is an end connected to L₅ₓ, and a "~~" end is an end connected to L_{5y};
R^{f} is selected from 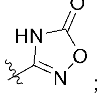
m is 2 or 3; q is 1, 2, or 3;
n, o, and p are each independently 0, 1, or 2;
w is 0, 1, or 2;
the R^{e3} is as defined in claim 1;
provided that the following compounds are not included:

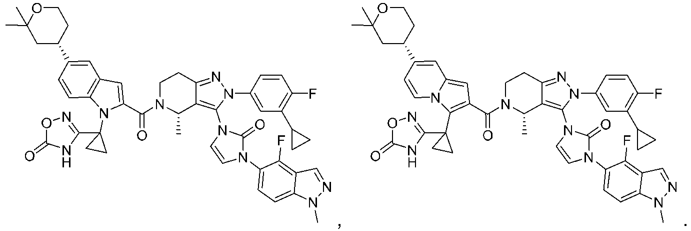

3. The compound according to claim 1 or 2, or stereoisomers, tautomers or pharmaceutically acceptable salts thereof, wherein
provided that, when L₄ is 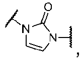 at least 1 (for example, 1 or 2) of R^{a} or substituents on the Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene; or, when neither R^{a} nor the substituents on the Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene, two R^{e} (preferably two R^{e} located on different rings), together with atoms to which they are each attached, form optionally substituted 5-7 membered heterocyclyl, and the heterocyclyl (preferably heterocycloalkyl) contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; the R^{e3} is as defined for the compound of formula (I);
preferably, the C₃₋₅ cycloalkyl is selected from cyclopropyl, cyclobutyl, and cyclopentyl; further preferably cyclopropyl;
provided that the following compounds are not included:

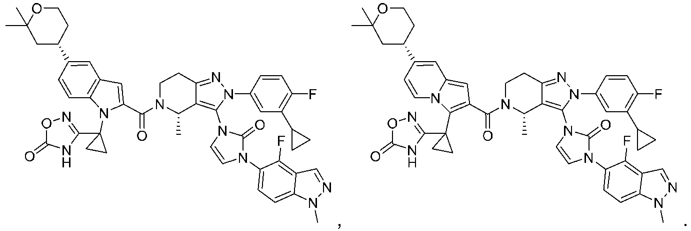

4. The compound according to any one of claims 1-3, or stereoisomers, tautomers or pharmaceutically acceptable salts thereof, wherein
provided that at least 1 (for example, 1 or 2) of R^{a} or substituents on the Ring E (R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene; or, when neither R^{a} nor the substituents on the Ring E (or R^{e}) is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene, two R^{e} (preferably two R^{e} located on different rings), together with atoms to which they are each attached, form optionally substituted 5-7 membered heterocyclyl, and the heterocyclyl (preferably heterocycloalkyl) contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; the R^{e3} is as defined for the compound of formula (I);
preferably, the C₃₋₅ cycloalkyl is selected from cyclopropyl, cyclobutyl, and cyclopentyl; further preferably cyclopropyl.

5. The compound according to any one of claims 1-4, or stereoisomers, tautomers or pharmaceutically acceptable salts thereof, wherein the compound is a compound as shown in the following formula: 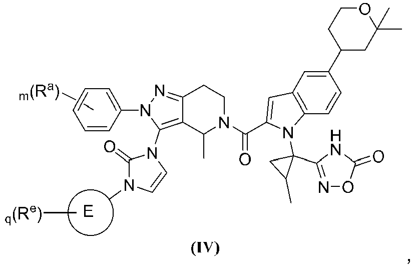
the Ring E is selected from 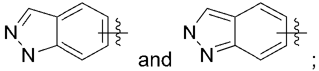
R^{a}, at each occurrence, is each independently selected from fluoro, methyl, and C₃₋₅ cycloalkyl;
m is 2 or 3;
q is 2 or 3;
R^{e}, at each occurrence, is each independently selected from fluoro or the following optionally substituted groups: C₁₋₃ alkyl (for example, methyl or ethyl) and C₃₋₅ cycloalkyl; provided that at least 1 (for example, 1 or 2) of R^{a} or R^{e} is cyclopropyl, cyclobutyl, cyclopentyl, cyclopropyl-methylene, cyclopropyl-ethylene, cyclobutyl-methylene, cyclobutyl-ethylene, cyclopentyl-methylene, or cyclopentyl-ethylene;
or two R^{e} (preferably two adjacent R^{e}; further preferably, two R^{e} that are located on different rings and are adjacent), together with atoms to which they are each attached, form optionally substituted 5-7 membered heterocyclyl, and the heterocyclyl contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3};
R^{e3} is independently selected from deuterium, halogen (for example, fluoro or chloro), hydroxy, amino, oxo, cyano, -R^{e4}, -OR^{e4}, -S(O)₂R^{e4}, -C(O)R^{e4}, -N(R^{e4})(R^{e5}), -S(O)₂N(R^{e4})(R^{e5}), -N(R^{e4})S(O)₂R^{e5}, and - P(O)(R^{e4})R^{e5}; R^{e4} and R^{e5} are respectively independently selected from hydrogen or C₁₋₃ alkyl (for example, methyl, ethyl, and propyl) or C₃₋₆ membered cycloalkyl (preferably, 3-5 membered cycloalkyl, for example, cyclopropyl, cyclobutyl, and cyclopentyl) optionally substituted with one or more (for example, 2 or 3) of hydrogen, deuterium, halogen (for example, fluoro or chloro), hydroxy, amino, cyano, oxo, and methoxy;
preferably, the C₃₋₅ cycloalkyl is selected from cyclopropyl, cyclobutyl, and cyclopentyl; further preferably cyclopropyl.

6. The compound according to any one of claims 1-5, or stereoisomers, tautomers or pharmaceutically acceptable salts thereof, wherein
when 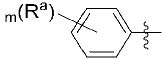 is 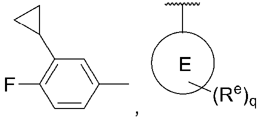 is 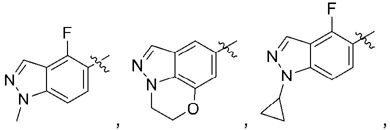 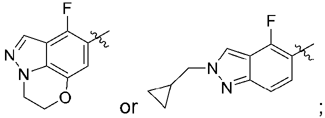 preferably, 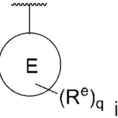 is 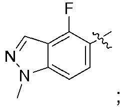
when 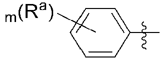 is 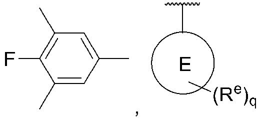 is 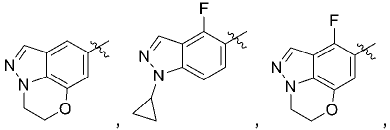 or

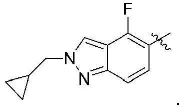

7. The compound according to any one of claims 1-6, or stereoisomers, tautomers or pharmaceutically acceptable salts thereof, wherein the compound is a compound as shown in the following formula:

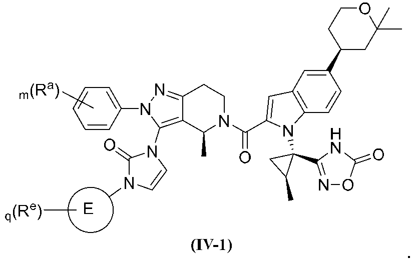

8. The compound according to any one of claims 1-7, or stereoisomers, tautomers or pharmaceutically acceptable salts thereof, wherein the compound is a compound as shown in the following formula: 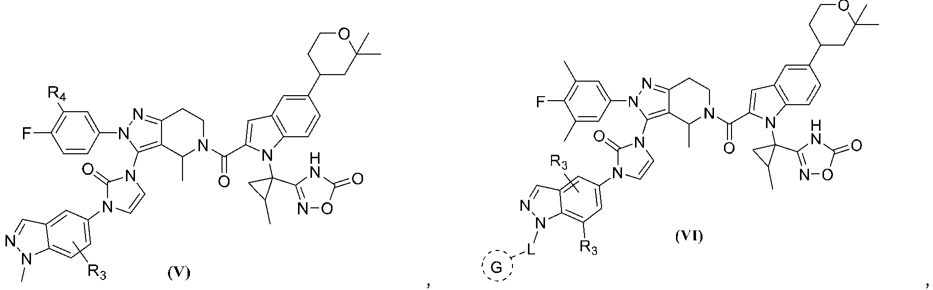 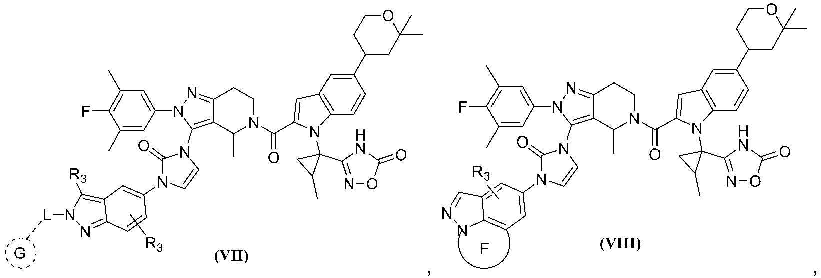 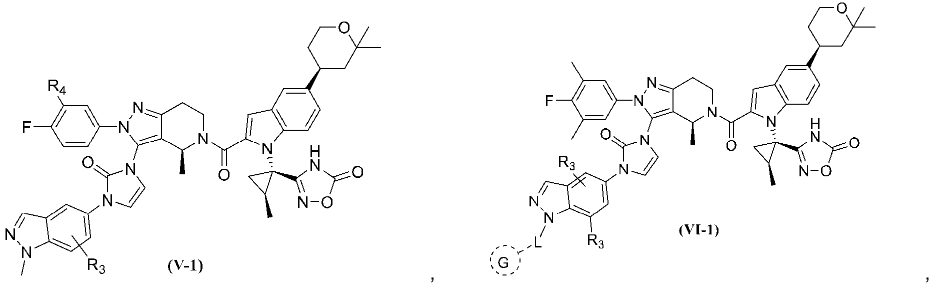 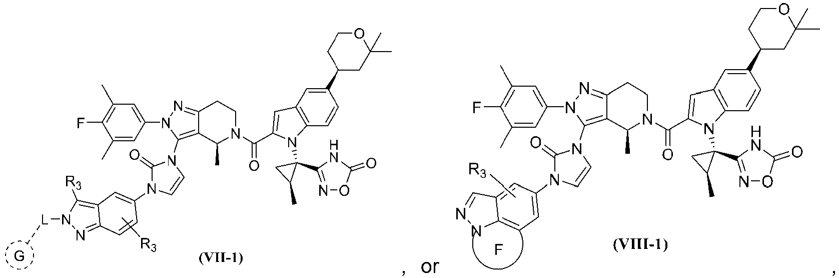 wherein 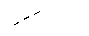 indicates that the bond is present or absent; 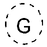
indicates that Ring G is present or absent; when the Ring G is present, the Ring G is C₃₋₅ cycloalkyl (for example, cyclopropyl, cyclobutyl, and cyclopentyl; preferably cyclopropyl);
L is a bond, C₁₋₃ alkylene (for example, methylene, ethylene, or propylene; preferably methylene), or C₁₋₃ alkyl (for example, methyl, ethyl, or propyl; preferably methyl);
or L cyclizes with R₃ adjacent thereto to form optionally substituted 5-7 membered heterocyclyl, and the heterocyclyl contains 1-3 (for example, 1, 2, or 3) heteroatoms each independently selected from N, O, or S, in which case the Ring G is absent; R₃ is selected from H, F, or methyl; R₄ is selected from C₃₋₅ cycloalkyl (for example, cyclopropyl, cyclobutyl, and cyclopentyl; preferably cyclopropyl);
Ring F is optionally substituted 5-7 membered heterocyclyl, and the heterocyclyl contains 1-3 (for example, 1, 2, or 3) heteroatoms each independently selected from N, O, or S;
optionally substituted means that hydrogen on a substituted group is unsubstituted or one or more substitutable sites of the substituted group are independently substituted with R^{e3}; R^{e3} is independently selected from deuterium, halogen (for example, fluoro or chloro), oxo, hydroxy, amino, cyano, -R^{e4}, -OR^{e4}, -SO₂(R^{e4}), -C(O)R^{e4}, -N(R^{e4})(R^{e5}), -SO₂N(R^{e4})(R^{e5}), -N(R^{e4})SO₂(R^{e5}), and -P(O)(R^{e4})R^{e5}; R^{e4} and R^{e5} are respectively independently selected from hydrogen or C₁₋₃ alkyl (for example, methyl, ethyl, and propyl) or C₃₋₆ membered cycloalkyl (preferably, 3-5 membered cycloalkyl, for example, cyclopropyl, cyclobutyl, and cyclopentyl) optionally substituted with one or more (for example, 2 or 3) of hydrogen, halogen (for example, fluoro or chloro), hydroxy, amino, cyano, oxo, and methoxy.

9. The compound according to any one of claims 1-8, or stereoisomers, tautomers or pharmaceutically acceptable salts thereof, wherein the compound is a compound as shown in the following formula:

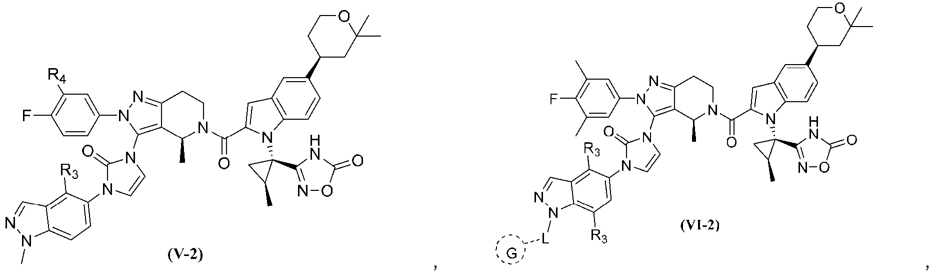

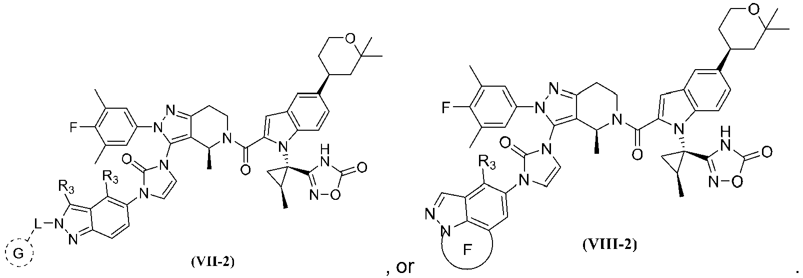

10. Compounds below, or stereoisomers, tautomers or pharmaceutically acceptable salts thereof, wherein the compounds are selected from:
| **Compound Structural Formula and No.** | **Compound Structural Formula and No.** |
|---|---|
| 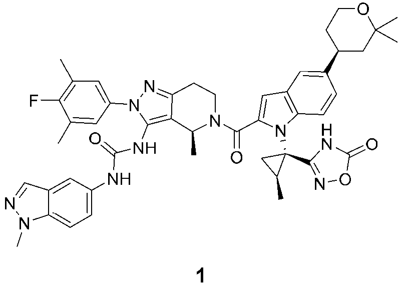 | 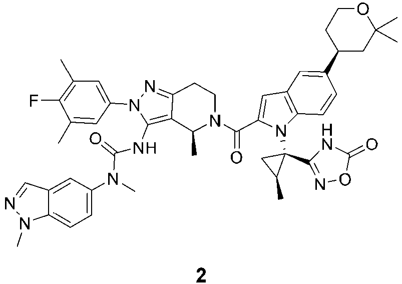 |
| 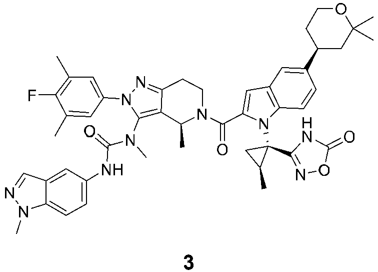 | 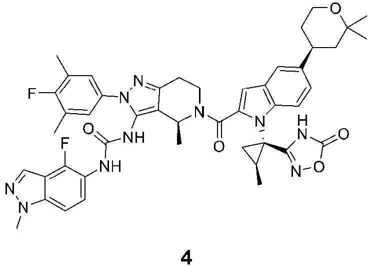 |
| 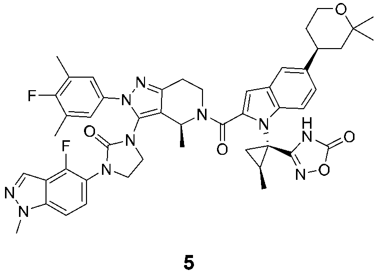 | 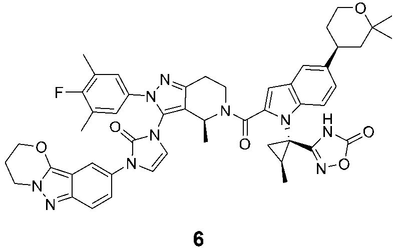 |
| 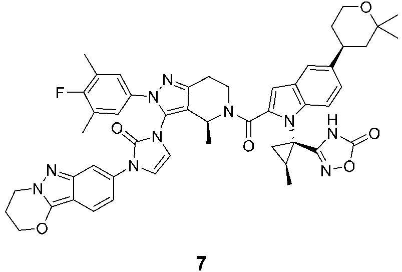 | 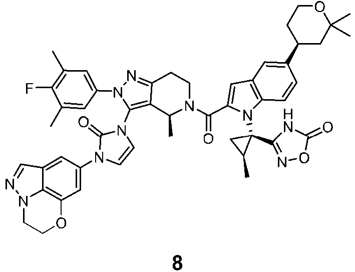 |
| 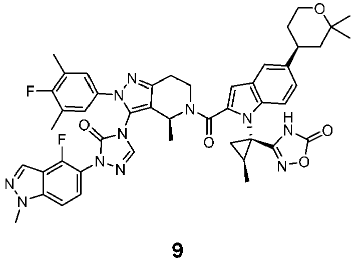 | 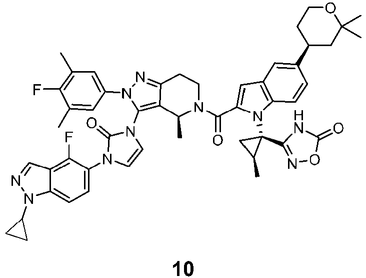 |
| 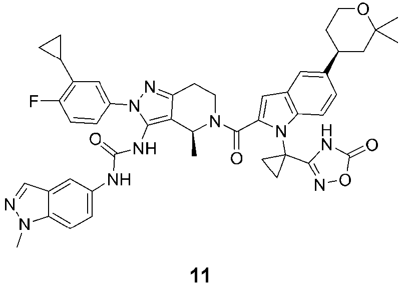 | 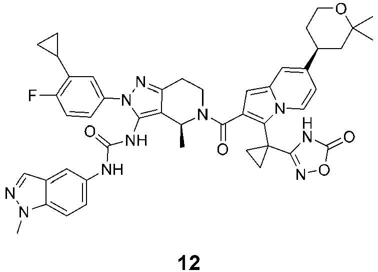 |
| 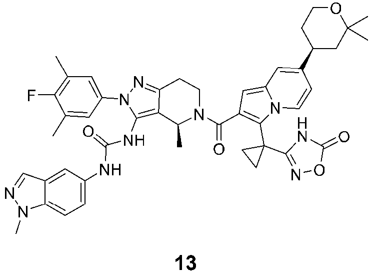 | 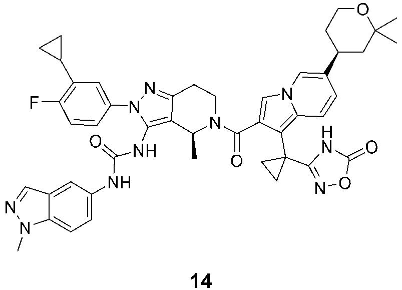 |
| 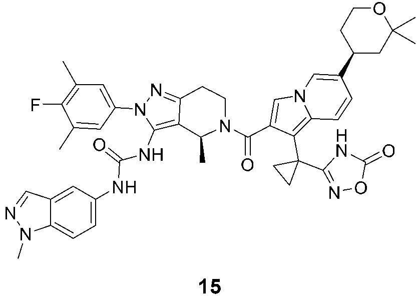 | 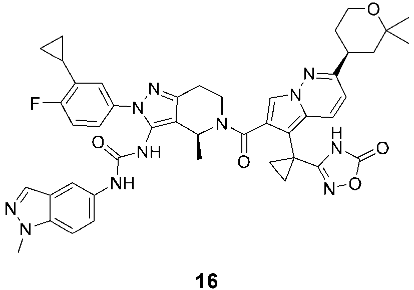 |
| 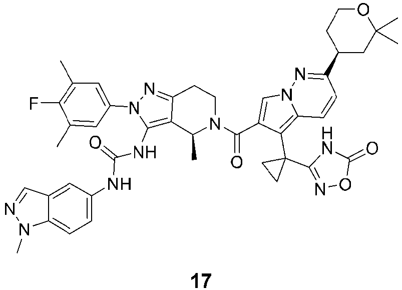 | 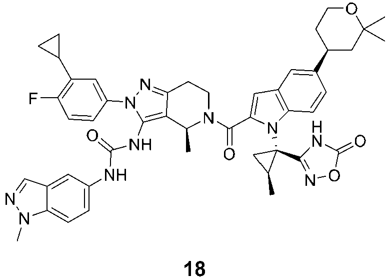 |
| 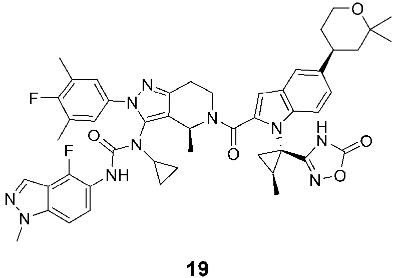 | 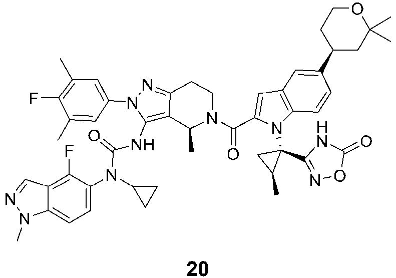 |
| 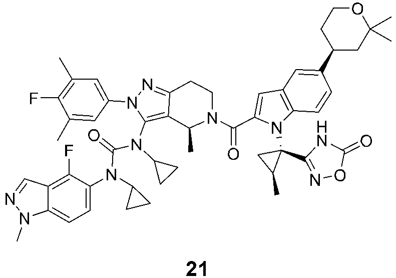 | 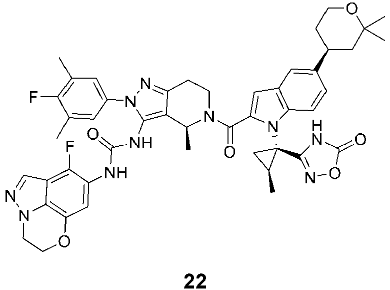 |
| 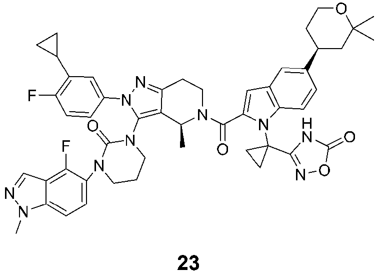 | 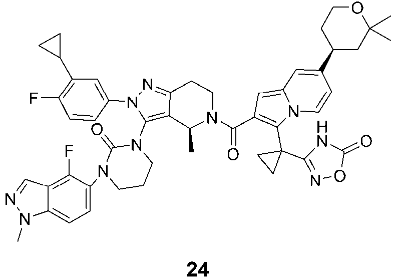 |
| 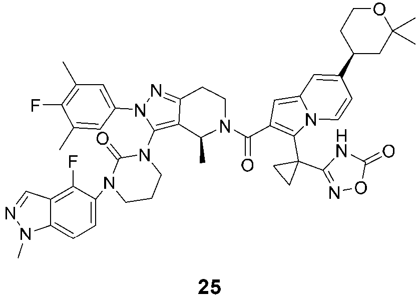 | 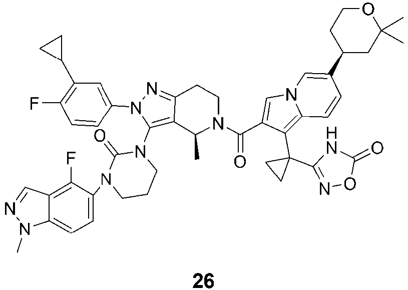 |
| 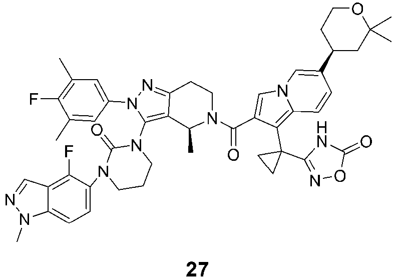 | 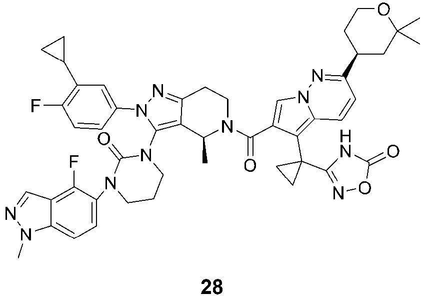 |
| 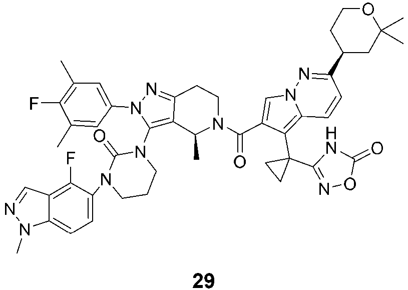 | 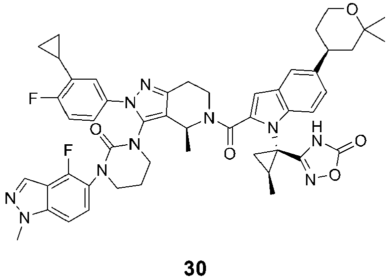 |
| 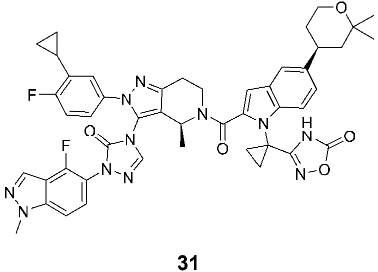 | 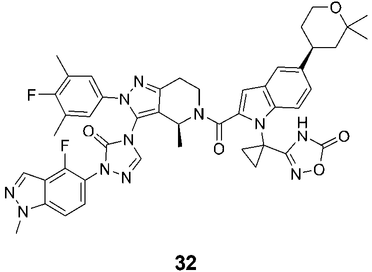 |
| 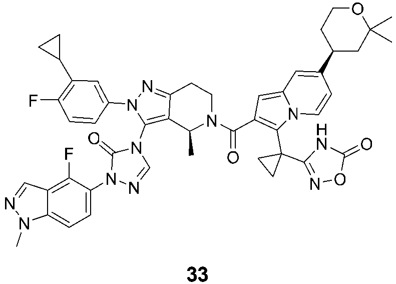 | 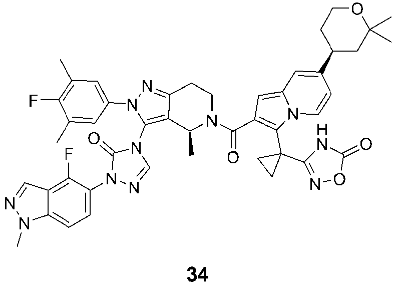 |
| 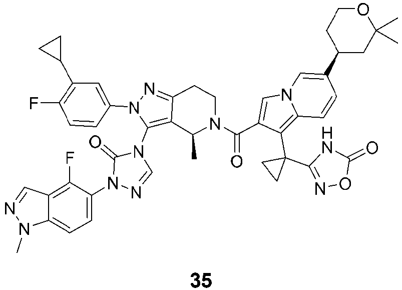 | 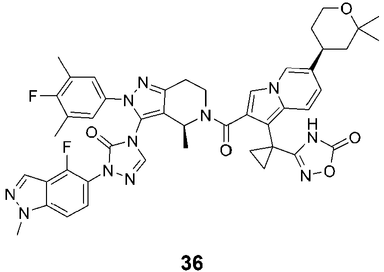 |
| 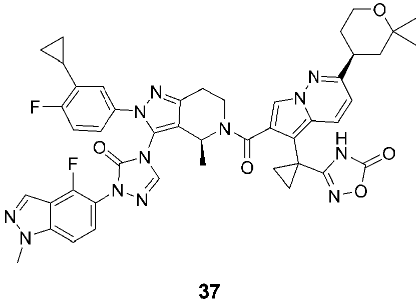 | 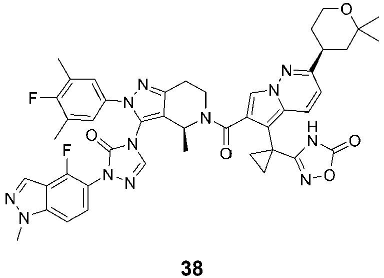 |
| 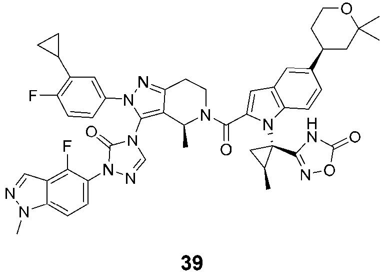 | 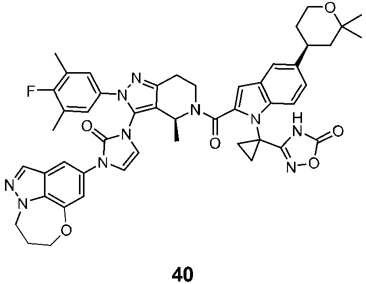 |
| 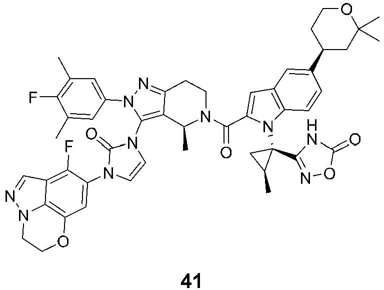 | 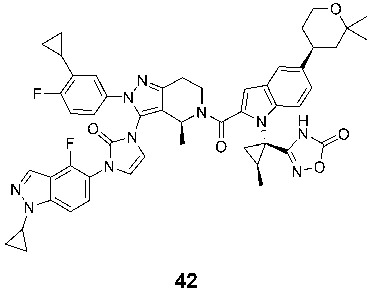 |
| 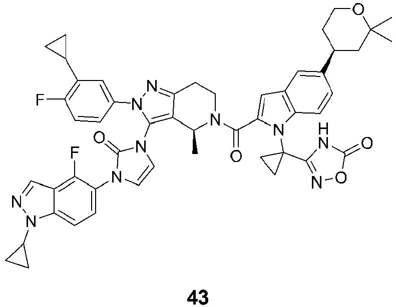 | 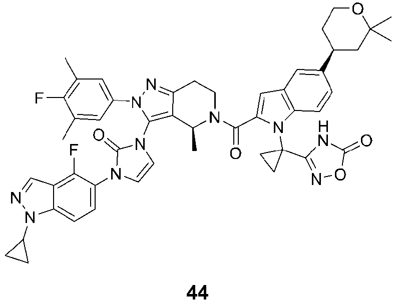 |
| 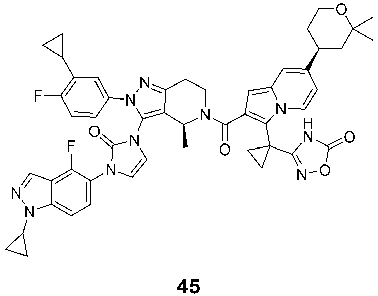 | 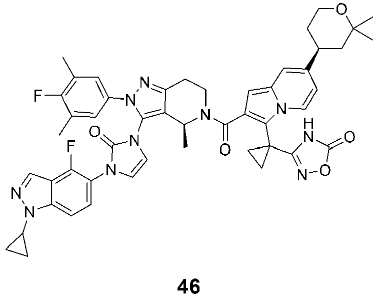 |
| 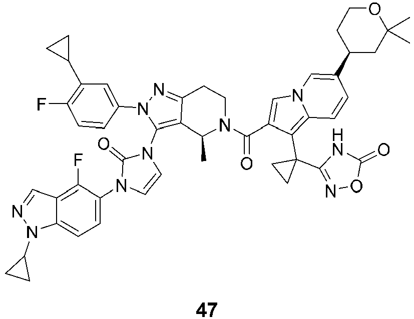 | 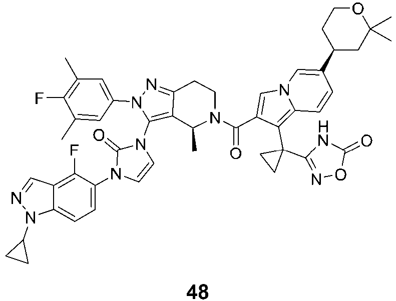 |
| 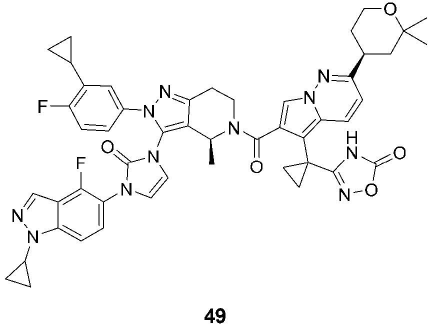 | 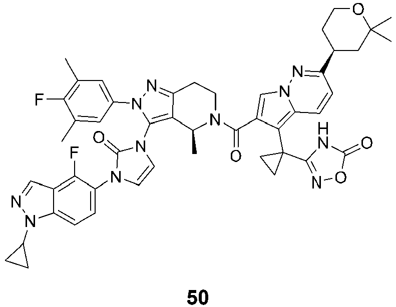 |
| 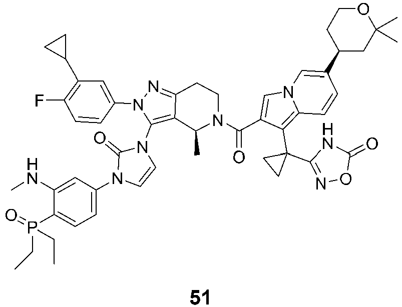 | 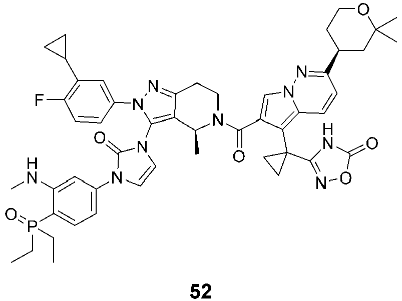 |
| 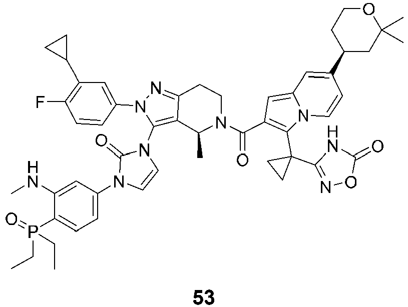 | 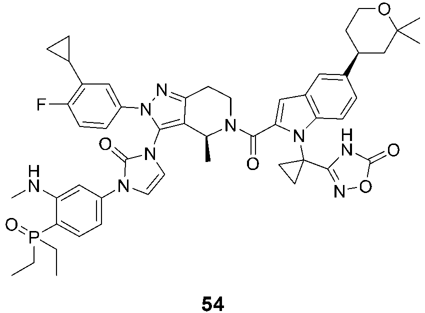 |
| 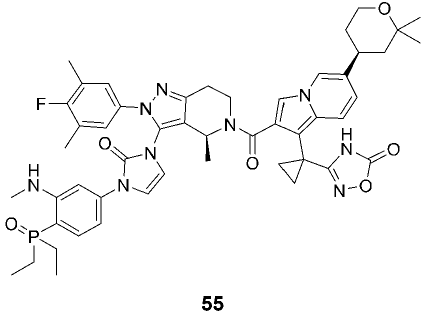 | 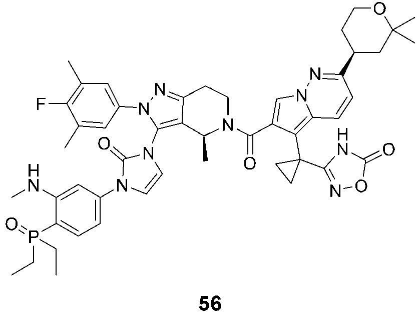 |
| 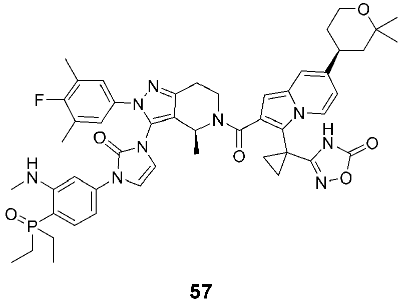 | 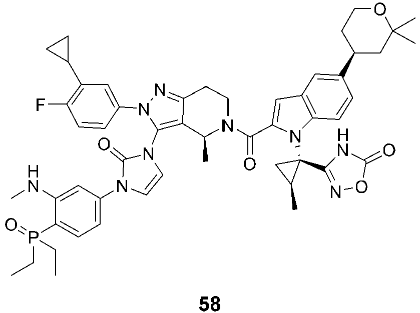 |
| 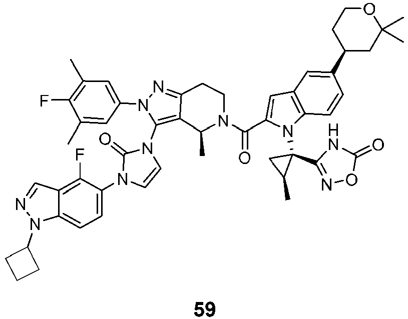 | 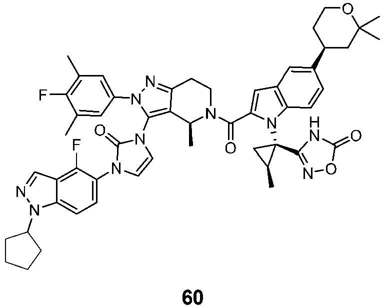 |
| 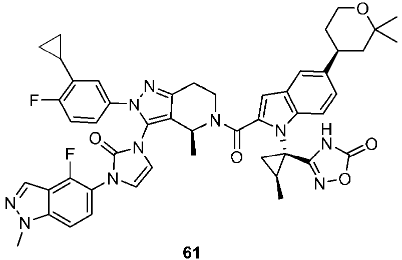 | 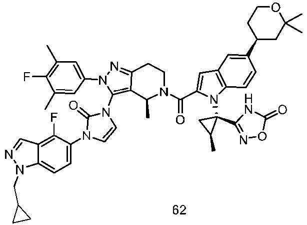 |
| 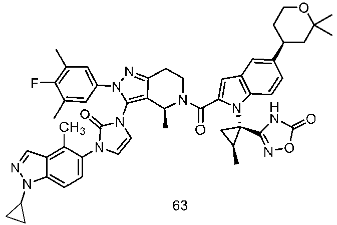 | 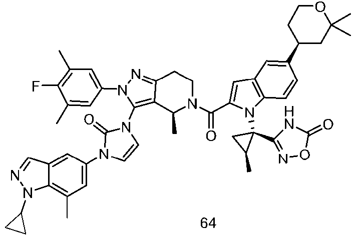 |
| 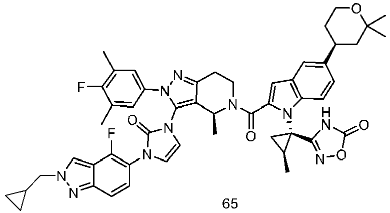 | |

11. A pharmaceutical composition, comprising the compound according to any one of claims 1-10, or stereoisomers, tautomers or pharmaceutically acceptable salts thereof, and optionally further comprising a pharmaceutically acceptable excipient.

12. Use of the compound according to any one of claims 1-10, or stereoisomers, tautomers or pharmaceutically acceptable salts thereof, or the pharmaceutical composition according to claim 11 as a drug or in the manufacture of a drug.

13. The use according to claim 12, wherein the drug is a GLP-1R agonist; further preferably, the drug is a drug for treating and/or preventing disorders related to GLP-1R; further preferably, the drug is a drug for treating and/or preventing disorders mediated with the involvement of GLP-1R; further preferably, the drug is a drug for treating and/or preventing disorders caused by reduced GLP-1R activity or mediated with the involvement of GLP-1R; further preferably, the drug is a drug for preventing and/or treating disorders by agonizing GLP-1R-mediated cascade signals; further preferably, the disorders comprise diabetes, overweight or obesity, non-alcoholic fatty liver disease, Alzheimer's disease, and the like; further preferably, the diabetes is type 2 diabetes.

14. The use according to claim 12, wherein the drug is a drug for treating and/or preventing diabetes, overweight or obesity, non-alcoholic fatty liver disease, or Alzheimer's disease; preferably, the drug is a drug for treating and/or preventing diabetes, overweight or obesity; preferably, the diabetes is type 2 diabetes.
